# EUROPEAN PATENT SPECIFICATION

(11) **EP 1 962 849 B1**
(45) Date of publication and mention of the grant of the patent: **30.09.2009**
(21) Application number: 06831520.9
(22) Date of filing: 19.12.2006
(51) Int. Cl.: A61K 31/4706, A61K 31/4709, A61K 31/4741, C07D 215/42, C07D 215/44, C07D 215/46, C07D 401/04, C07D 405/12, C07D 405/14, A61P 25/00

(54) **QUINOLINE DERIVATIVES USEFUL IN THE TREATMENT OF MGLUR5 RECEPTOR-MEDIATED DISORDERS**
CHINOLIN-DERIVATE ZUR BEHANDLUNG VON MGLUR5-REZEPTOR-VERMITTELTEN ERKRANKUNGEN
DERIVES DE LA QUINOLEINE UTILES DANS LE TRAITEMENT DE PATHOLOGIES INDUITES PAR LES RECEPTEURS MGLUR5

(30) Priority: 20.12.2005 HU 0501165; 18.12.2006 HU 0600918
(43) Date of publication of application: 03.09.2008
(73) Proprietor: Richter Gedeon Nyrt., 1103 Budapest (HU)
(72) Inventor: KESERÜ, György, H-2089 Telki (HU); WÉBER, Csaba, H-1142 Budapest (HU); BIELIK, Attila, H-2377 Örkény (HU); BOBOK, Amrita, Ágnes, H-1025 Budapest (HU); GÁL, Krisztina, H-1193 Budapest (HU); MESZLÉNYINÉ SIPOS, Márta, H-1032 Budapest (HU); MOLNÁR, László, H-1194 Budapest (HU); VASTAG, Mónika, H-1025 Budapest (HU)
(74) Representative: HOFFMANN EITLE
(86) International application number: PCT/HU2006/000121
(87) International publication number: WO 2007/072093

(56) References cited:
- WO-A-02/28837
- WO-A2-03/080580
- DATABASE CHEMCATS [Online] CHEMICAL ABSTRACTS SERVICE, COLUMBUS, OHIO, US; 18 January 2005 (2005-01-18), XP002422866 retrieved from STN & 18 January 2005 (2005-01-18), INTERCHIM , 211 BIS AV J.F. KENNEDY, BP 1140, MONTLUCON, 03103 FRANCE

## Description

### FIELD OF THE INVENTION

The present invention relates to a new class of compounds for use in the treatment of mGluR5 receptor-mediated disorders, to the process for their preparation, to the intermediates of the preparation process, to the pharmaceutical formulations containing these compounds and to their use in the prevention and/or treatment of mGluR5 receptor-mediated disorders.

### BACKGROUND OF THE INVENTION

A major excitatory neurotransmitter in the mammalian central nervous system (CNS) is the glutamate molecule, which binds to neurons, thereby activating cell surface receptors. These receptors can be divided into two major classes, ionotropic and metabotropic glutamate receptors, based on the structural features of the receptor proteins.

Metabotropic glutamate receptors (mGluRs) are G protein-coupled receptors that activate a variety of intracellular second messenger systems following the binding of glutamate. Activation of mGluRs in intact mammalian neurons elicits one or more of the following responses: activation of phospholipase C; increases in phosphoinositide (PI) hydrolysis; intracellular calcium release; activation of phospholipase D; activation or inhibition of adenyl cyclase; increases or decreases in the formation of cyclic adenosine monophosphate (cAMP); activation of guanylyl cyclase; increases in the formation of cyclic guanosine monophosphate (cGMP); activation of phospholipase A2; increases in arachidonic acid release; and increases or decreases in the activity of voltage- and ligand-gated ion channels. (Schoepp et al., Trends Pharmcol. Sci. 1993, 14:13*;* Schoepp, Neurochem. Int. 1994, 24:439*,* Pin et al., Neuropharmacology 1995, 34:1*;* Bordi and Ugolini, Prog. NeurobioL 1999, 59:55*).*

Eight distinct mGluR subtypes, termed mGluRl through mGluR8, have been identified by molecular cloning (Nakanishi, Neuron 1994, 13:1031*;* Pin et al., Neuropharmacology 1995, 34:1*;* Knopfel et al., J. Med Chem. 1995, 38:1417*).* Further receptor diversity occurs via expression of alternatively spliced forms of certain mGluR subtypes (Pin et al., PNAS 1992, 89:10331*;* Minakami et al., BBRC 1994, 199:1136*;* Joly et al., J. Neurosci. 1995, 15: 3970*).*

Metabotropic glutamate receptor subtypes may be subdivided into three groups, Group I, Group II, and Group III mGluRs, based on amino acid sequence homology, the second messenger systems utilized by the receptors, and by their pharmacological characteristics. Group I mGluR comprises mGluR1, mGluR5 and their alternatively spliced variants.

Attempts at elucidating the physiological roles of Group I mGluRs suggest that activation of these receptors elicits neuronal excitation. Evidence indicates that this excitation is due to direct activation of postsynaptic mGluRs, but it also has been suggested that activation of presynaptic mGluRs occurs, resulting in increased neurotransmitter release (Pin et al., Neuropharmacology 1995, 34:1*;* Watkins et al., Trends Pharmacol. Sci. 1994, 15:33*).*

Metabotropic glutamate receptors have been implicated in a number of normal processes in the mammalian CNS. Activation of mGluRs has been shown to be required for induction of hippocampal long-term potentiation and cerebellar long-term depression (Bashir et al., Nature 1993, 363:347*;* Bortolotto et al., Nature 1994, 368:740*;* Aiba et al., Cell 1994, 79:365*;* Aiba et al., Cell 1994, 79:377*).* A role for mGluR activation in nociception and analgesia also has been demonstrated *(*Meller et al., Neuroreport 1993, 4: 879*;* Bordi and Ugolini, Brain Res. 1999, 871:223*).*

Group I metabotropic glutamate receptors and mGluR5 in particular, have been suggested to play roles in a variety of pathophysiological processes and disorders affecting the CNS. These include stroke, head trauma, anoxic and ischemic injuries, hypoglycemia, epilepsy, neurodegenerative disorders such as Alzheimer's disease, acute and chronic pain, substance abuse and withdrawal, obesity and gastroesophageal reflux disease (GERD) *(*Schoepp et al., Trends Pharmacol. Sci. 1993, 14:13*;* Cunningham et al., Life Sci. 1994, 54:135*;* Hollman et al., Ann. Rev. Neurosci. 1994, 17:31*;* Pin et al., Neuropharmacology 1995, 34:1*;* Knopfel et al., J. Med. Chem. 1995, 38:1417*;* Spooren et al., Trends Pharmacol. Sci. 2001, 22:331*;* Gasparini et al. Curr. Opin. Pharmacol. 2002, 2:43*;* Neugebauer Pain 2002, 98:1*,* Slassi et al., Curr Top Med Chem. 2005; 5(9): 897-911*).* MGluR5-selective compounds such as 2-methyl-6-(phenylethynyl)-pyridine ("MPEP") are effective in animal models of mood disorders, including anxiety and depression *(*Spooren et al., J. Pharmacol. Exp. Ther. 2000, 295:1267*;* Tatarczynska et al., Br. J. Pharmacol. 2001, 132:1423*;* Klodzynska et al., Pol. J. Pharmacol, 2001, 132:1423*).* Much of the pathology in these conditions is thought to be due to excessive glutamate-induced excitation of CNS neurons. As Group I mGluRs appear to increase glutamate-mediated neuronal excitation via postsynaptic mechanisms and enhanced presynaptic glutamate release, their activation probably contributes to the pathology. Therefore, selective antagonists of Group I mGluR receptors could be therapeutically beneficial, especially as neuroprotective agents, analgesics or anticonvulsants.

Various derivatives of compounds that have the above mentioned pharmacological activity are described in the literature.

International Patent Application WO 02/20489 relates to new quinoline derivatives and methods of their use for the treatment of cGMP-associated conditions, cardiovascular disorders, sexual dysfunction, diabetes and gastrointestinal disorders. The compounds are stated to be particularly potent and selective inhibitors of cGMP PDE-5.

International Patent Application WO 03/080580 describes novel quinolin compounds and their use in the treatment of anxiety, depression, obesity and cognitive memory disorders. These compounds are stated to be useful as 5-HT6 receptor antagonists. They are useful for treating neurological diseases and disorders.

International Patent Application WO 05/058834 relates to novel quinoline derivatives for use in treating liver X receptor (LXR) mediated diseases particularly multiple sclerosis, rheumatoid arthritis, inflammatory bowel disease and atherosclerosis, which compounds suppress Th-1 type lymphokine production, resulting in increased HDL levels, and cholesterol metabolism.

However there remains a need for novel compounds and composition that exhibit an activity at metabotropic glutamate receptors (mGluRs), especially at the mGluR5 receptor.

### SUMMARY OF THE INVENTION

The present invention relates to compounds for use in the treatment of mGluR5 receptor-mediated disorders. These compounds are represented by the formula (I): 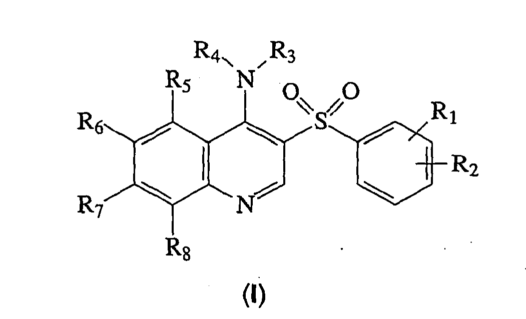 wherein
R₁ and R₂ are independently selected from the group consisting of hydrogen, halogen, alkyl, alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, alkyl, alkylhydroxy, alkyloxycarbonyl, aminocarbonyl, -OCH₂CH₂O- benzyl and substituted phenyl group;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

Another aspect of the present invention provides processes for the synthesis of compounds of formula (I).

A further aspect of the present invention relates to the intermediates of the preparation process.

A further aspect of the present invention provides pharmaceutical compositions containing a therapeutically effective amount of a compound of formula (I) and/or enantiomers and/or racemates and/or diastereomers and/or salts and/or hydrates or solvates thereof as active ingredient and pharmaceutically acceptable diluents, excipients and/or inert carriers.

The compounds of formula (I) are for use in the prevention and/or treatment of mgluR5 receptor mediated disorders, particularly neurological disorders, psychiatric disorders, acute and chronic pain and neuromuscular dysfunctions of the lower urinary tract and gastrointestinal disorders.

A further aspect of the present invention provides the use of a compound of formula (I) for the manufacture of a medicament for the prevention and/or treatment of mGluR5 receptor-mediated disorders, particularly neurological disorders, psychiatric disorders, acute and chronic pain and neuromuscular dysfunctions of the lower urinary tract and gastrointestinal disorders.

These and other aspects of the present invention are described in detail herein.

### DETAILED DESCRIPTION OF THE INVENTION

The compounds for use in the treatment of mGluR5 receptor-mediated disorders according to the present invention are represented by formula (I): 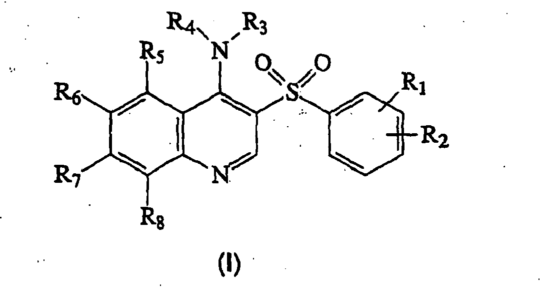 wherein
R₁ and R₂ are independently selected from the group consisting of hydrogen, halogen, alkyl alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, alkyl, alkylhydroxy, alkyloxycarbonyl, aminocarbonyl, -OCH₂CH₂O-, benzyl and substituted phenyl group;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

Also disclosed are compounds of formula (I), wherein R₁ and R₂ are independently selected from the group consisting of hydrogen, halogen, C₁₋₄ alkyl, C₁₋₄ alkoxy, cyano, optionally substituted amino group or saturated heterocyclyl group, wherein the heteroatom is N;
R₃ and R₄ are independently selected from the group consisting of hydrogen, C₁₋₄ alkyl, substituted aryl group having at least one substituent selected from the group of hydrogen, halogen, C₁₋₄ alkyl, C₁₋₄ alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, C₁₋₄ alkyl, C₁₋₄ alkylhydroxy, alkyloxycarbonyl, aminocarbonyl, -OCH₂CH₂O- benzyl and substituted phenyl group, which may be optionally substituted by 1 or 2 groups selected from hydrogen, halogen, C₁₋₄ alkyl, C₁₋₄ alkoxy group;
R₅, R₆, R₇ and R₈ are independently selected from the group consisting of hydrogen, halogen, C₁₋₄ alkyl, C₁₋₄ alkoxy, cyano group, or
R₆ and R₇ together with the atoms to which they are attached can form an unsaturated 5 to 7 membered heterocyclyl group, containing 1 or 2 O atom(s);
and/or enantiomers and/or racemates and/or diastereomers and/or pharmaceutically acceptable salts thereof formed with acids or bases.

Also disclosed are compounds of formula (I), wherein R¹ and R² are independently selected from the group consisting of hydrogen, chloro, fluoro, C₁₋₂ alkyl, C₁₋₂ alkoxy, cyano or piperidinyl group;
R₃ and R₄ are independently selected from the group consisting of hydrogen, C₁₋₂ alkyl, benzyl group substituted 1 or 2 groups independently selected from hydrogen, halogen, C₁₋₄ alkyl, C₁-₄ alkoxy group,
R₃ and R₄ together with the N atoms to which they are attached can form pyrrolidinyl, homopiperidinyl, morpholinyl group or piperidinyl group which may be optionally substituted by the groups selected from hydrogen, halogen, C₁₋₄ alkyl, hydroxymethyl, alkyloxycarbonyl, aminocarbonyl, and -OCH₂CH₂O- group or piperazinyl group which may be substituted at N(4) by the groups selected from C₁₋₄ alkyl, benzyl, -alkyloxycarbonyl and phenyl, which may be optionally substituted by 1 or 2 groups selected from hydrogen, halogen, C₁₋₄ alkyl, alkoxy group;
R₅, R₆, R₇ and R₈ are independently selected from the group consisting of hydrogen, chloro, fluoro, C₁₋₂ alkyl, C₁₋₂ alkoxy, cyano group, or
R₆ and R₇ together with the atoms to which they are attached can form 2,3-dihydro-[1,4]dioxine or 2,5-dihydro-furan ring;
and/or enantiomers and/or racemates and/or diastereomers and/or pharmaceutically acceptable salts thereof formed with acids or bases.

Listed below are definitions of various terms used in the specification and claims to describe the present invention.

For the avoidance of doubt it is to be understood that where in this specification a group is qualified by 'hereinbefore defined, defined hereinbefore or defined above the said group encompasses the first occurring and broadest definition as well as each and all of the other definitions for that group.

For the avoidance of doubt it is to be understood that in this specification "C₁₋₄" means a carbon containing linear or branched group having 1, 2, 3 or 4 carbon atoms.

As used herein the term "alkyl" as well as other groups having the prefix "alk" such as alkoxy means carbon chains which may be linear or branched or combinations thereof. Examples of alkyl groups include methyl, ethyl, propyl, isopropyl, butyl, sec- and tert-butyl, pentyl, hexyl, heptyl and the like.

The term "hetero" unless specifically stated otherwise includes one or more O or N atoms. For example heterocyclic ring systems that contain one or more O or N atoms in the ring, including mixtures of such atoms. Heteroatoms replace carbon atoms. Examples of heterocyclic rings include pyrrolidinyl, piperidinyl, piperazinyl, morpholinyl and piperidin-2-on groups.

The term "halogen" includes fluorine, chlorine, bromine and iodine atoms.

The term "amino group" unless specifically stated otherwise includes -NH₂ group and groups, derived from primary or secondary amines, which also can mean aromatic amines.

The term "optionally substituted" is intended to include both substituted and unsubstituted groups.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Among the salts formed with bases especially important are the salts formed with alkali metals, e.g. sodium, potassium, alkali-earth metals, e.g. calcium and magnesium, as well as with ammonia or organic amines. The latter bases can have further substituents, e.g. hydroxy or amino groups, which can influence e.g. the solubility and the handling of the product. Both organic and inorganic acids can be used for the formation of acid addition salts. Suitable inorganic acids can be e.g. hydrochloric acid, sulfuric acid and phosphoric acid. Representatives of monovalent organic acids can be e.g. formic acid, acetic acid, trifluoroacetic acid, propionic acid, and different butyric acids, valeric acids and capric acids. Representatives of bivalent organic acids can be e.g. oxalic acid, malonic acid, maleic acid, fumaric acid and succinic acid. Other organic acids can also be used, such as hydroxy acids e.g. citric acid, tartaric acid, or aromatic carboxylic acids e.g. benzoic acid or salicylic acid, as well as aliphatic and aromatic sulfonic acids e.g. methanesulfonic acid and p-toluenesulfonic acid. Especially valuable group of the acid addition salts is in which the acid component itself does not have therapeutical effect in the applied dose or it does not have unfavorable influence on the effect of the active ingredient. These acid addition salts are pharmaceutically acceptable acid addition salts. The reason why acid addition salts, which do not belong to the pharmaceutically acceptable acid addition salts belong to the present invention is, that in given case they can be advantageous in the purification and isolation of the desired compounds.

Compounds described herein can contain one or more asymmetric centers and may thus give rise to diastereomers and optical isomers. The present invention includes all such possible diastereomers as well as their racemic mixtures and their substantially pure enantiomers.

Especially important compounds of formula (I) are the following:
3-(3,4-Dimethyl-benzenesulfonyl)-4-(morpholin-4-yl)quinoline
3-(4-Methyl-benzenesulfonyl)-4-(3-methyl-piperidin-1-yl)-quinoline
3-(3,4-Dimethyl-benzenesulfonyl)-4-(4-methyl-piperidin-1-yl)-quinoline
3-(4-Methyl-benzenesulfonyl)-4-(4-methyl-piperidin-1-yl)-quinoline
3-Benzenesulfonyl-4-(piperidin-1-yl)-quinoline
3-(4-Methyl-benzenesulfonyl)-4-(piperidin-1-yl)-quinoline
4-Benzylamino-3-(4-methyl-benzenesulfonyl)-quinoline
6-Ethyl-4-(4-methyl-piperidin-1-yl)-3-(4-methoxy-benzenesulfonyl)-quinoline
6-Fluoro-3-(4-methyl-benzenesulfonyl)-4-(4-methyl-piperidin-1-yl)-quinoline
6-Ethoxy-3-(4-chloro-benzenesulfonyl)-4-(4-fluoro-benzylamino)-quinoline
4-(Azepan-1-y1)-3-(4-methyl-benzenesulfonyl)-quinoline
4-(Azepan-1-yl)-3-(4-chloro-benzenesulfonyl)-quinoline
6-Methyl-3-(4-methyl-benzenesulfonyl)-4-(4-methyl-piperidin-1-yl)-quinoline
4-(4-Methylpiperidin-1-yl)-3-benzenesulfonyl-quinoline
9-(4-methyl-piperidin-1-yl)-8-benzenesulfonyl-2,3-dihydro-[1,4] dioxino[2,3-g]quinoline
6-Ethyl-4-(4-ethyloxycarbonyl-piperidin-1-yl)-3-(4-chloro-benzenesulfonyl)-quinoline
4-Diethylamino-3-(4-methyl-benzenesulfonyl)-quinoline
4-(4-Benzyl-piperazin-1-yl)-3-(4-chloro-benzenesulfonyl)-quinoline
4-(Azepan-1-yl)-3-benzenesulfonyl-quinoline
3-(3-Cyano-benzenesulfonyl)-6-fluoro-4-(4-methyl-piperidin-1-yl)-quinoline
6-Fluoro-3 -(4-methoxy-benzenesulfonyl)-4-(4-methyl-pipendin-1 -yl)-quinoline
6-Fluoro-3-(3-methoxy-benzenesulfonyl)-4-(4-methyl-piperidin-1-yl)-quinoline
6-Fluoro-3-(3,4-dimethyl-benzenesulfonyl)-4-(4-methyl-piperidin-1-yl)-quinoline
3-(3-Chloro-4-methoxy-benzenesulfonyl)-6-fluoro-4-(4-methyl-piperidin-1-yl)-quinoline
3-(3-Chloro-4-fluoro-benzenesulfonyl)-6-fluoro-4-(4-methyl-piperidin-1-yl)-quinoline
3-(3,4-Dichloro-benzenesulfonyl)-6-fluoro-4-(4-methyl-piperidin-1-yl)-quinoline
3-(3-Chloro-benzenesulfonyl)-6-fluoro-4-(4-methyl-piperidin-1-yl)-quinoline
3-(4-Chloro-benzenesulfonyl)-6-methyl-4-(4-methyl-piperidin-1-yl)-quinoline
3-(3-Fluoro-benzenesulfonyl)-6-methyl-4-(4-methyl-piperidin-1-yl)-quinoline
3-(3-Methoxy-benzenesulfonyl)-6-methyl-4-(4-methyl-piperidin-1-yl)-quinoline
3-(3,4-Dimethyl-benzenesulfonyl)-6-methyl-4-(4-methyl-piperidin-1-yl)-quinoline
3-(3-Chloro-4-methoxy-benzenesulfonyl)-6-methyl-4-(4-methyl-piperidin-1-yl)-quinoline
3-(3-Chloro-benzenesulfonyl)-6-methyl-4-(4-methyl-piperidin-1-yl)-quinoline
3-(3-Fluoro-4-methyl-benzenesulfonyl)-6-methyl-4-(4-methyl-piperidin-1-yl)-quinoline
3-(3-Chloro-4-methyl-benzenesulfonyl)-6-methyl-4-(4-methyl-piperidin-1-yl)-quinoline
3-(3-Chloro-4-fluoro-benzenesulfonyl)-6-methyl-4-(4-methyl-piperidin-1-yl)-quinoline
3-(3,4-Dichloro-benzenesulfonyl)-7-fluoro-4-(4-methyl-piperidin-1-yl)-quinoline
7-Fluoro-3-(3-cyano-benzenesulfonyl)-4-(4-methyl-piperidin-1-yl)-quinoline
7-Fluoro-3-(4-cyano-benzenesulfonyl)-4-(4-methyl-piperidin-1-yl)-quinoline
3-(3-Chloro-4-methyl-benzenesulfonyl)-7-fluoro-4-(4-methyl-piperidin-1-yl)-quinoline
7-Fluoro-3-(3-methoxy-benzenesulfonyl)-4-(4-methyl-piperidin-1-yl)-quinoline
3-(3,4-Difluoro-benzenesulfonyl)-7-fluoro-4-(4-methyl-piperidin-1-yl)-quinoline
3-(3-chloro-4-fluoro-benzenesulfonyl)-7-fluoro-4-(4-methyl-piperidin-1-yl)-quinoline
7-Chloro-3-(3,5-dichloro-benzenesulfonyl)-4-(4-methyl-piperidin-1-yl)-quinoline
7-Chloro-3-(3,5-difluoro-benzenesulfonyl)-4-(4-methyl-piperidin-1-yl)-quinoline
7-Chloro-3-(3-cyano-benzenesulfonyl)-4-(4-methyl-piperidin-1-yl)-quinoline
7-Chloro-3-(4-methyl-benzenesulfonyl)-4-(4-methyl-piperidin-1-yl)-quinoline
7-Chloro-3-(4-fluoro-benzenesulfonyl)-4-(4-methyl-piperidin-1-yl)-quinoline
3-Benzenesulfonyl-7-Chloro-4-(4-methyl-piperidin-1-yl)-quinoline
7-Chloro-3-(4-chloro-benzenesulfonyl)-4-(4-methyl-piperidin-1-yl)-quinoline
7-Chloro-3-(3,4-dimethoxy-benzenesulfonyl)-4-(4-methyl-piperidin-1-yl)-quinoline
7-Chloro-3-(3-fluoro-benzenesulfonyl)-4-(4-methyl-piperidin-1-yl)-quinoline
7-Chloro-3-(3-methoxy-benzenesulfonyl)-4-(4-methyl-piperidin-1-yl)-quinoline
7-Chloro-3-(3-chloro-4-methoxy-benzenesulfonyl)-4-(4-methyl-piperidin-1-yl)-quinoline
7-Chloro-3-(3-chloro-benzenesulfonyl)-4-(4-methyl-piperidin-1-yl)-quinoline
7-Chloro-3-(3-chloro-4-fluoro-benzenesulfonyl)-4-(4-methyl-piperidin-1-yl)-quinoline
6-Chloro-3-(3,5-difluoro-benzenesulfonyl)-4-(4-methyl-piperidin-1-yl)-quinoline-6-Chloro-3-(4-methyl-benzenesulfonyl)-4-(4-methyl-piperidin-1-yl)-quinoline
6-Chloro-3-(4-chloro-benzenesulfonyl)-4-(4-methyl-piperidin-1-yl)-quinoline
8-Fluoro-3-(3-fluoro-4-methyl-benzenesulfonyl)-4-(4-methyl-piperidin-1-yl)-quinoline
8-Fluoro-3-(4-methyl-benzenesulfonyl)-4-(4-methyl-piperidin-1-yl)-quinoline
3-(3,4-Dichloro-benzenesulfonyl)-8-fluoro-4-(4-methyl-piperidin-1-yl)-quinoline
3-(3-chloro-4-fluoro-benzenesulfonyl)-8-fluoro-4-(4-methyl-piperidin-1-yl)-quinoline
3-(3,4-Difluoro-benzenesulfonyl)-8-fluoro-4-(4-methyl-piperidin-1-yl)-quinoline
3-Benzenesulfonyl-6-methyl-4-(morpholin-1-yl)-quinoline
3-(3-Chloro-benzenesulfonyl)-6-methoxy-4-(morpholin-1-yl)-quinoline
3-Benzenesulfonyl-6-fluoro-4-(morpholin-1-yl)-quinoline
6-Chloro-3-(4-chloro-benzenesulfonyl)-4-(morpholin-1-yl)-quinoline
3-(3-Chloro-4-methyl-benzenesulfonyl)-7-fluoro-4-(morpholin-1-yl)-quinoline
3-(3,4-Dichloro-benzenesulfonyl)-7-fluoro-4-(morpholin-1-yl)-quinoline
7-Chloro-3-(3,5-dichloro-benzenesulfonyl)-4-(morpholin-1-yl)-quinoline
7-Chloro-3-(3,4-dimethyl-benzenesulfonyl)-4-(morpholin-1-yl)-quinoline
7-Chloro-3-(3-chloro-benzenesulfonyl)-4-(morpholin-1-yl)-quinoline
7-Chloro-3-(3-chloro-4-methyl-benzenesulfonyl)-4-(morpholin-1-yl)-quinoline
7-Chloro-3-(3,4-dichloro-benzenesulfonyl)-4-(morpholin-1-yl)-quinoline
7-Chloro-3-(3-chloro-4-fluoro-benzenesulfonyl)-4-(morpholin-1-yl)-quinoline.

### Pharmaceutical formulations

The present invention provides in a further aspect pharmaceutical compositions comprising a compound represented by formula (I) and/or enantiomers and/or racemates and/or diastereomers and/or pharmaceutically acceptable salts and/or hydrates and/or solvates thereof as active ingredient and one or more pharmaceutically acceptable carriers.

The compounds of formula (I) and/or enantiomers and/or racemates and/or diastereomers and/or pharmaceutically acceptable salts and/or hydrates and/or solvates thereof may be administered by any convenient method, for example by oral, parenteral (including subcutaneous, intramuscular, and intravenous), buccal, sublingual, nasal, rectal or transdermal administration and the pharmaceutical compositions adapted accordingly.

The compounds of formula (I) and/or enantiomers and/or racemates and/or diastereomers and/or pharmaceutically acceptable salts and/or hydrates and/or solvates thereof which are active when given orally can be formulated as liquids or solids, for example syrups, suspensions or emulsions, tablets, capsules and lozenges.

A liquid formulation of the compounds of formula (I) and/or enantiomers and/or racemates and/or diastereomers and/or pharmaceutically acceptable salts and/or hydrates and/or solvates thereof generally consist of a suspension or solution of the compound of formula (I) and/or enantiomers and/or racemates and/or diastereomers and/or pharmaceutically acceptable salts and/or hydrates and/or solvates thereof in a suitable liquid carrier(s) for example an aqueous solvent, such as water, ethanol or glycerine, or a non-aqueous solvent, such as polyethylene glycol or an oil. The formulation may also contain a suspending agent, preservative, flavouring or colouring agent.

A composition in the solid form of a tablet can be prepared using any suitable pharmaceutical carrier(s) routinely used for preparing solid formulations. Examples of solid carriers include lactose, terra alba, sucrose, talcum, gelatine, agar, pectin, acacia, magnesium stearate, stearic acid etc. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent.

A composition in the solid form of a capsule can be prepared using routine encapsulation procedures. For example, pellets containing the active ingredient can be prepared using standard carriers and then filled into a hard gelatine capsule; alternatively, a dispersion or suspension can be prepared using any suitable pharmaceutical carrier(s), for example aqueous gums, celluloses, silicates or oils and the dispersion or suspension then filled into a soft gelatine capsule.

Typical parenteral compositions consising of a solution or suspension of the compound of formula (I) and/or enantiomers and/or racemates and/or diastereomers and/or pharmaceutically acceptable salts and/or hydrates and/or solvates thereof in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

Compositions of the present invention for nasal administration containing a compound of formula (I) and/or enentiomers and/or racemates and/or diastereomers and/or salts thereof may conveniently be formulated as aerosols, drops, gels and powders. Aerosol formulations of the present invention typically comprise a solution or fine suspension of the compound of formula (I) and/or enantiomers and/or racemates and/or diastereomers and/or pharmaceutically acceptable salts and/or hydrates and/or solvates thereof in a pharmaceutically acceptable aqueous or non-aqueous solvent and are usually presented in a single or multidose quantities in sterile form is a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal once the contents of the container have been exhausted. Where the dosage form comprises an aerosol dispenser, it will contain a propellant which can be a compressed gas, such as compressed air or an organic propellant, such as a fluorochlorohydrocarbon. The aerosol dosages form can also take the form af a pump-atomiser.

Compositions of the present invention containing a compound of formula (I) and/or enantiomers and/or racemates and/or diastereomers and/or pharmaceutically acceptable salts and/or hydrates and/or solvates are suitable for buccal or sublingual administration including tablets, lozenges and pastilles, wherein the active ingredient is formulated with a carrier, such as sugar and acacia, tragacanth, or gelatine, glycerin etc.

Compositions of the present invention containing a compound of formula (I) and/or enantiomers and/or racemates and/or diastereomers and/or pharmaceutically acceptable salts and/or hydrates and/or solvates thereof for rectal administration are conveniently in the form of suppositories containing a conventional suppository base, such as cocoa butter and other materials commonly used in the art. The suppositories may be conveniently formed by first admixing the composition with the softened or melted carrier(s) followed by chilling and shaping in moulds.

Compositions of the present invention containing a compound of formula (I) and/or enantiomers and/or racemates and/or diastereomers and/or pharmaceutically acceptable salts and/or hydrates and/or solvates thereof for transdermal administration include ointments, gels and patches.

The compositions of the present invention containing a compound of formula (I) and/or enantiomers and/or racemates and/or diastereomers and/or pharmaceutically acceptable salts and/or hydrates and/or solvates thereof is preferably in the unit dose form, such as tablet, capsule or ampoule.

Each dosage unit of the present invention for oral administration contains preferably from 0.1 to 500 mg of a compound of formula (I) and/or enantiomers and/or racemates and/or diastereomers and/or pharmaceutically acceptable salts and/or hydrates and/or solvates thereof calculated as a free base.

Each dosage unit of the present invention for parenteral administration contains preferably from 0.1 to 500 mg of a compound of formula (I) and/or enantiomers and/or racemates and/or diastereomers and/or pharmaceutically acceptable salts and/or hydrates and/or solvates thereof calculated as a free base.

The pharmaceutically acceptable compounds of formula (I) and/or enantiomers and/or racemates and/or diastereomers and/or pharmaceutically acceptable salts and/or hydrates and/or solvates thereof can be administered in a daily dosage regimen. In the treatment of mGluR5 mediated disorders, such as schizophrenia, anxiety, depression, panic, bipolar disorders, and circadian disorders or chronic and acute pain disorders the dosage levels from about 0,01 mg/kg to about 140 mg/kg of body weight per day are useful or alternatively about 0.5 mg to about 7 g per patient per day.

The amount of active ingredient that may be combined with the carrier materials to produce a single dosage form will vary depending upon the host treated and the particular mode of administration. For example, a formulation intended for the oral administration to humans may conveniently contain from about 0.5 mg to about 5 g of active agent, compounded with an appropriate and convenient amount of carrier material which may vary from about 5 to about 95 percent of the total composition. Unit dosage forms will generally . contain between from about 1 mg to about 1000 mg of the active ingredient, typically 25 mg, 50 mg, 100 mg, 200 mg, 25-300 mg, 400 mg, 500 mg, 600 mg, 800 mg or 1000 mg.

It is understood, however, that the specific dose level for any particular patient will depend upon a variety of factors including the age, body weight, general health, sex, diet, time of administration, route of administration, rate of excretion, drug combination and the severity of the particular disease undergoing therapy.

### Medical use

The compounds of formula (I) and/or enantiomers and/or racemates and/or diastereomers and/or pharmaceutically acceptable salts and/or hydrates and/or solvates of the present invention have been found to exhibit biological activity at mGluR5 receptors and are expected to be useful in the treatment of mGluR5 mediated disorders.

It has been found that the compounds according to the present invention or salts thereof, exhibit a high degree of potency and selectivity for individual metabotropic glutamate receptor (mGluR) subtypes. In particular there are compounds according to the present invention that are potent and selective for mGluR5 receptor. Accordingly, the compounds of the present invention are expected to be useful in the prevention and/or treatment of conditions associated with excitatory activation of an mGluR5 receptor and for inhibiting neuronal damage caused by excitatory activation of an mGluR5 receptor. The compounds may be used to produce an inhibitory effect of mGluR5, in mammals, including human.

Thus, it is expected that the compounds of the invention are well suited for the prevention and/or treatment of mGluR5 receptor-mediated disorders such as acute and chronic neurological and psychiatric disorders, chronic and acute pain disorders.

The compounds of the present invention are also well suited for the treatment of neuromuscular dysfunction of the lower urinary tract, such as urinary urgency, overactive bladder, greater urinary frequency, reduced urinary compliance, cystitis, incontinence, enuresis and dysuria.

The dose required for the therapeutic or preventive treatment of a particular disorder will necessarily be varied depending on the host treated and the route of administration.

The invention relates to compounds of formula (I) and/or enantiomers and/or racemates and/or diastereomers and/or pharmaceutically acceptable salts and/or hydrates and/or solvates thereof as defined hereinbefore, for use in therapy.

The invention relates to compounds of formula (I) as defined hereinbefore, for use in prevention and/or treatment of mGluR5 receptor-mediated disorders.

The invention relates to compounds of formula (I) as defined hereinbefore, for use in prevention and/or treatment of neurological disorders.

The invention relates to compounds of formula (I) as defined hereinbefore, for use in prevention and/or treatment of psychiatric disorders.

The invention relates to compounds of formula (I) as defined hereinbefore, for use in prevention and/or treatment of chronic and acute pain disorders.

The invention relates to compounds of formula (I) as defined hereinbefore, for use in prevention and/or treatment of neuromuscular dysfunctions of the lower urinary tract and gastrointestinal disorders.

The invention relates to compounds of formula (I) as defined hereinbefore, for use in prevention and/or treatment of pain related to migraine, inflammatory pain, neuropathic pain disorders such as diabetic neuropathies, arthritis and rheumatoid diseases, low back pain, post-operative pain and pain associated with various conditions including angina, in renal or biliary colic, menstruation, migraine and gout.

The invention relates to compounds of formula (I) as defined hereinbefore, for use in prevention and/or treatment of Alzheimer's disease senile dementia, AIDS-induced dementia Parkinson's disease, amyotrophic lateral sclerosis, Huntington's Chorea, migraine, epilepsy, schizophrenia, depression, anxiety, acute anxiety, obesity, obsessive compulsive disorder, ophthalmological disorders such as retinopathies, diabetic retinopathies, glaucoma, auditory neuropathic disorders such as tinnitus, chemotherapy induced neuropathies, post-herpetic neuralgia and trigeminal neuralgia, tolerance, dependency, Fragile X, autism, mental retardation, schizophrenia and Down's Syndrome.

The invention relates to compounds of formula (I) as defined hereinbefore, for use in prevention and/or treatment of stroke, head trauma, anoxic and ischemic injuries, hypoglycemia, cardiovascular diseases and epilepsy.

The compounds are also well suited for the treatment of neuromuscular dysfunction of the lower urinary tract, such as urinary urgency, overactive bladder, greater urinary frequency, reduced urinary compliance, cystitis, incontinence, enuresis and dysuria.

The compounds are also well suited for the treatment of gastrointestinal disorders, such as transient lower esophageal sphincter relaxation (TLESR), gastrointestinal reflux disease and irritable bowel syndrome.

The present invention relates also to the use of a compound of formula (I) as defined hereinbefore, in the manufacture of a medicament for the prevention and/or treatment of mGluR5 receptor-mediated disorders and any disorder listed above.

The invention also provides a method of treatment and/or prevention of mGluR5 receptor mediated disorders and any disorder listed above, in a patient suffering from, or at risk of, said condition, which comprises administering to the patient an effective amount of a compound of formula (I), as hereinbefore defined.

In the context of the present specification, the term "therapy" includes treatment as well as prevention, unless there are specific indications to the contrary. The terms "therapeutic" and "therapeutically" should be construed accordingly.

In this specification, unless stated otherwise, the term "antagonist" means a compound that by any means, partly or completely blocks the transduction pathway leading to the production of a response by the ligand.

The term "disorder", unless stated otherwise, means any condition and disease associated with metabotropic glutamate receptor activity.

### Methods of preparation

### Abbreviations

The abbreviations used herein have the following tabulated meanings. Abbreviations not tabulated below have their meanings as commonly used unless specifically stated otherwise.
- DMF: N,N-dimethylformamide
- t-BuOH: 2-methyl-2-propanol
- AcOH: acetic acid

- THF: tetrahydrofuran

According to the present invention a process for the preparation of a compound of formula (I): 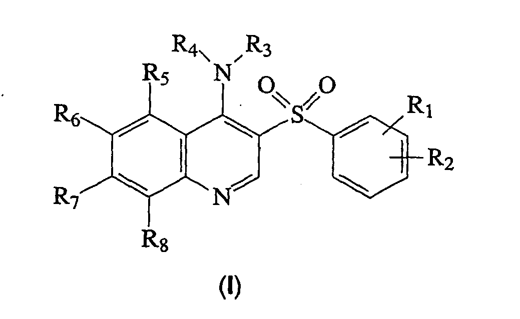 wherein
R₁ and R₂ are independently selected from the group consisting of hydrogen, halogen, alkyl, alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, alkyl, alkylhydroxy, alkyloxycarbonyl, aminocarbonyl, -OCH₂CH₂O-, benzyl and substituted phenyl group;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases
a.) converting a compound of formula (VI) 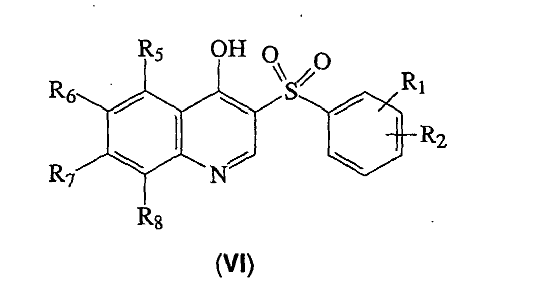 wherein R₁, R₂, R₅, R₆, R₇ and R₈ are as defined above for a compound of formula (I), to a compound of formula (VII) 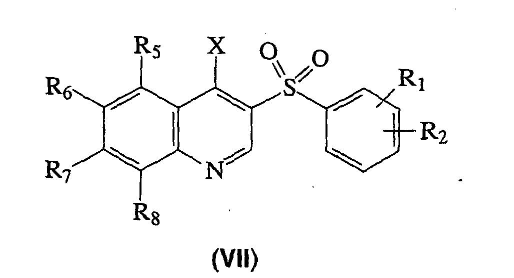 wherein X is selected from halogen, benzenesulfonyloxy or trifluoromethanesulfonyloxy groups and R₁, R₂, R₅, R₆, R₇ and R₈ are as defined above for a compound of formula (I), thereafter reacting the obtained compound of formula (VII) with a compound of formula (VIII): 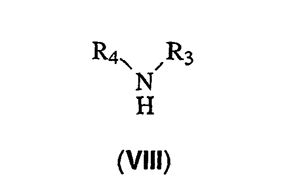 wherein R₃ and R₄ are as defined above for a compound of formula (I) to give a compound of formula (I), and optionally forming enantiomers and/or racemates and/or diastereomers and/or pharmaceutically acceptable salts thereof, or
b.) reacting a compound of formula (XV): 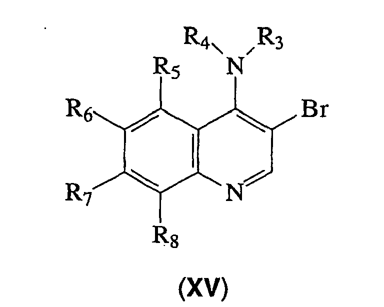 wherein R₃, R₄, R₅, R₆, R₇ and R₈ are as defined above for a compound of formula (I), with a compound of formula (III): 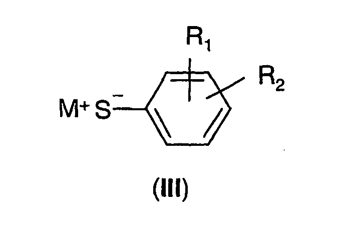 wherein M is selected from alkali or alkaline-earth metals, R₁ and R₂ are as defined above for a compound of formula (I), to give a compound of formula (XVI): 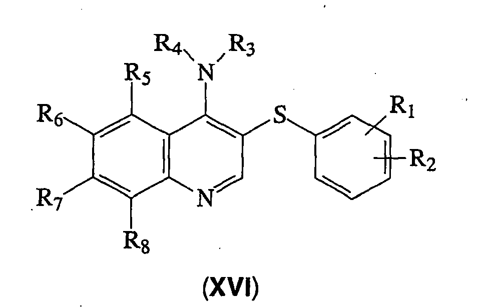 wherein R₁, R₂, R₃, R₄, R₅, R₆, R7 and R₈ are as defined above for a compound of formula (I), thereafter oxidizing a compound of formula (XVI) to obtain a compound of formula (XVII): 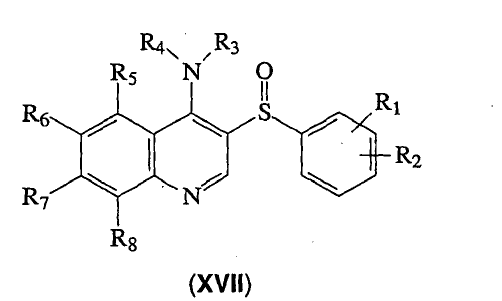 wherein R₁, R₂, R₃, R₄, R₅, R₆, R₇ and R₈ are as defined above for a compound of formula (I), thereafter oxidizing a compound of formula (XVII) to obtain a compound of formula (I), and optionally forming enantiomers and/or racemates and/or diastereomers and/or pharmaceutically acceptable salts thereof or
c.) interconverting one compound of formula (I), wherein the meaning of R₁, R₂, R₃, R₄, R₅, R₆, R₇ and R₈ is as defined above for the formula (I) to a different compound of formula (I), wherein the meaning of R₁, R₂, R₃, R₄, R₅, R₆, R₇ and R₈ is as described above for the formula (I);
   where appropriate, separating the enantiomers and/or racemates and/or diastereomers of compounds of formula (1), wherein the meaning of R₁, R₂, R₃, R₄, R₅, R₆, R₇ and R₈ is as described above for the formula (I) by conventional methods;
   and optionally thereafter forming salts and/or hydrates and/or solvates of compounds of formula (I).

A process for the preparation of a compound of formula (VI) wherein 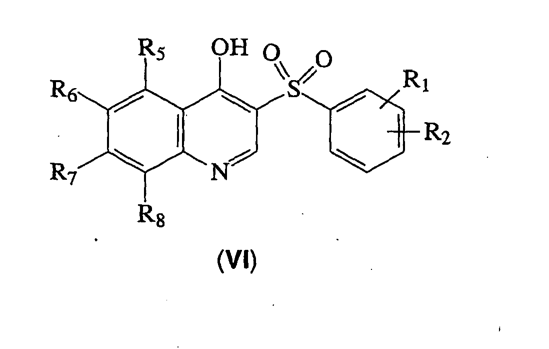 R₁ and R₂ are independently selected from the group consisting of hydrogen, halogen, alkyl, alkoxy, cyano, optionally substituted amino group or saturated heterocyclyl group, wherein the heteroatom is N;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.
a.) reacting a compound of formula (II): 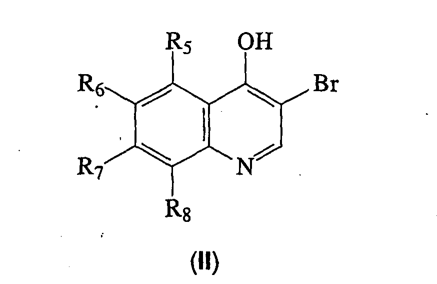 wherein R₅, R₆, R₇ and R₈ are as defined above for a compound of formula (I), with a compound of formula (III): 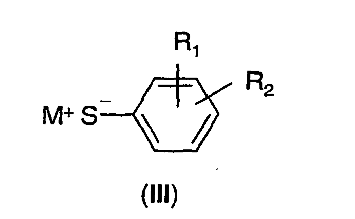 wherein M is selected from alkali metals or alkaline-earth metals, R₁ and R₂ are as defined above for a compound of formula (I), to give a compound of formula (IV): 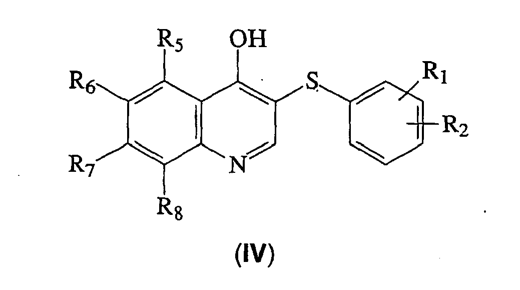 wherein R₁, R₂, R₅, R₆, R₇ and R₈ are as defined above for a compound of formula (I), thereafter oxidizing a compound of formula (IV) to obtain a compound of formula (V): 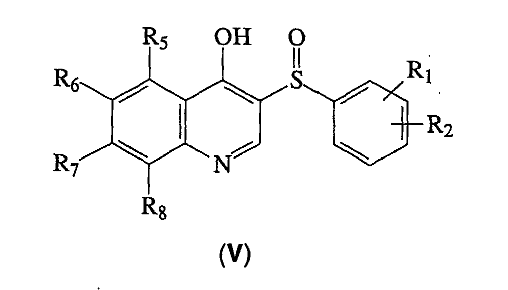 wherein R₁, R₂, R₅, R₆, R₇ and R₈ are as defined above for a compound of formula (I), thereafter oxidizing a compound of formula (V) to obtain a compound of formula (VI), and optionally forming enantiomers and/or racemates and/or diastereomers and/or pharmaceutically acceptable salts thereof or
b.) reacting a compound of formula (IX): 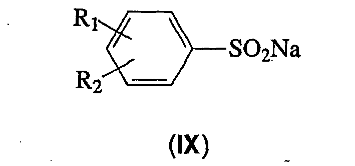 whererein R₁ and R₂ are as defined above for a compound of formula (I), with α-halogen-acetic acid esters of formula (X):

   Hlg-CH₂-COOR₉ (**X**)

   wherein Hlg is halogen and R₉ is an ethyl or methyl group, to obtain a compound of formula (XI): 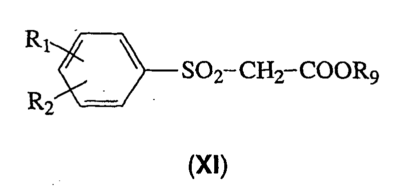
wherein R₁ and R₂ are as defined above for a compound of formula (I) and R₉ is as defined above for compounds of formula (X); reacting a compound of formula (XI) with a trialkyl orthoformate of formula (XII):

CH(OR₁₀)₃ (**XII**)

wherein R₁₀ is an ethyl or methyl group, to obtain a compound of formula (XIII): 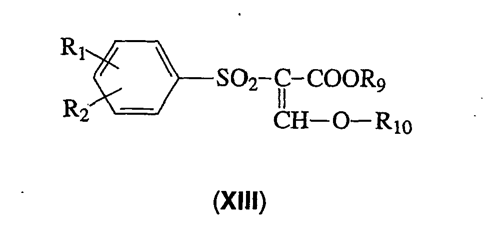 wherein R₁ and R₂ are as defined above for a compound of formula (I), R₉ is as defined above for compounds of formula (X) and R₁₀ is as defined above for compounds of formula (XII); reacting a compound of formula (XIII) with an aniline derivative of formula (XIV): 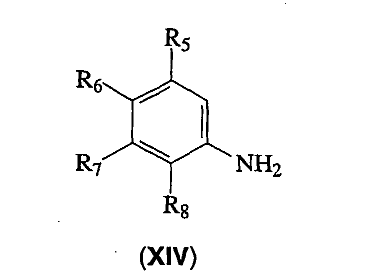 wherein R₅, R₆, R₇ and R₈ are as defined above for a compound of formula (I), to obtain a compound of formula (VI), and optionally forming enantiomers and/or racemates and/or diastereomers and/or pharmaceutically acceptable salts thereof.

According to Scheme la, 3-bromo-derivatives of formula (II) can be substituted by alkali or alkaline-earth metal salts (e.g. sodium salt) of thiophenols of formula (III) to provide a compound of formula (IV) *(*Bioorg. Med. Chem. Lett. 2001, 9, 1141-1144*).* Advantageously the substitution can be carried out by palladium catalyzation and under microwave conditions. To decrease the time of the reaction Pd-catalysis and/or microwave irradiation is required.

Oxidation of 3-arenesulfanyl-4-hydroxyquinolines of formula (IV) can be accomplished in a suitable acid (e.g.: trifluoroacetic acid) with hydrogen peroxid to give sulfoxides of formula (V) and sulfones of formula (VI), respectively.

Conversion of 4-hydroxyquinoline derivatives of formula (VI) to compounds of formula (VII) can be carried out by known halogenation methods with suitable reagents (e.g. POCl₃, SOCl₂, PCl₅, POBr₃, PBr₃) or by known acylation methods eg. with benzenenesulfonyl chloride or trifluoromethanesulfonis anhydride.

To obtain compounds of formula (VII) the reaction can also be accomplished by halogenation or acylation of 4-hydroxyquinoline derivatives of formula (V) and oxidation of the resulted compound by the known methods or according to the above mentioned methods.

Compounds of formula (I) can be prepared by the aromatic nucleophilic substitution reaction of compounds of formula (VII) with primary or secondary amines of formula (VIII).

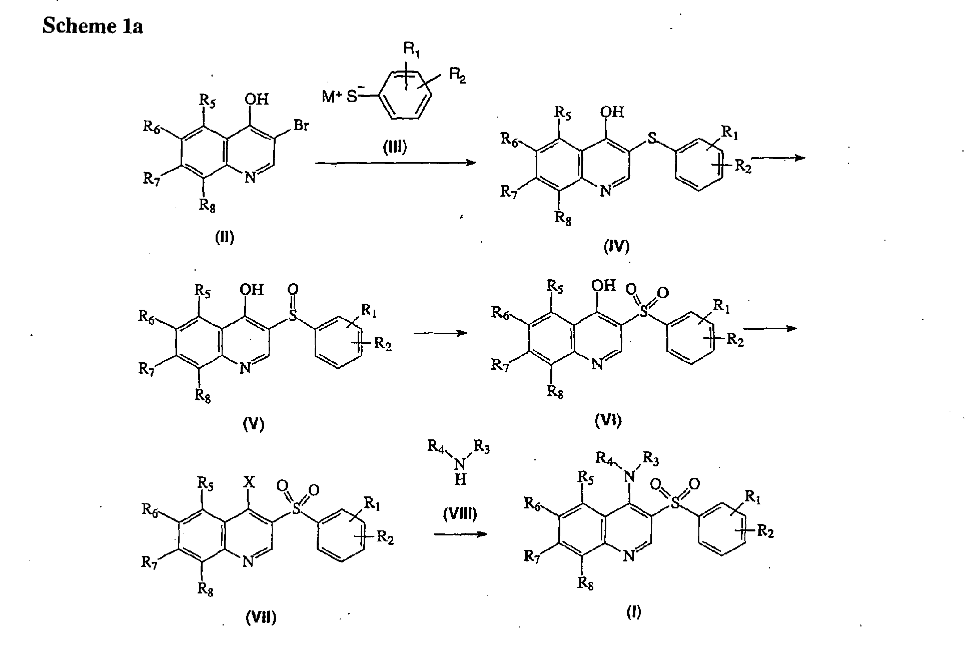

According to Scheme 1b another method to prepare intermediates of formula (I) via intermadiates of formula (VI) is to react compounds of formula (IX) with α-halogen-acetic acid esters of formula (X) in a suitable solvent (e.g. DMF, water). Compounds of formula (IX) can be purchased or can be prepared from the appropriate benzenesulfonyl chloride derivatives by known methods (e.g. Org. Lett. 2003, 5(21), 3895-3898*).* Compounds of formula (XIII) can be prepared by the reaction of compounds of formula (XI) and compounds of formula (XI) in the presence of acetic anhydride [J. Org. Chem. USSR (Engl. Transl., 1980, 16(7), 1275-1278*;* Zh. Org. Khim, 1980, 16(7), 1483-1487*].* Reaction of compounds of formula (XIII) with aniline derivatives of compounds of formula (XIV) gives benzenesulfonil-phenylamino-acrylic acid esters [e.g. J. Org. Chem. USSR (Engl. Transl., 1980, 16(7), 1275-1278*;* Zh. Org. Khim, 1980, 16(7), 1483-1487*]* that can *be in situ* converted into 4-hydroxy-quinoline derivatives of formula (VI) (see analogous reaction: J. Chem. Soc. Perkin Trans. 1, 1994, 4, 387-392*).* To obtain compounds of formula (I) the same preparation steps can be used as described above in scheme 1a.

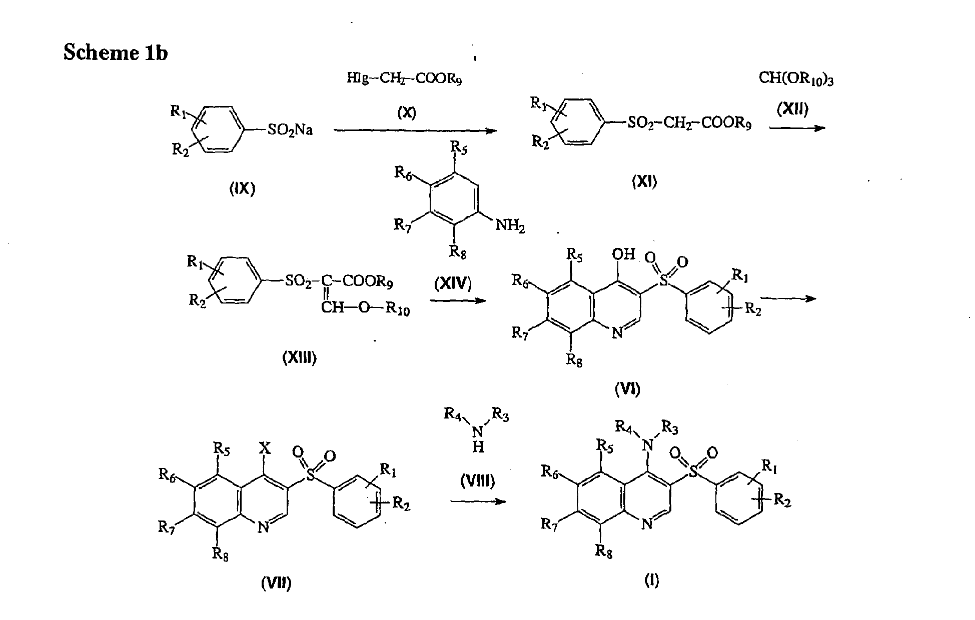

Compounds of formula (IV), compounds of formula (V), compounds of formula (VI) and compounds of formula (VII) and/or enantiomers and/or racemates and/or diastereomers and/or pharmaceutically acceptable salts thereof formed with acids or bases are new.

According to scheme 2 4-amino-3-bromoquinolines of formula (XV) can be prepared by known methods (J. Med. Chem. 2000, 43, 4667-4677*).* According to Scheme 2, 4-amino-3-bromoquinolines of formula (XV) can be substituted by e.g. sodium salts of thiophenols of formula (III) to provide compounds of formula (XVI). The aromatic nucleophilic reaction can be carried out advantageously by palladium catalyst and/or under microwave conditions.

Oxidation of 4-amino-3-arylsulfanylquinolines of formula (XVI) can be accomplished by known methods, preferably in a suitable acid (e.g. acetic acid) at 0-5 °C with potassium permanganate to give 4-amion-3-arylsulfinylquinolines of formula (XVII) or with aqueous hydrogen peroxid in a suitable acid (e.g.: acetic acid or trifluoroacetic acid).

To obtain compounds of formula (I) a further oxidation of a compound of formula (XVII) is required. The reaction can be carried out by known methods, preferably in a suitable acid (e.g. acetic acid) at 0-5 °C with potassium permanganate.

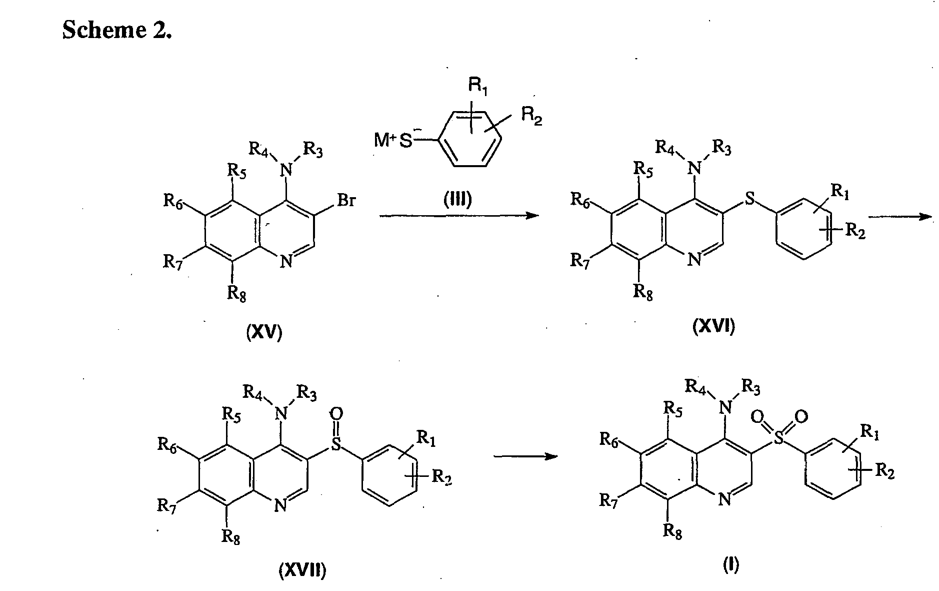

Compounds of formula (XVI) and compounds of formula (XVII) and/or enantiomers and/or racemates and/or diastereomers and/or pharmaceutically acceptable salts thereof formed with acids or bases are new.

Certain compounds of formula (I) can exist as enantiomers and racemates and diastereomers, too. These stereoisomers can optionally be separated, e.g. by chiral column chromatography or by crystallization (in the case of diastereomers).

The pure enantiomers and/or racemates and/or diastereomers of compounds of formula (I) can be prepared from stereochemically and/or geometrically pure precursors, too.

Compounds of formula (I) containing basic function(s) can be transformed into the salts thereof with acids and/or can be liberated from the obtained acid addition salts by treatment with a base.

Compounds of formula (I) can be transformed into hydrates and/or solvates.

The compounds of formula (I) can optionally be intercoverted to a different compound of formula (I) by conventional synthetic methods.

### Biological test methods

### mGluR5 receptor binding test

The mGluR5 receptor binding was determined according to the modified method of Gasparini et.al. (Bioorg. Med. Chem. Lett., 2000, 12, 407)*.* Based on the high homology between the human and rat mGluR5 receptors, rat cerebro-cortical membrane preparation was used to determine the binding characteristics of the reference compounds and novel compounds to the rat mGluR5. The A18 cell line expressing hmGluR5a (purchased from Euroscreen) was used to determine binding characteristics of the chemical compounds to the human mGluR5a receptor. [³H]-M-MPEP (2 nM) was used as radioligand. The nonspecific binding was determined in the presence of 10 µM M-MPEP.

### Assessment of functional activity

### Cell cultures for native rat mGluR5 receptors

Functional potency at native rat mGluR5 receptors was estimated using primary neocortical cell cultures derived from 17 day old Charles River rat embryos (for the details on the preparation of neural cell cultures see Johnson, M.I.; Bunge, R.P. (1992): Primary cell cultures of peripheral and central neurons and glia. In: Protocols for Neural Cell Culture, eds: Fedoroff, S.; Richardson A., The Humana Press Inc., 51-77*).* After isolation the cells were plated onto standard 96-well microplates and the cultures were maintained in an atmosphere of 95% air-5% CO₂ at 37 °C. The neocortical cultures were used for the calcium measurements after 5-7 days in vitro.

### Cell cultures for recombinant human mGluR5a receptors

Chinese hamster ovary (CHO) cells stably expressing recombinant human mGluR5a (CHO-mGluR5a, Euroscreen) receptors were cultured in F12 medium containing 10% FCS, 1 % antibiotic antimycotic solution, 400 µg/ml G418, 250 µg/ml zeocin, 5 µg/ml puromycin. Cells were kept at 37 °C in a humidified incubator in an atmosphere of 5% CO₂/95% air and were passaged three times a week. Cells were plated at 2.5-3.5×104 cell/well on standard 96-well microplates, receptor expression was induced by adding 600 ng/ml doxycycline on the next day. The calcium measurements were carried out 16-24 hours after the addition of the inducing agent.

### Fluorimetric measurement of cytosolic calcium concentration

Measurements of cytosolic calcium concentration ([Ca²⁺]_{¡}) were carried out on primary neocortical cell cultures and on CHO-mGluR5a cells stably expressing human mGluR5a receptors. Cells were grown in standard 96-well microplates and before the measurement were loaded with a fluorescent Ca²⁺-sensitive dye, fluo-4/AM (2 µM): the neural cultures were loaded in their growth medium, CHO-mGluR5a cells were loaded in assay buffer (145 mM NaCl, 5 mM KCI, 2 mM MgCl₂, 2 mM CaCl₂,10 mM HEPES, 20 mM D-glucose, 2 mM probenecid, pH=7.4) supplemented with 2 mM Na-pyruvate and 30 µg/ml glutamate-pyruvate transaminase (in case of CHO-mGluR5a cells these supplements were also present during the course of the [Ca²⁺]ᵢ measurements). Loading was done by incubating the cells with 100 µl/well dye solution at 37 °C in a humidified incubator in an atmosphere of 5% CO₂/95% air for 40-120 min. To stop dye loading cells were washed twice with assay buffer. After washing, various concentrations of the test compounds (diluted in assay buffer from a DMSO or a dimethylformamide (DMF) stock solution, final DMSO/DMF concentration was <0.1%) or buffer were added to each well depending on the experimental setup. In the case of neocortical cultures the assay buffer also contained TTX (0.5 µM, to suppress spontaneous oscillations of [Ca²⁺]¡.

After incubation at 37 °C for 10-20 minutes baseline and agonist-evoked changes of [Ca²⁺]¡ were measured column by column with a plate reader fluorimeter (FlexStation II, Molecular Devices). Excitation and detection of emission was carried out from the bottom of the plate. The whole measurement process was performed at 37 °C and was controlled by custom software. Inhibitory potency of the test compounds was assessed by measuring the reduction in the agonist-evoked [Ca²⁺]ᵢ -elevation in the presence of different concentrations of the compounds. DHPG was used as agonist for both cultures, the concentration was 20 µM for the neocortical cultures. In the case of CHO-mGluR5a cells DHPG was applied at an EC80 concentration, the EC80-values were derived from daily determined dose-response curves.

Fluorescence data were expressed as AF/F (fluorescence change normalized to baseline). All treatments on a single plate were measured in multiple wells. Data from all wells with the same treatment were averaged and the average values were used for analysis. Inhibitory potency of a compound at a single concentration point was expressed as percent inhibition of the control agonist response. Sigmoidal concentration-inhibition curves were fitted to the data (derived from at least three independent experiments) and IC₅₀-values were determined as the concentration that produces half of the maximal inhibition caused by the compound. Raw fluorescence data were analyzed using Soft Max Pro (Molecular Devices), curve fitting was done with GraphPad Prism.

### Results

Compounds of formula (I) of the present invention showed affinity for both rat and human mGluR5 receptors and proved to be functional antagonists, that is they inhibited functional responses elicited by stimulation of mGluR5 receptors.

The invention is further illustrated by the following non-limiting examples.

Unless specifically stated otherwise, all operation were carried out at room temperature, that is at a temperature range of 18-25 °C. The course of reactions was followed by thin layer chromatography (TLC) and reaction times are given for illustration only. The structure of all intermediates and end products were elucidated by IR, NMR and MS spectroscopy. When given yields are for illustration only. When given, NMR data are in the form of delta (δ) values for major diagnostic protons, given in parts per million (ppm) relative to tetramethylsilane (TMS) as internal standard, using the indicated solvent. Conventional abbreviations are used for signal shape.

### Examples

All starting materials are either commercially available or can be synthesized by different known methods described in the literature.

### Example 1

### 3-(3,4-Dimethyl-benzenesulfonyl)-4-(morpholin-4-yl)-quinoline

### Table I compound 1

### 4-(Morpholin-4-yl)-quinoline

A mixture of morpholine (0.86 ml, 9.9 mmol) and 4-chloroquinoline (0.74 g, 4.5 mmol) was heated up to 195 °C in a microwave reactor during 60 seconds. The reaction mixture was cooled to 150 °C and it was kept at the same temperature for 10 minutes. The mixture was dissolved in dichloromethane (40 ml), extracted with water (5 X 40 ml) and the solvent was evaporated in vacuo to obtain the title compound.
Yield.: 94%.
¹H NMR (300 MHz, DMSO-*d₆*, 30°C): 3.19-3.27 (m, 4H); 3.95-4.04 (m, 4H); 7.02 (d, 1H, *J* = 5.0 Hz); 7.49 (ddd, 1H, *J* = 8.4, 6.9, 1.3 Hz); 7.67 (ddd, 1H, *J* = 8.4, 6.9, 1.5 Hz); 8.03 (ddm, 1H, *J* = 8.4, 1.5 Hz); 8.07 (ddm, 1H, *J* = 8.4, 1.3 Hz); 8.76 (d, 1H, J= 5.0 Hz). MS (EI) M⁺= 214.

### 3-Bromo-4-(morpholin-4-yl)-quinoline

4-(Morpholin-4-yl)-quinoline (2.8 g, 13 mmol) was dissolved in a mixture of acetic acid (26 mL) and dichloromethane (9 ml) at 0-5 °C. A solution of bromine (0.7 ml, 13.6. mmol) in a mixture of dichloromethane (3.5 ml) and acetic acid (7.0 ml) was added dropwise for 3 hours. The reaction mixture was stirred for 2 hours at 0-5 °C. The residue was dissolved in ethyl acetate (200 ml), extracted with sodium carbonate (170 ml, 5 % m/m) and dried over anhydrous sodium sulfate. The solvent was evaporated, and the crude product (3.5 g) was purified by gradient silica gel flash chromatography (300 g silica gel, eluent A: n-hexane:chloroform = 3:7, eluent B:chloroform) to give the title compound.
Yield.: 74%.
¹H NMR (300 MHz, DMSO-*d₆*, 30°C): 3.20-3.57 (brm, 4H); 3.80-3.91 (m, 4H); 7.65 (ddd, 1H, *J* = 8.4, 6.9, 1.4 Hz); 7.79 (ddd, 1H, *J =* 8.4, 6.9, 1.4 Hz); 8.01 (ddm, 1H, *J* = 8.4, 1.4 Hz); 8.29 (ddm, 1H, *J* = 8.4, 1.4 Hz); 8.84 (s, 1 H). MS (EI): M⁺ = 292 (⁷⁹Br)

### 3-(3,4-Dimethyl-benzenesulfanyl)-4-(morpholin-4-yl)-quinoline hydrochloride

A mixture of 3-bromo-4-(morpholin-4-yl)-quinoline (293 mg, 1 mmol), 3,4-dimethylthiophenol (0.27 ml, 1.95 mmol), sodium-*tert*-butylate (187 mg, 1.95 mmol), tetrakis-(triphenylphosphin)palladium (230 mg, 0.2 mmol) and *tert*-butanol (0.7 ml) or DMF (0.7 ml) was heated in a microwave reactor at 143 °C for 2 hours. The solvent was evaporated in vacuo and the residue was dissolved in dichloromethane, extracted with water, dried over anhydrous sodium sulphate and the solvent was removed in vacuo. The residue was purified by gradient flash chromatography (40g silicagele, eluent A: n-hexane:chloroform = 3:7, eluent B:chloroform, flow rate: 40 ml/min) to give the crude product. The crude product was dissolved in ethyl acetate (5 ml) and a solution of hydrogen chloride in ethyl acetate (0.5 ml, c = 1.6 M, 0.8 mmol) was added dropwise. The precipitate was filtered off, washed with ethyl acetate and dried in vacuo to give the title compound.
Yield.: 64%.
¹H NMR (300 MHz, DMSO-*d₆*, 30 °C): 2.18 (s, 3H); 2.19 (s, 3H); 3.70-3.87 (m, 8H); 6.98 (dd, 1H, J = 7.9, 2.1 Hz); 7.09-7.17 (m, 2H); 7.78 (ddd, 1H, *J* = 8.6, 7.0, 1.1 Hz); 8.01 (ddd, 1H, *J* = 8.5, 7.0, 1.1 Hz); 8.23 (dd, 1H, *J* = 8.5, 1.1 Hz); 8.30 (dd, 1H, J = 8.6, 1.1 Hz); 8.74 (s, 1H).
MS (EI): M⁺ = 350

### 3-(3,4-Dimethyl-benzenesulfinyl)-4-(morpholin-4-yl)-quinoline hydrochloride

3-(3,4-Dimethyl-benzenesulfanyl)-4-(morpholin-4-yl)-quinoline hydrochloride (280 mg, 0.8 mmol) was dissolved in a mixture of acetic acid (6 ml) and water (22 ml) at 0-5 °C. To the mixture a solution of potassium permanganate in water (c = 0.1 M, 1.65 ml, 0.165 mmol) was added dropwise for 3 hours at 0-5 °C. The reaction mixture was stirred for 1 hour at 0-5 °C and water (30 ml), dichloromethane (30 ml) and potassium carbonate (= 10 g) was added to the solution. The aqueous phase was extracted with dichloromethane (2x40 ml) and the organic layer was dried over anhydrous sodium sulfate. The solvent was evaporated and the crude product was purified by gradient flash chromatography (100 g silicagele, eluent A: n-hexane:chloroform = 3:7, eluent B:chloroform). The purified product was dissolved in ethyl acetate (1 ml) and a solution of hydrogen chloride in ethyl acetate (0.4 ml, c = 1.6 M, 0.64 mmol) was added. The precipitate was filtered off, washed with ethyl acetate and dried in vacuo to give the title compound.
Yield.: 68 %.
¹H NMR (300 MHz, DMSO-*d₆*, 30°C): 2.26 (s, 6H); 3.31-3.44 (m, 2H); 3.61-3.72 (m, 2H); 3.72-3.83 (m, 2H); 3.84-3.95 (m, 2H); 7.34 (d, 1H, *J =* 7.8 Hz); 7.43 (dd, 1H, *J =* 7.8,2.1 Hz); 7.54 (d, 1H, *J* = 2.1 Hz); 7.78 (ddd, 1H, *J* = 8.6, 7.0, 1.3 Hz); 8.02 (ddd, 1H, *J* = 8.5, 7.0, 1.3 Hz); 8.17-8.28 (m, 2H); 9.06 (s, 1H).
MS (EI): M⁺ = 366.

### 3-(3,4-Dimethyl-benzenesulfonyl)-4-(morpholin-4-yl)-quinoline

### Table I compound 1

3-(3,4-Dimethyl-benzenesulfinyl)-4-(morpholin-4-yl)-quinoline hydrochloride (322 mg, 0.8 mmol) was dissolved in a mixture of acetic acid (7 ml) and water (25 ml) at 0-5 °C. To the mixture a solution of potassium permanganate in water (c = 0.1 M, 4 ml. 4 mmol) was added dropwise for 4 hours at 0-5 °C. The reaction mixture was stirred for 1 hour at 0-5 °C, water (30 ml), dichloromethane (30 ml) and potassium carbonate (≅ 10 g) was added to the solution. The aqueous phase was extracted with dichloromethane (2x40 ml) and the organic layer was dried over anhydrous sodium sulfate. The solvent was evaporated and the crude product was purified by gradient flash chromatography (100 g silicagele, eluent A: n-hexane:chloroform = 3:7, eluent B:chloroform) to give the title compound.
Yield.: 38%.
¹H NMR (500 MHz, DMSO-*d₆*, 30°C): 2.28 (s, 3H); 2.30 (s, 3H); 3.05-3.13 (m, 4H); 3.45-3.52 (m, 4H); 7.39 (d, 1H, *J* = 7.9 Hz); 7.64 (dd, 1H, *J* = 7.9, 1.9 Hz); 7.67 (d, 1H, *J* = 1.9 Hz); 7.74 (ddd, 1H, *J* = 8.6, 6.9, 1.2 Hz); 7.96 (ddd, 1H, *J* = 8.5, 6.9, 1.2 Hz); 8.20 (dd, 1H, *J* = 8.5, 1.2 Hz); 8.32 (dd, 1H, *J*= 8.6, 1.2 Hz); 9.39 (s, 1H).
MS (FAB): [M+H]⁺ = 383

### 3-(3,4-Dimethyl-benzenesulfonyl)-4-(morpholin-4-yl)-quinoline hydrochloride

3-(3,4-Dimethyl-benzenesulfonyl)-4-(morpholin-4-yl)-quinoline (80 mg, 0.21 mmol) was dissolved in ethyl acetate (3 ml) and a solution of hydrogen chloride in ethyl acetate (c = 1.6 M, 0.28 ml, 0.45 mmol) was added dropwise to the solution. The solid was filtered off, washed with ethyl acetate and dried in vacuo to give the title compound.
Yield.: 86%.
¹H NMR (300 MHz, DMSO-*d₆*, 30°C): 2.29 (s, 3H); 2.30 (s, 3H); 3.13-3.23 (m, 4H); 3.48-3.57 (m, 4H); 7.40 (d, 1H, *J* = 8.0 Hz); 7.67 (dd, 1H, *J* = 8.0, 2.1 Hz); 7.70 (d, 1H, *J* = 2.1 Hz); 7.77 (ddd, 1H, *J =* 8.6, 6.9, 1.3 Hz); 7.99 (ddd, 1H, *J =* 8.5, 6.9, 1.3 Hz); 8.22 (dd, 1H, *J* = 8.5,1.3 Hz); 8.33 (dd, 1H, *J* = 8.6, 1.3 Hz); 9.39 (s, 1H).
MS (FAB): [M+H]⁺ = 383

### Example 2

### 3-(4-Methyl-benzenesulfonyl)-4-(4-methyl-piperidin-1-yl)-quinoline

### Table I compound 2

### 3-(4-Methyl benzenesulfanyl)-4-hydroxy-quinoline

A mixture of 3-bromo-4-hydroxyquinoline (0.448 g, 2 mmol; J. Am. Chem. Soc. 1946, 68, 1229-1231), 4-methylbenzenethiol (0.30 g, 2.4 mmol), tetrakis-(triphenylphosphin)palladium (0.115 g, 0.1 mmol), sodium-tert-butylate (0.23 g, 2.4 mmol) and DMF (2.0 ml) was stirred and irradiated at 142°C for 3 hours in a 8-ml microwave vial. The solvent was evaporated in vacuo and the residue was purified by gradient silica gel flash chromatography (80 g silica gel, eluent A: CHCl3, eluent B: CHCl3:MeOH = 95:5) to give the title compound.
Yield: 61%.
¹H NMR (500 MHz, DMSO-*d₆*, 25°C): 2.23 (s, 3H); 7.04-7.09 (m, 4H); 7.39 (ddd, 1H, *J* = 8.1, 7.0, 1,1 Hz); 7.61 (ddm, 1H, *J* = 8.4, 1.1 Hz); 7.70 (ddd, 1H, *J* = 8.4, 7.0, 1.5 Hz); 8.12 (ddm, 1H, *J* = 8.1,1.5 Hz); 8.30 (s, 1H); 12.23 (brs, 1H).
M⁺(EI) = 267

### 3-(4-Methyl-benzenesulfonyl)-4-quinoline

3-(4-Methyl-benzenesulfanyl)-4-hydroxyquinoline (0.25 g, 0.936 mmol) was dissolved in trifluoroacetic acid (5.0 ml) and a solution of hydrogen peroxide in trifluoroacetic acid (c = 3.0M, 3.7 ml) was added dropwise for 8 hours at room temperature. 6ml water was added dropwise to the reaction mixture. The precipitate was filtered off, washed with water and dried in vacuo to give the title compound.
Yield: 87%.
¹H NMR (500 MHz, DMSO-*d₆*, 25 °C): 2.37 (s, 3H); 7.34-7.40 (m, 2H); 7.44- (ddd, 1H, *J* = 8.1, 7.0, 1.2 Hz); 7.69 (ddm, 1H, *J* = 8.4, 1.2 Hz); 7.76 (ddd, 1H, *J* = 8.4, 7.0, 1.5 Hz); 7.88-7.95 (m, 2H); 8.05 (ddm, 1H, *J* = 8.1, 1.5 Hz); 8.74 (d, 1H, *J* = 6.8 Hz); 12.75 (d, 1H, J = 6.8 Hz).
MS (FAB): [M+H]⁺ = 300

### 4-Chloro-3-(4-methyl-benzenesulfonyl)-quinoline

A mixture of 3-(4-methyl-benzenesulfonyl)-4-hydroxyquinoline (0.24 g, 0.8 mmol) and phosphoryl chloride (15 ml) Was stirred at 135 °C for 5 hours. Phosphoryl chloride was distilled off, and the residue was poured onto ice. The slurry was stirred for 2 hours at 0-5 °C, neutralized with sodium carbonate and extracted with chloroform (50 ml). The organic layer was dried over anhydrous sodium sulphate, filtered and the solvent was removed in vacuo to give the title compound.
Yield: 91 %.
¹H NMR (500 MHz, CDCl₃, 25 °C): 2.42 (s, 3H); 7.31-7.36 (m, 2H); 7.72 (ddd, 1H, *J* = 8.6, 6.9, 1.1 Hz); 7.91 (ddd, 1H, *J* = 8.5, 6.9, 1.4 Hz); 7.92-7.96 (m, 2H); 8.21 (dm, 1H, *J* = 8.5 Hz); 8.33 (ddm, 1H, *J* = 8.6, 1.4 Hz); 9.63 (s, 1H).
MS (EI) [M+H]⁺ = 285 (³⁵Cl)

### 3-(4-Methyl-benzenesulfonyl)-4-(4-methyl-piperidin-1-yl)quinoline

### Table I compound 2

4-Chloro-3-(4-methyl-benzenesulfonyl)-quinoline (0.25 mmol, 79 mg) was dissolved in acetonitrile (0.5 ml) and 4-methylpiperidine (63 µL, 0.55 mmol) was added. The reaction mixture was stirred at 50 °C for three hours then it was stirred at room temperature overnight. The solvent was evaporated in vacuo and the residue was purified by flash chromatography (1.0 g silica gel, chloroform).
Yield: 87 %.
¹H NMR (500 MHz, DMSO-*d₆*, 25°C): 0.92 (d, 3H, *J* = 6.6 Hz); 0.92-1.04 (m, 2H); 1.38-1.47 (m, 2H); 1.47-1.61 (m, 1H); 2.39 (s, 3H); 2.83-2.93 (m, 2H); 3.26-3.34 (m, 2H); 7.39-7.45 (m, 2H); 7.71 (ddd, 1H, *J =* 8.6, 7.0, 1.2 Hz); 7.72-7.78 (m, 2H); 7.93 (ddd, 1H, *J =* 8.4, 7.0,1.2 Hz); 8.16 (dd, 1H, *J* = 8.4,1.2 Hz); 8.28 (dd, 1H, *J* = 8.6,1.2 Hz); 9.35 (s, 1H).
MS (EI): M⁺ = 380

### 3-(4-Methyl-benzenesulfonyl)-4-(4-methyl-piperidin-1-yl)-quinoline hydrochloride

3-(4-Methyl-benzenesulfonyl)-4-(4-methyl-piperidin-1-yl)-quinoline (40 mg, 0.105 mmol) was dissolved in ethyl acetate (0.5 ml) then a solution of hydrogen chloride in ethyl acetate (c = 1.6 M, 65 µl 0.105 mmol) was added dropwise to the solution. The solvent was removed in vacou to give the title compound.
Yield: 100%.
¹H NMR (500 MHz, DMSO-*d₆*, 25°C): 0.93 (d, 3H, *J* = 6.5 Hz); 1.08-1.20 (m, 2H); 1.48-1.57 (m, 2H); 1.57-1.68 (m, 1H); 2.41 (s, 3H); 3.19-3.29 (m, 2H); 3.32-3.42 (m, 2H); 7.42-7.49 (m, 2H); 7.77 (ddd, 1H, *J =* 8.6, 7.0, 1.2 Hz); 7.81-7.86 (m, 2H); 8.01 (ddd, 1H, *J =* 8.5, 7.0, 1.2 Hz); 8.19 (dd, 1H, *J* = 8.5, 1.2 Hz); 8.29 (dd, 1H, *J*= 8.6, 1.2 Hz); 9.37 (s, 1H).
MS (EI) M⁺= 380.

### Example 3

### 3-(3,4-Dimethyl-benzenesulfonyl)-4-(4-methyl-piperidin-1-yl)-quinoline

### Table I compound 3

### 3-(3,4-Dimethyl-benzenesulfanyl)-4-hydroxyquinoline

The title compound was prepared from 3-bromo-4-hydroxyquinoline and 3,4-dimethylthiophenol according to the method described in Example 2 for the preparation of 3-(4-Methyl-benzenesulfanyl)-4-hydroxyquinoline.
Yield: 58%.
¹H NMR (500 MHz, DMSO-*d₆*, 25°C): 2.14 (s, 3H); 2.15 (s, 3H); 6.90 (dd, 1H, *J* = 7.8, 2.0 Hz); 6.98-7.04 (m, 2H); 7.38 (ddd, 1H, *J* = 8.1, 7.0, 1.2 Hz); 7.60 (ddm, 1H, *J* = 8.4, 1.2 Hz); 7.69 (ddd, 1H, *J* = 8.4, 7.0, 1.5 Hz); 8.11 (ddm, 1H, *J* = 8.1, 1.5 Hz); 8.25 (s, 1H); 12.18 (brs, 1H).
MS (EI) M⁺ = 281.

### 3-(3,4-Dimethyl-benzenesulfonyl)-4-hydroxyquinoline

The title compound was prepared according to the method described in Example 2 for the preparation of 3-(4-Methyl-benzenesulfonyl)-4-hydroxyquinoline.
Yield: 89%.
¹H NMR (500 MHz, DMSO-*d₆*, 25 °C): 2.30 (s, 3H); 2.32 (s, 3H); 7.27 (d, 1H, *J* = 8.5 Hz); 7.39 (ddd, 1H, *J* = 8.1, 7.0, 1.2 Hz); 7.62 (ddm, 1H, *J* = 8.4, 1.2 Hz); 7.68 (ddd, 1H, *J* = 8.4, 7.0, 1.5 Hz); 7.78-7.82 (m, 2H); 8.15 (ddm, 1H, *J* = 8.1, 1.5 Hz); 8.66 (s, 1H); 12.63 (brs, 1H).
MS (FAB): [M+H]⁺ = 314

### 4-Chloro-3-(3,4-dimethylbenzenesulfonyl)-quinoline

The title compound was prepared according to the method described in Example 2 for the preparation of 4-Chloro-3-(4-methylbenzenesulfonyl)-quinoline.
Yield: 92%.
¹H NMR (500 MHz, CDCl₃, 25 °C): 2.32 (s, 3H); 2.33 (s, 3H); 7.32 (d, 1H, *J* = 8.1 Hz); 7.77 (d, 1H, *J* = 2.1 Hz); 7.82 (dd, 1H, *J* = 8.1, 2.1 Hz); 7.85 (ddd, 1H, *J* = 8.6, 7.0, 0.9 Hz); 8.05 (ddd, 1H, *J* = 8.5, 7.0,1.3 Hz); 8.39-8.46 (m, 2H); 9.66 (s, 1H).
MS (EI) M⁺= 331 (³⁵Cl)

### 3-(3,4-Dimethylbenzenesulfonyl)-4-(4-methyl-piperidin-1-yl)-quinoline

The title compound was prepared according to the method described in Example 2 for the preparation of compound 2 in Table I.
Yield: 87%.
¹H NMR (500 MHz, DMSO-*d₆*, 25 °C): 0.91 (d, 3H, *J* = 6.5 Hz); 0.92-1.03 (m, 2H); 1.38-1.46 (m, 2H); 1.47-1.61 (m, 1H); 2.29 (s, 3H); 2.30 (s, 3H); 2.82-2.91 (m, 2H); 3.28-3.36 (m, 2H); 7.36 (d, 1H, *J* = 8.1 Hz); 7.54 (dd, 1H, *J* = 8.1, 2.0 Hz); 7.67 (d, 1H, *J* = 2.0 Hz); 7.70 (ddd, 1H, *J* = 8.5, 6.9, 1.2 Hz); 7.93 (ddd, 1H, *J* = 8.5, 6.9, 1.2 Hz); 8.16 (dd, 1H, *J* = 8.5, 1.2 Hz); 8.27 (dd, 1H, *J* = 8.5,1.2 Hz); 9.35 (s, 1H).
MS (EI) M⁺= 394.

### 3-(3,4-Dimethyl-benzenesulfonyl)-4-(4-methyl-piperidin-1-yl)-quinoline hydrochloride

The title compound was prepared according to the method described in Example 2 for the preparation of the hydrochloride salt of compound 2 in Table I.
Yield: 100%.
¹H NMR (500 MHz, DMSO-*d₆*, 25°C): 0.93 (d, 3H, *J* = 6.5 Hz); 1.03-1.16 (m, 2H); 1.46-1.55 (m, 2H); 1.55-1.67 (m, 1H); 2.30 (s, 3H); 2.31 (s, 3H); 3.10-3.20 (m, 2H); 3.32-3.42 (m, 2H); 7.39 (d, 1H, *J* = 8.1 Hz); 7.61 (dd, 1H, *J* = 8.1, 2.0 Hz); 7.72 (d, 1H, *J* = 2.0 Hz); 7.75 (ddd, 1H, *J* = 8.6, 7.0, 1.2 Hz); 7.99 (ddd, 1H, *J* = 8.6, 7.0, 1.2 Hz); 8.18 (dd, 1H, *J* = 8.6, 1.2 Hz); 8.28 (dd, 1H, *J* = 8.6, 1.2 Hz); 9.36 (s, 1H).
MS (EI) M⁺= 394.

### Example 4

### 3-(4-Methyl-benzenesulfonyl)-4-(3-methyl-piperidin-1-yl)-quinoline

### Table I compound 4

The title compound was prepared from 4-chloro-3-(4-methyl-benzenesulfonyl)-quinoline according to the method described in Example 2 for the preparation of compound 2 in Table I.
Yield: 90%.
¹H NMR (500 MHz, DMSO-*d₆*, 25°C): 0.67 (d, 3H, *J* = 6.7 Hz); 0.99-1.11 (m, 1H); 1.28-1.41 (m, 2H); 1.41-1.52 (m, 1H); 1.63-1.74 (m, 1H); 2.39 (s, 3H); 2.70-2.79 (m, 1H); 2.81-2.96 (m, 2H); 3.20-3.29 (m, 1H); 7.39-7.44 (m, 2H); 7.71 (ddd, 1H, *J* = 8.5, 6.9, 1.2 Hz); 7.72-7.77 (m, 2H); 7.93 (ddd, 1H, *J* = 8.4, 6.9, 1.2 Hz); 8.16 (dd, 1H, *J* = 8.4, 1.2 Hz); 8.28 (dd, 1H, *J*= 8.5,1.2 Hz); 9.36 (s, 1H).
MS (EI) M⁺ = 380.

### Example 5

### 7-Chloro-3-(4-chloro-benzenesulfonyl)-4-(4-methyl-piperidin-1-yl)-quinoline

### Table I compound 195

### (4-Chloro-benzenesulfonyl)-acetic acid methyl ester

The mixture of methyl bromoacetate (11.25 ml, 116 mmol) sodium 4-chloro-benzenesulfinate (25.2g, 116 mmol) in DMF (120 ml) was stirred and heated at 80°C for 2 h. The solution was diluted with water (360 ml). The separated oil was extracted with chloroform (200 ml) and washed with water (3 X 80 ml). The organic phase was evaporated *in vacuo.* The yield was 22.4 g (77.7%).
MS (EI) M⁺ = 249.

Applying the above procedure the following compounds were prepared: e.g.: (3-chloro-benzenesulfonyl)-acetic acid methyl ester (MS (EI) M⁺ = 249); (3,4-dichloro-benzenesulfonyl)-acetic acid methyl ester (MS (EI) M⁺= 284); (4-methoxy-benzenesulfonyl)-acetic acid methyl ester (MS (EI) M⁺ = 245); (3-chloro-4-fluoro-benzenesulfonyl)-acetic acid methyl ester (MS (EI) M⁺ = 267).

### 2-(4-Chloro-benzenesulfonyl)-3-ethoxy-acrylic acid methyl ester

The mixture of (4-Chloro-benzenesulfonyl)-acetic acid methyl ester (22.4 g, 90 mmol), triethyl orthoformate (33.2 ml, 216 mmol) and acetic anhydride (19.1 ml, 203 mmol) was refluxed for 3 h with simultaneous distillation of ethanol, triethyl orthoformate and acetic anhydride, and then evaporated to dryness. The crude material (22.7 g, 82.8%) was used in the next step without purification.
MS (EI) M⁺ = 305.

Applying the above procedure the following compounds were prepared: e.g.: 2-(3-chloro-benzenesulfonyl)-3-ethoxy-acrylic acid methyl ester (MS (EI) M⁺ = 305); 2-(3-chloro-4-fluoro-benzenesulfonyl)-3-ethoxy-acrylic acid methyl ester (MS (EI) M⁺ = 323); 2-(3,4-dichloro-benzenesulfonyl)-3-ethoxy-acrylic acid methyl ester (MS (EI) M⁺ = 340); 2-(4-fluoro-benzenesulfonyl)-3-ethoxy-acrylic acid methyl ester (MS (EI) M⁺= 289).

### 7-Chloro-3-(4-chloro-benzenesulfonyl)-4-hydroxyquinoline

The mixture of 2-(4-Chloro-benzenesulfonyl)-3-ethoxy-acrylic acid methyl ester (7.62 g, 25 mmol) and 3-chloroaniline (3.19 g, 25 mmol) in diphenyl ether (20 ml) was heated at near reflux for 1 h. After cooling the precipitate was filtered and washed with ether. The yield was 4.25g (48.0%).
MS (EI) M⁺= 355.

Applying the above procedure the following compounds were prepared: e.g.: 8-chloro-3-(4-chloro-benzenesulfonyl)-4-hydroxyquinoline (MS (EI) M⁺ = 355); 6-chloro-3-(4-fluoro-benzenesulfonyl)-4-hydroxyquinoline (MS (EI) M⁺ = 338); 6-cyano-3-(4-fluoro-benzenesulfonyl)-4-hydroxyquinoline (MS (EI) M⁺ = 329); 8-chloro-3-(3,4-dichloro-benzenesulfonyl)-4-hydroxyquinoline (MS (EI) M⁺ = 390); 8-ethyl-3-(4-chlorobenzenesulfonyl)-4-hydroxyquinoline (MS (EI) M⁺= 348).

### 3-(4-chloro-benzenesulfonyl)-4,7-dichloroquinoline

7-Chloro-3-(4-chloro-benzenesulfonyl)-4-hydroxyquinoline (4.25 g, 12 mmol) in phosphorus(III) oxychloride (5.6 ml, 60 mmol) was refluxed for 3 h. The reaction mixture was poured into water (50 ml) and was alkalized with 5M sodium hydroxyde solution. After cooling the precipitate was filtered and washed with water. The yield was 3.8 g (85.0%).
MS (EI) M⁺= 373.

Applying the above procedure the following compounds were prepared: e.g.: 3-(3-chloro-benzenesulfonyl)-4,6-dichloroquinoline (MS (EI) M⁺ = 373); 3-(4-chlorobenzenesulfonyl)-4,8-dichloroquinoline (MS (EI) M⁺ = 373); 4-chloro-6-cyano-3-(4-fluoro-benzenesulfonyl)-quinoline (MS (EI) M⁺ = 347); 4-chloro-3-(4-chloro-benzenesulfonyl)-6-fluoro-quinoline (MS (EI) M⁺= 357).

### 7-Chloro-3-(4-chloro-benzenesulfonyl)-4-(4-methyl-piperidin-1-yl)-quinoline

### Table I compound 195

3-(4-Chloro-benzenesulfonyl)-4,7-dichloroquinoline (186 mg, 0.5 mmol), 4-methylpiperidine (71 µl, 0.6 mmol) and DBU (90 µl, 0.6 mmol) in DMF (6 ml) were stirred for 1 h at 80°C. After cooling, water (30 ml) was added, The solid was filtered and crystallized from methanol. The yield was 167 mg (76.6%).
MS (EI) M⁺ = 436.

Examples of compounds of formula (I) and their affinity for rat mGluR5 receptors are given in the table below.

**Table I**

| **Comp. No.** | **Structure** | **(M+H)⁺** | **Kᵢ(nM)** |
|---|---|---|---|
| 1 | 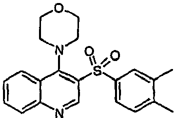 | 383 | *** |
| 2 | 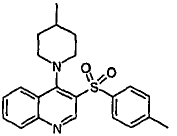 | 381 | *** |
| 3 | 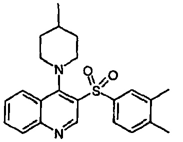 | 394 | *** |
| 4 | 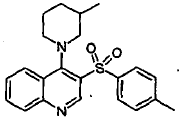 | 381 | *** |
| 5 | 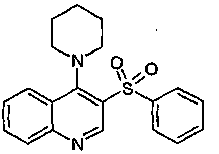 | 353 | ** |
| 6 | 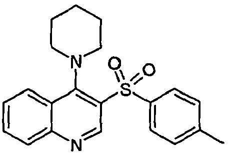 | 367 | *** |
| 7 | 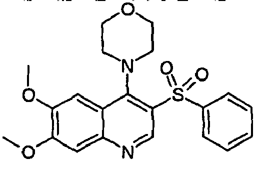 | 415 | * |
| 8 | 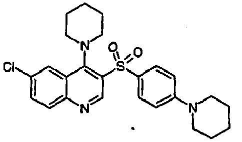 | 470 | * |
| 9 | 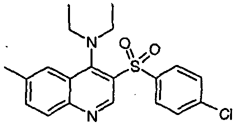 | 389 | *** |
| 10 | 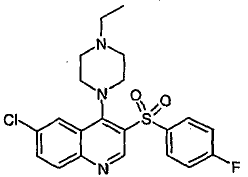 | 434 | * |
| 11 | 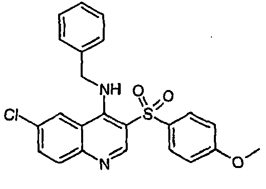 | 439 | * |
| 12 | 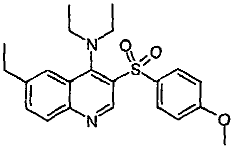 | 399 | * |
| 13 | 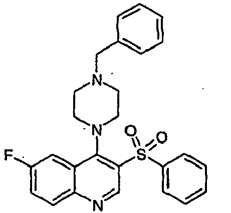 | 462 | * |
| 14 | 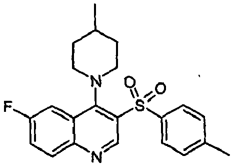 | 399 | ** |
| 15 | 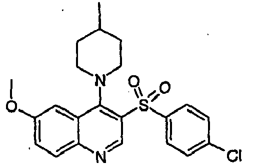 | 431 | * |
| 16 | 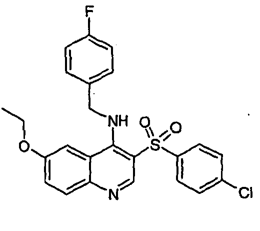 | 471 | *** |
| 17 | 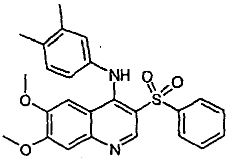 | 434 | * |
| 18 | 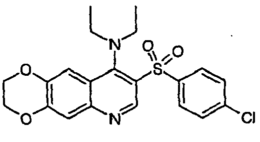 | 433 | * |
| 19 | 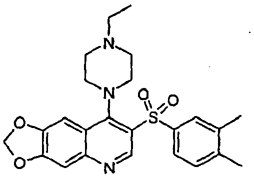 | 454 | * |
| 20 | 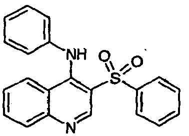 | 361 | * |
| 21 | 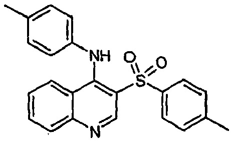 | 389 | * |
| 22 | 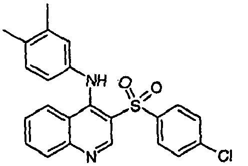 | 423 | * |
| 23 | 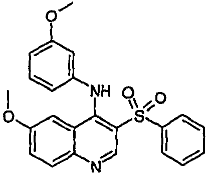 | 421 | * |
| 24 | 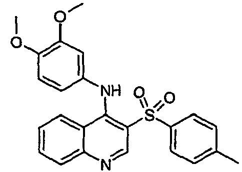 | 435 | * |
| 25 | 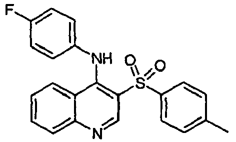 | 393 | * |
| 26 | 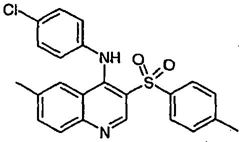 | 423 | * |
| 27 | 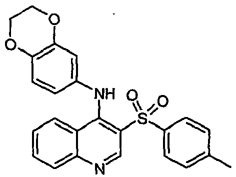 | 433 | * |
| 28 | 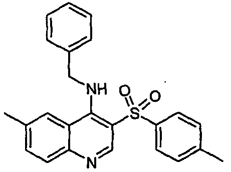 | 403 | * |
| 29 | 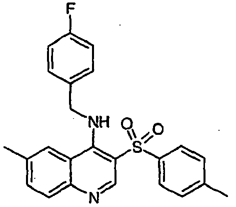 | 421 | * |
| 30 | 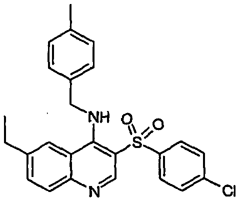 | 451 | * |
| 31 | 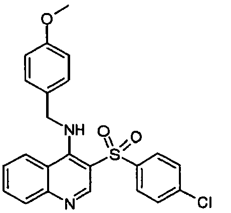 | 439 | * |
| 32 | 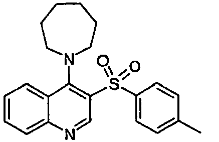 | 381 | *** |
| 33 | 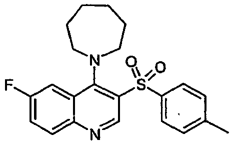 | 399 | * |
| 34 | 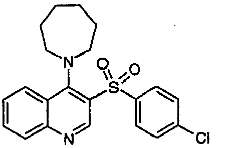 | 401 | ** |
| 35 | 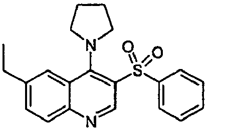 | 367 | * |
| 36 | 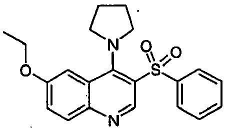 | 383 | * |
| 37 | 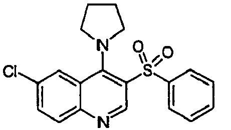 | 373 | * |
| 38 | 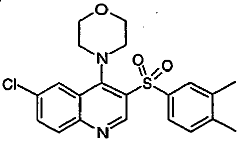 | 417 | * |
| 39 | 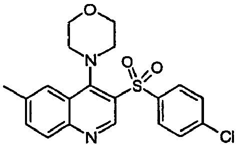 | 403 | * |
| 40 | 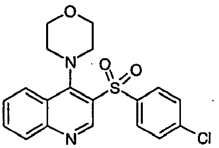 | 389 | * |
| 41 | 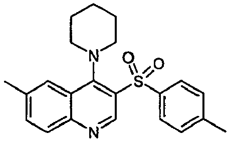 | 381 | * |
| 42 | 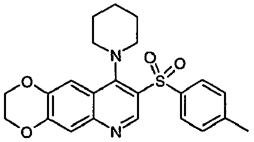 | 425 | * |
| 43 | 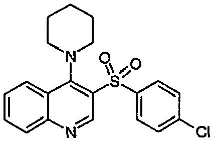 | 387 | * |
| 44 | 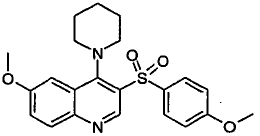 | 413 | * |
| 45 | 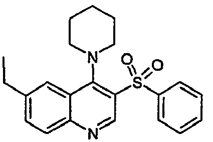 | 381 | * |
| 46 | 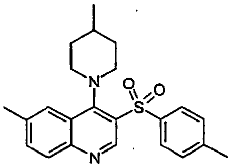 | 395 | *** |
| 47 | 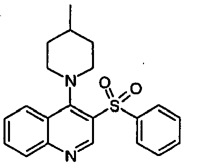 | 367 | *** |
| 48 | 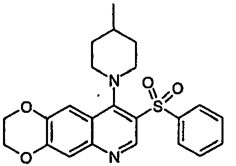 | 425 | *** |
| 49 | 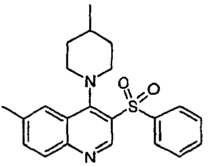 | 381 | * |
| 50 | 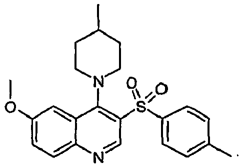 | 411 | * |
| 51 | 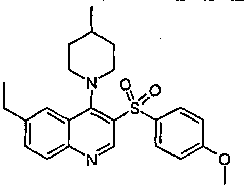 | 425 | *** |
| 52 | 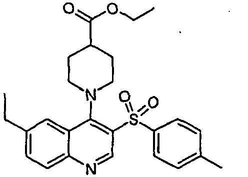 | 466 | * |
| 53 | 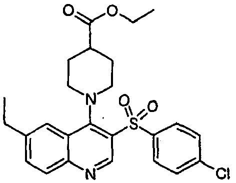 | 487 | *** |
| 54 | 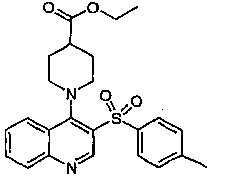 | 439 | * |
| 55 | 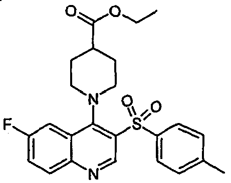 | 457 | * |
| 56 | 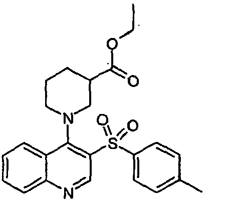 | 439 | * |
| 57 | 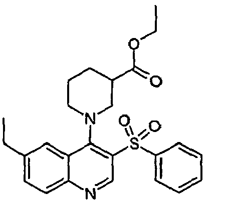 | 453 | * |
| 58 | 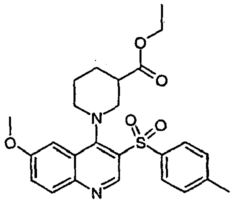 | 469 | * |
| 59 | 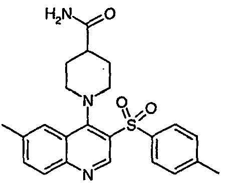 | 424 | * |
| 60 | 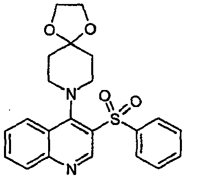 | 411 | * |
| 61 | 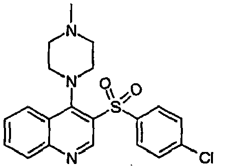 | 402 | * |
| 62 | 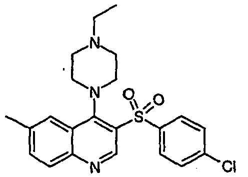 | 430 | * |
| 63 | 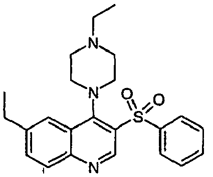 | 410 | * |
| 64 | 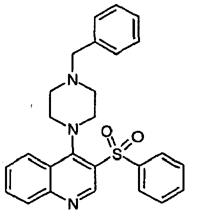 | 444 | * |
| 65 | 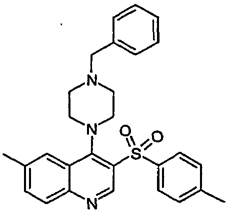 | 472 | * |
| 66 | 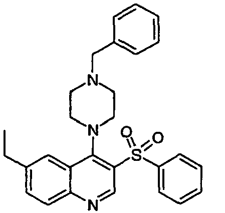 | 472 | * |
| 67 | 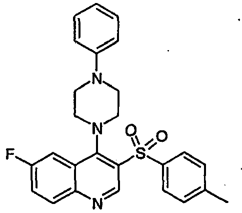 | 462 | * |
| 68 | 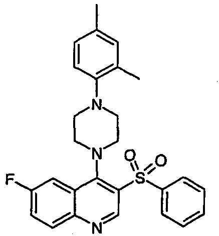 | 476 | * |
| 69 | 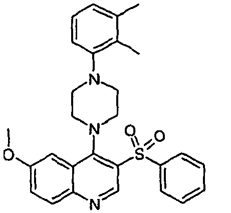 | 488 | * |
| 70 | 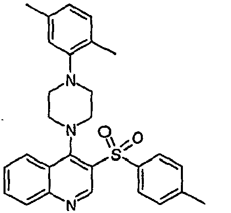 | 472 | * |
| 71 | 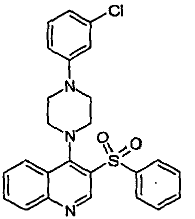 | 464 | * |
| 72 | 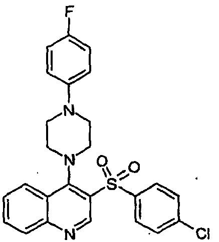 | 482 | * |
| 73 | 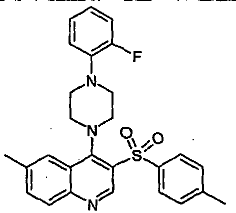 | 476 | * |
| 74 | 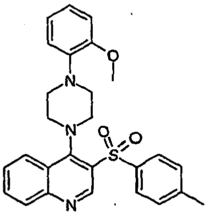 | 474 | * |
| 75 | 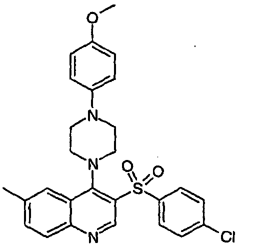 | 508 | * |
| 76 | 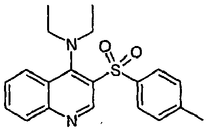 | 355 | ** |
| 77 | 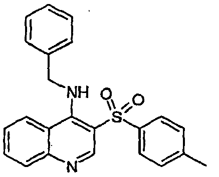 | 389 | *** |
| 78 | 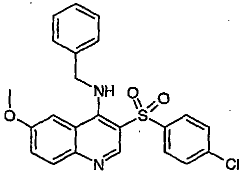 | 439 | * |
| 79 | 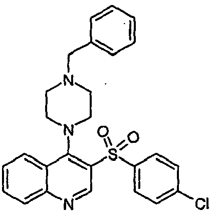 | 478 | ** |
| 80 | 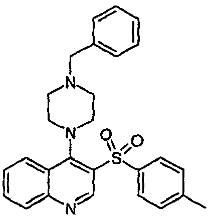 | 458 | * |
| 81 | 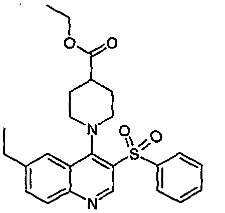 | 453 | * |
| 82 | 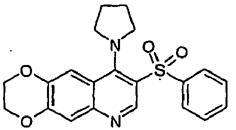 | 397 | * |
| 83 | 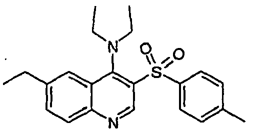 | 383 | * |
| 84 | 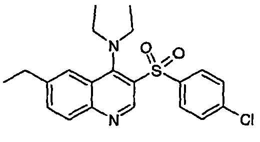 | 403 | * |
| 85 | 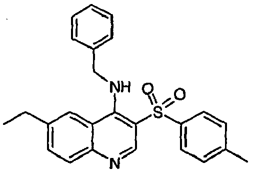 | 417 | * |
| 86 | 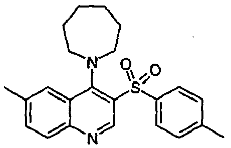 | 395 | * |
| 87 | 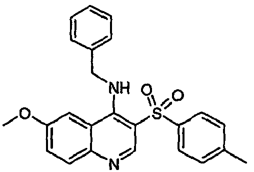 | 419 | * |
| 88 | 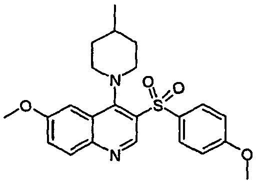 | 427 | * |
| 89 | 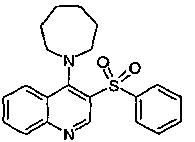 | 367 | ** |
| 90 | 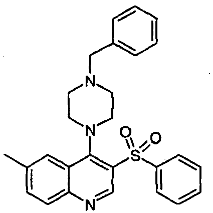 | 458 | * |
| 91 | 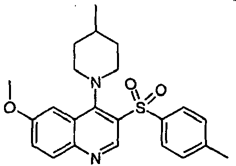 | 411 | * |
| 92 | 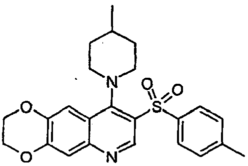 | 439 | * |
| 93 | 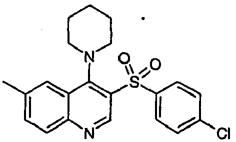 | 401 | * |
| 94 | 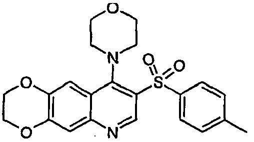 | 427 | * |
| 95 | 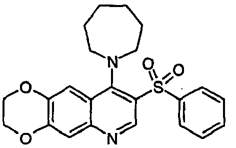 | 425 | * |
| 96 | 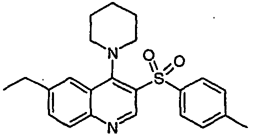 | 395 | * |
| 97 | 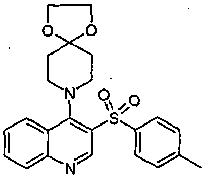 | 425 | * |
| 98 | 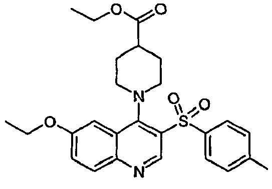 | 483 | * |
| 99 | 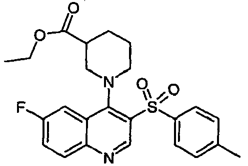 | 457 | * |
| 100 | 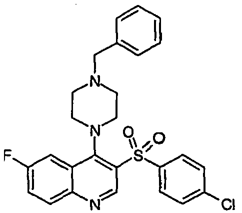 | 496 | * |
| 101 | 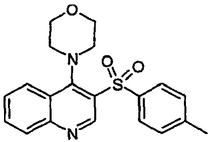 | 369 | * |
| 102 | 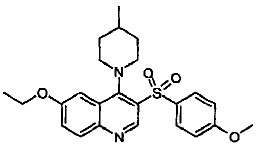 | 441 | * |
| 103 | 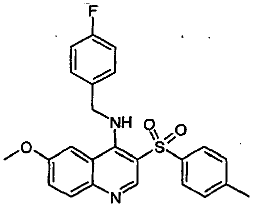 | 437 | * |
| 104 | 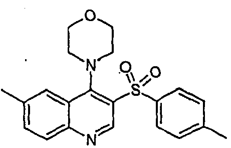 | 383 | * |
| 105 | 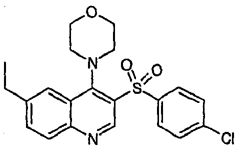 | 417 | * |
| 106 | 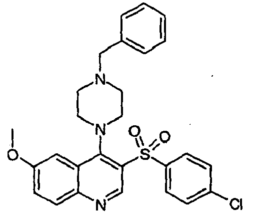 | 508 | * |
| 107 | 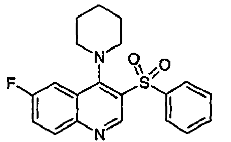 | 371 | * |
| 108 | 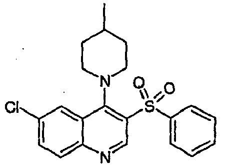 | 401 | * |
| 109 | 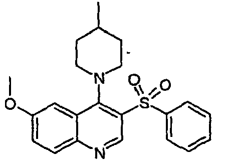 | 397 | * |
| 110 | 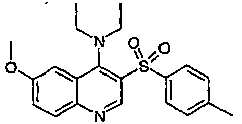 | 385 | * |
| 111 | 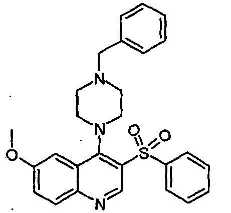 | 474 | * |
| 112 | 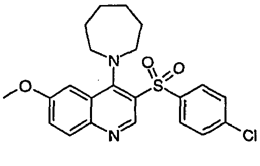 | 431 | * |
| 113 | 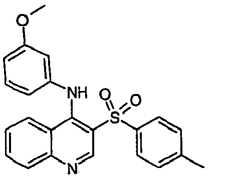 | 405 | * |
| 114 | 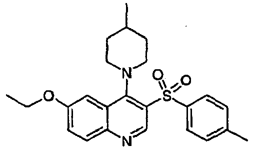 | 425 | * |
| 115 | 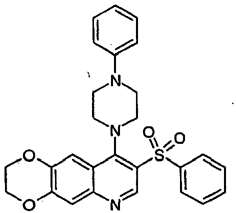 | 488 | * |
| 116 | 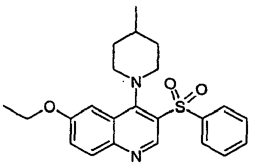 | 411 | * |
| 117 | 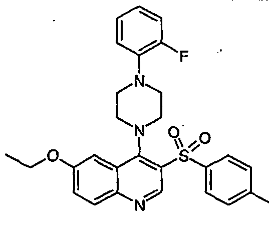 | 506 | * |
| 118 | 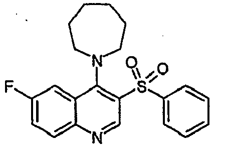 | 385 | * |
| 119 | 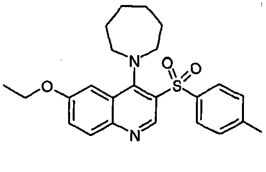 | 425 | * |
| 120 | 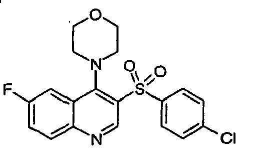 | 407 | * |
| 121 | 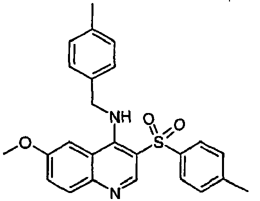 | 433 | * |
| 122 | 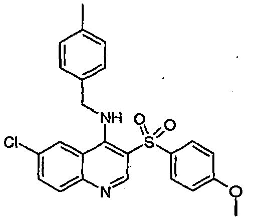 | 453 | * |
| 123 | 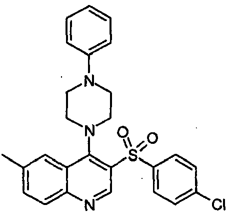 | 478 | * |
| 124 | 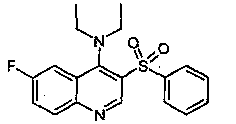 | 359 | * |
| 125 | 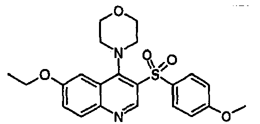 | 429 | * |
| 126 | 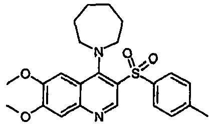 | 441 | * |
| 127 | 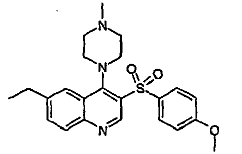 | 426 | * |
| 128 | 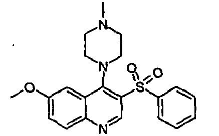 | 398 | * |
| 129 | 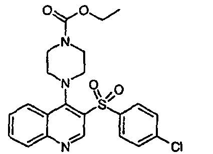 | 460 | * |
| 130 | 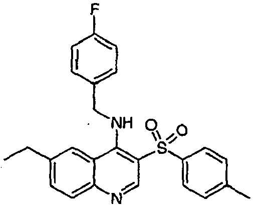 | 435 | * |
| 131 | 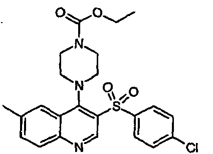 | 474 | ** |
| 132 | 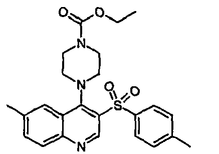 | 454 | * |
| 133 | 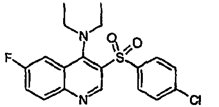 | 393 | * |
| 134 | 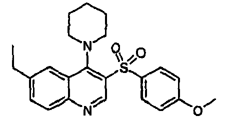 | 411 | * |
| 135 | 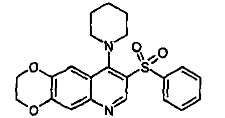 | 411 | * |
| 136 | 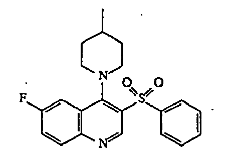 | 385 | * |
| 137 | 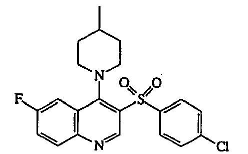 | 419 | *** |
| 138 | 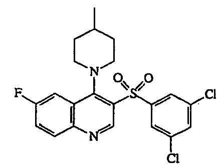 | 454 | ** |
| 139 | 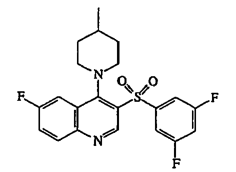 | 421 | * |
| 140 | 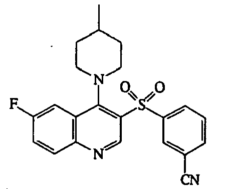 | 410 | *** |
| 141 | 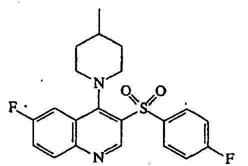 | 403 | *** |
| 142 | 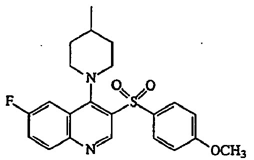 | 415 | *** |
| 143 | 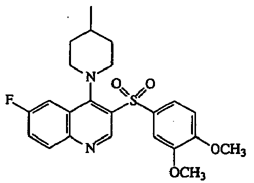 | 445 | ** |
| 144 | 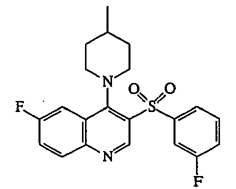 | 403 | ** |
| 145 | 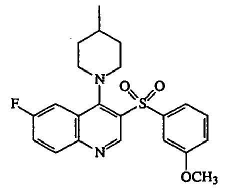 | 415 | *** |
| 146 | 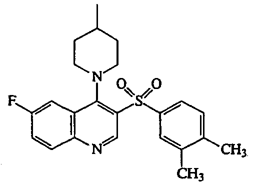 | 413 | *** |
| 147 | 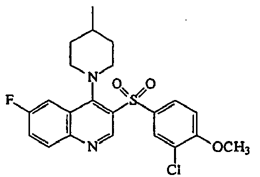 | 449 | *** |
| 148 | 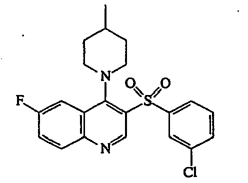 | 419 | *** |
| 149 | 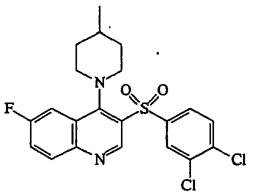 | 454 | *** |
| 150 | 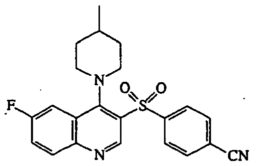 | 410 | ** |
| 151 | 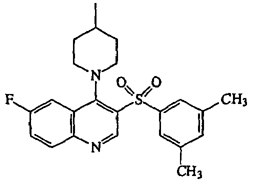 | 413 | *** |
| 152 | 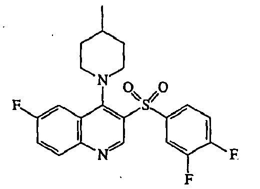 | 421 | *** |
| 153 | 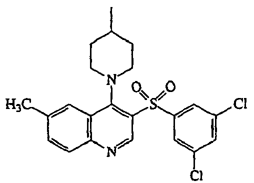 | 450 | ** |
| 154 | 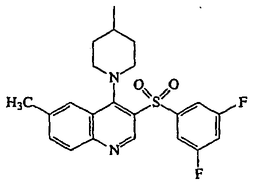 | 417 | ** |
| 155 | 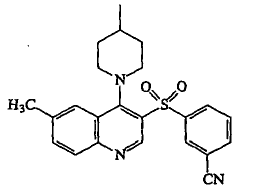 | 406 | * |
| 156 | 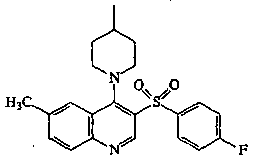 | 399 | *** |
| 157 | 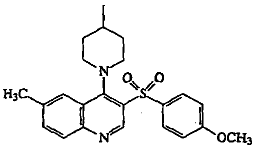 | 411 | ** |
| 158 | 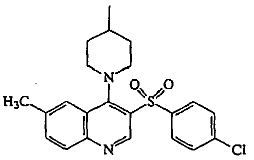 | 415 | ** |
| 159 | 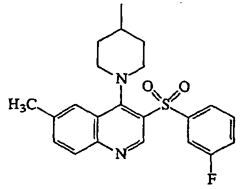 | 399 | *** |
| 160 | 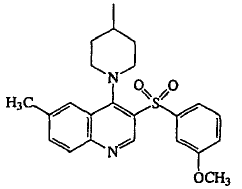 | 411 | *** |
| 161 | 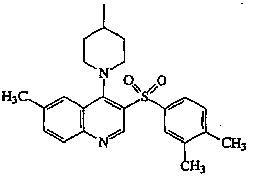 | 409 | *** |
| 162 | 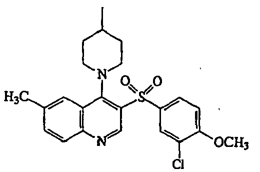 | 445 | *** |
| 163 | 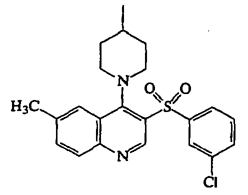 | 415 | *** |
| 164 | 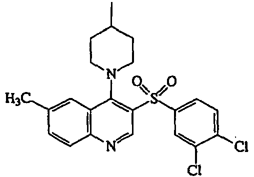 | 450 | *** |
| 165 | 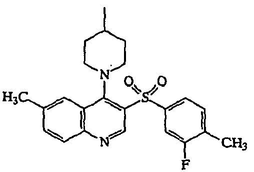 | 413 | *** |
| 166 | 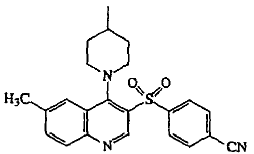 | 406 | ** |
| 167 | 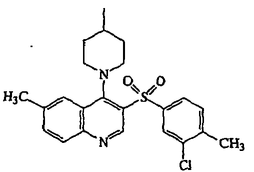 | 429 | *** |
| 168 | 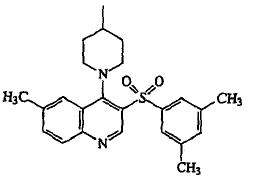 | 409 | ** |
| 169 | 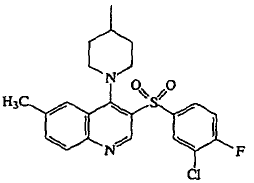 | 434 | *** |
| 170 | 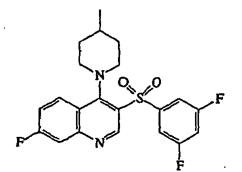 | 421 | ** |
| 171 | 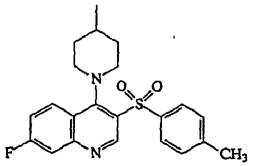 | 399 | *** |
| 172 | 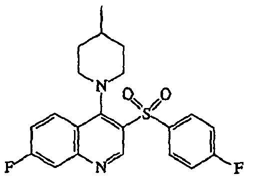 | 403 | ** |
| 173 | 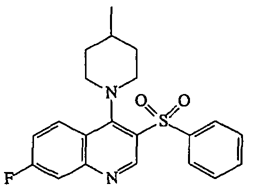 | 385 | *** |
| 174 | 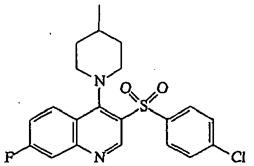 | 419 | *** |
| 175 | 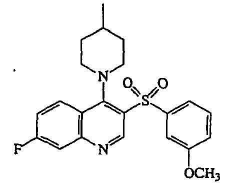 | 415 | *** |
| 176 | 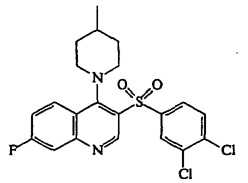 | 454 | *** |
| 177 | 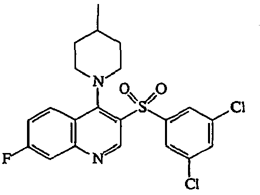 | 454 | *** |
| 178 | 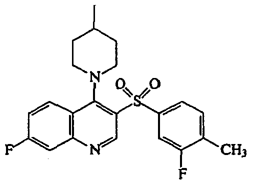 | 417 | *** |
| 179 | 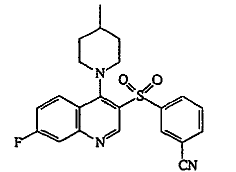 | 410 | *** |
| 180 | 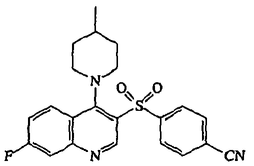 | 410 | *** |
| 181 | 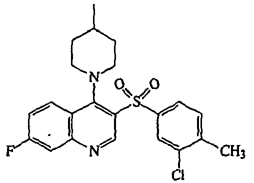 | 434 | *** |
| 182 | 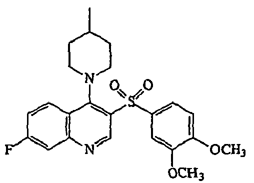 | 445 | *** |
| 183 | 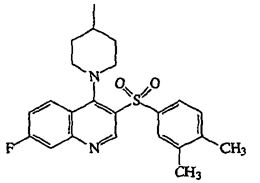 | 413 | *** |
| 184 | 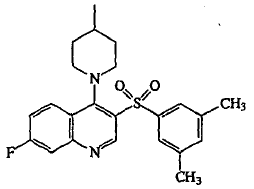 | 413 | ** |
| 185 | 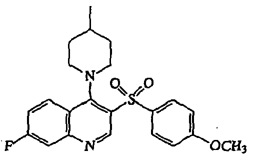 | 415 | *** |
| 186 | 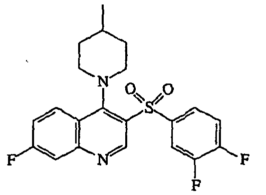 | 421 | *** |
| 187 | 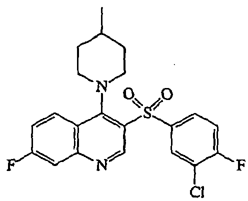 | 437 | *** |
| 188 | 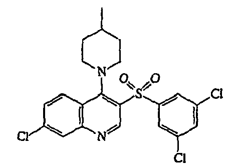 | 471 | *** |
| 189 | 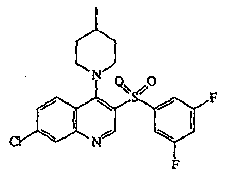 | 437 | *** |
| 190 | 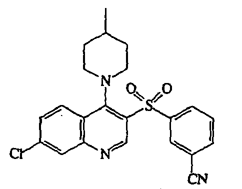 | 426 | *** |
| 191 | 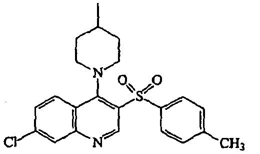 | 415 | *** |
| 192 | 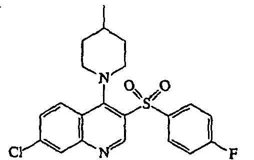 | 419 | *** |
| 193 | 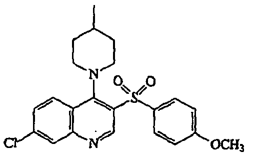 | 431 | ** |
| 194 | 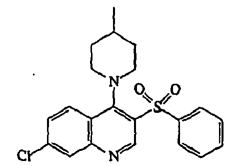 | 401 | *** |
| 195 | 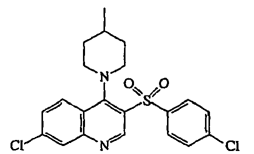 | 436 | *** |
| 196 | 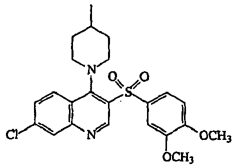 | 461 | **** |
| 197 | 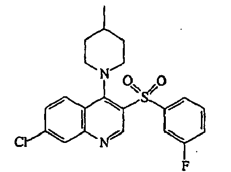 | 419 | *** |
| 198 | 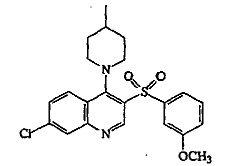 | 431 | *** |
| 199 | 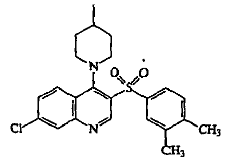 | 429 | *** |
| 200 | 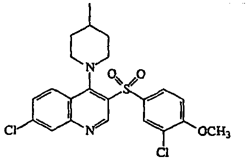 | 466 | *** |
| 201 | 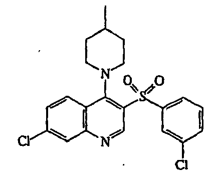 | 436 | *** |
| 202 | 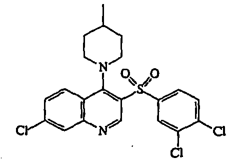 | 471 *** | |
| 203 | 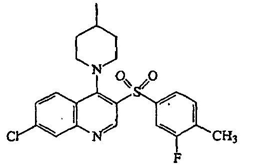 | 434 | *** |
| 204 | 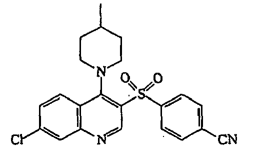 | 426 | ** |
| 205 | 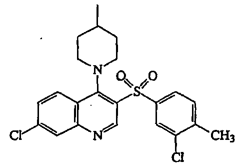 | 450 | *** |
| 206 | 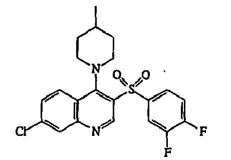 | 437 | *** |
| 207 | 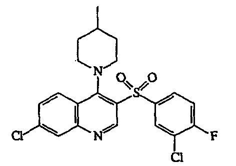 | 454 | *** |
| 208 | 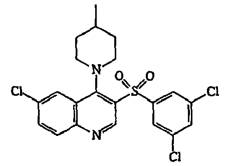 | 471 | *** |
| 209 | 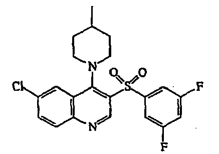 | 437 | *** |
| 210 | 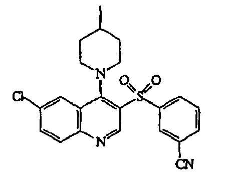 | 426 | *** |
| 211 | 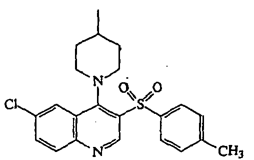 | 415 | *** |
| 212 | 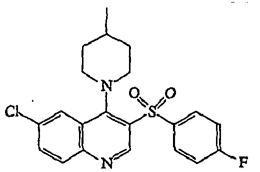 | 419 | *** |
| 213 | 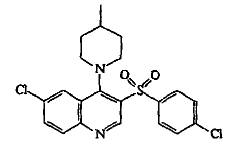 | 436 | *** |
| 214 | 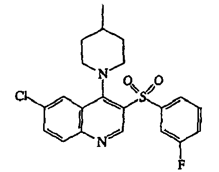 | 419 | ** |
| 215 | 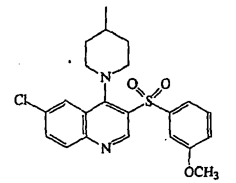 | 431 | **** |
| 216 | 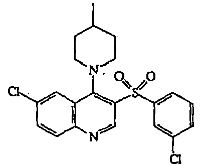 | 436 | * |
| 217 | 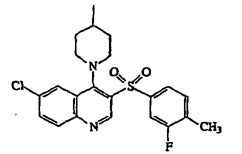 | 434 | ** |
| 218 | 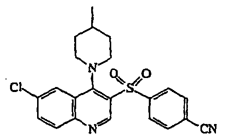 | 426 | * |
| 219 | 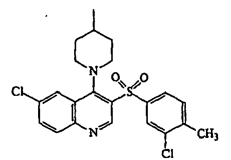 | 450 | ** |
| 220 | 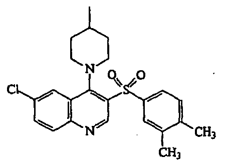 | 429 | *** |
| 221 | 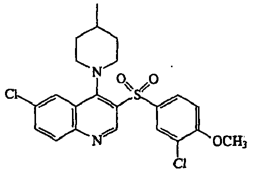 | 466 | * |
| 222 | 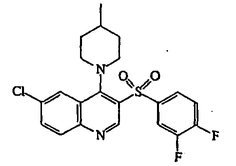 | 437 | ** |
| 223 | 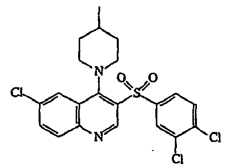 | 471 | *** |
| 224 | 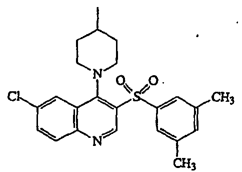 | 429 | * |
| 225 | 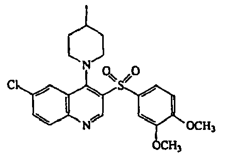 | 461 | ** |
| 226 | 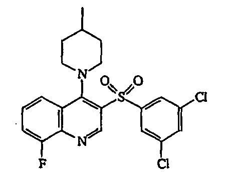 | 454 | *** |
| 227 | 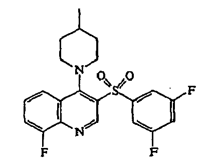 | 421 | * |
| 228 | 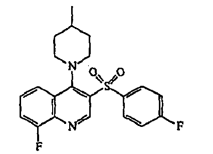 | 403 | * |
| 229 | 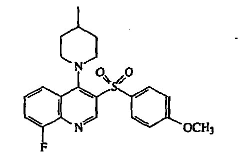 | 415 | ** |
| 230 | 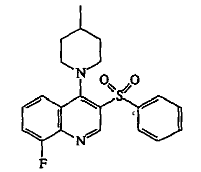 | 385 | * |
| 231 | 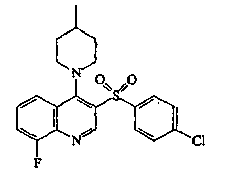 | 419 | *** |
| 232 | 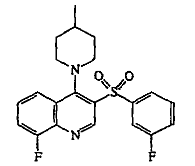 | 403 | ** |
| 233 | 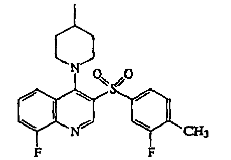 | 417 | *** |
| 234 | 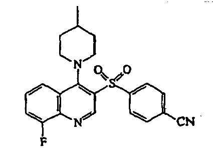 | 410 | *** |
| 235 | 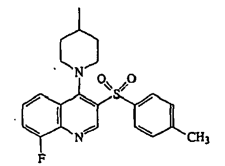 | 399 | *** |
| 236 | 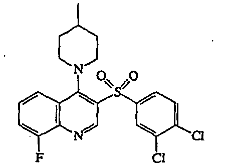 | 454 | *** |
| 237 | 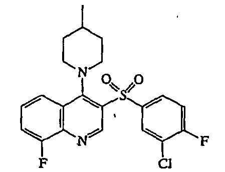 | 437 | *** |
| 238 | 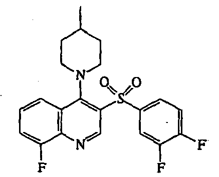 | 421 | *** |
| 239 | 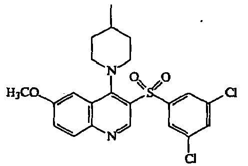 | 466 | *** |
| 240 | 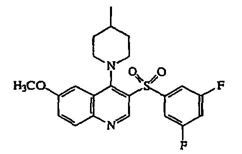 | 433 | * |
| 241 | 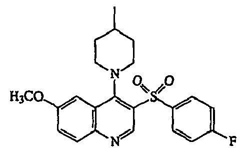 | 415 | * |
| 242 | 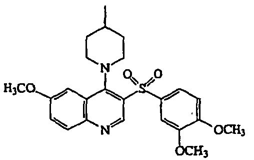 | 457 | * |
| 243 | 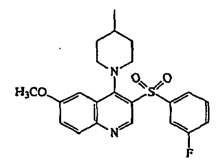 | 415 | ** |
| 244 | 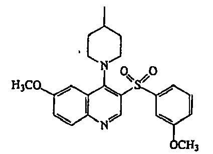 | 427 | * |
| 245 | 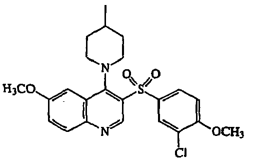 | 461 | * |
| 246 | 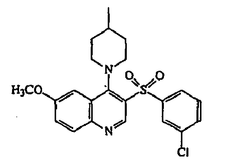 | 432 | * |
| 247 | 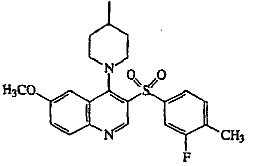 | 429 | ** |
| 248 | 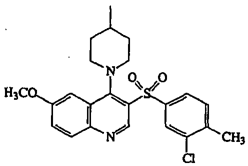 | 445 | *** |
| 249 | 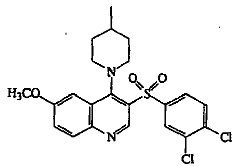 | 466 | *** |
| 250 | 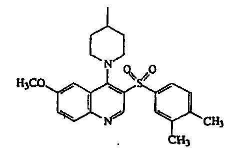 | 425 | ** |
| 251 | 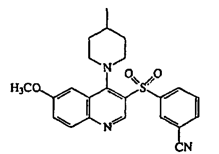 | 432 | *** |
| 252 | 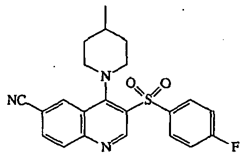 | 410 | ** |
| 253 | 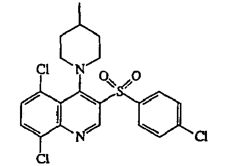 | 471 | ** |
| 254 | 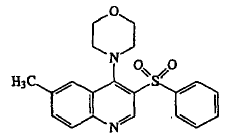 | 369 | *** |
| 255 | 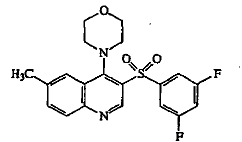 | 405 | * |
| 256 | 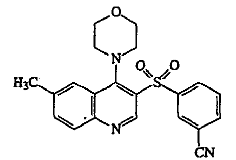 | 394 | ** |
| 257 | 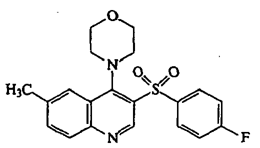 | 387 | * |
| 258 | 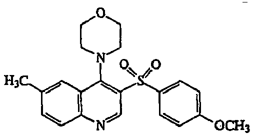 | 399 | * |
| 259 | 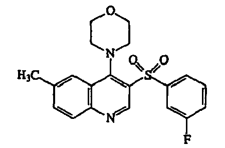 | 387 | * |
| 260 | 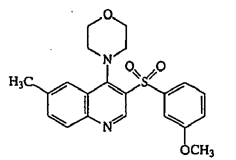 | 399 | ** |
| 261 | 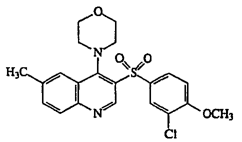 | 433 | ** |
| 262 | 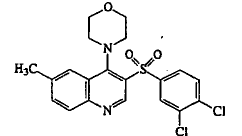 | 438 | ** |
| 263 | 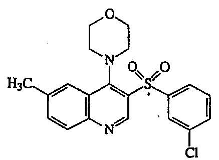 | 403 | * |
| 264 | 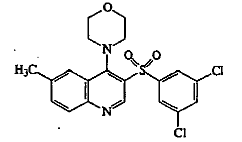 | 438 | ** |
| 265 | 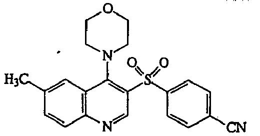 | 394 | * |
| 266 | 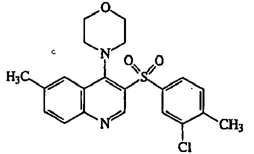 | 417 | ** |
| 267 | 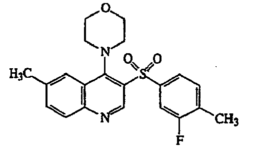 | 401 | * |
| 268 | 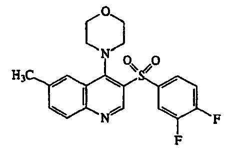 | 405 | * |
| 269 | 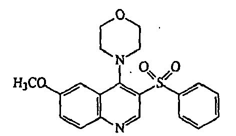 | 385 | ** |
| 270 | 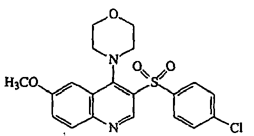 | 419 | *** |
| 271 | 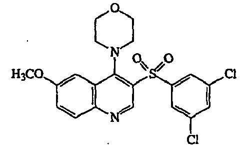 | 454 | * |
| 272 | 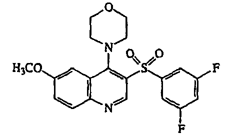 | 421 | * |
| 273 | 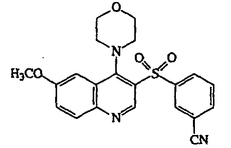 | 410 | * |
| 274 | 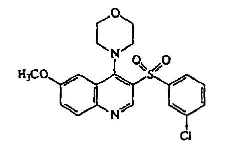 | 419 | * |
| 275 | 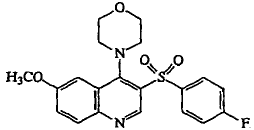 | 403 | * |
| 276 | 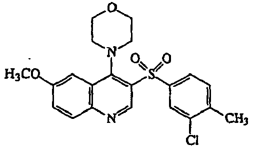 | 433 | * |
| 277 | 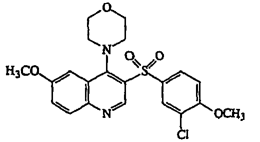 | 449 | * |
| 278 | 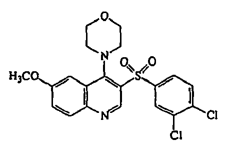 | 454 | * |
| 279 | 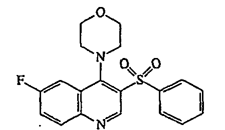 | 373 | *** |
| 280 | 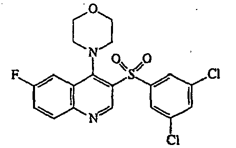 | 442 | * |
| 281 | 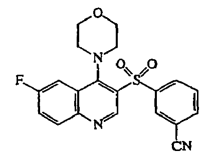 | 398 | * |
| 282 | 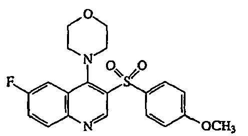 | 403 | * |
| 283 | 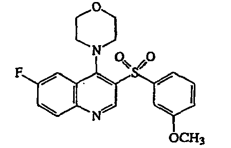 | 403 | * |
| 284 | 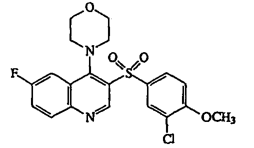 | 437 | * |
| 285 | 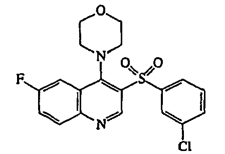 | 407 | * |
| 286 | 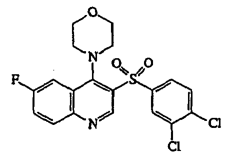 | 442 | ** |
| 287 | 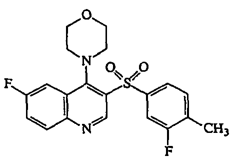 | 405 | * |
| 288 | 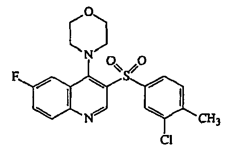 | 422 | ** |
| 289 | 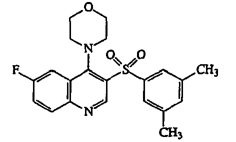 | 401 | * |
| 290 | 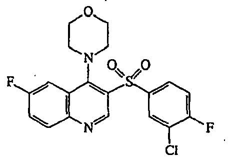 | 425 | ** |
| 291 | 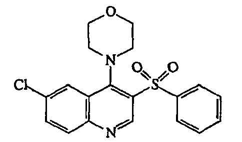 | 389 | * |
| 292 | 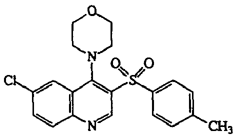 | 403 | *** |
| 293 | 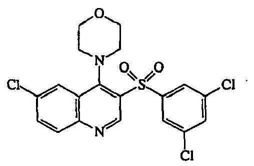 | 459 | * |
| 294 | 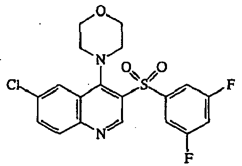 | 425 | * |
| 295 | 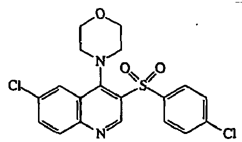 | 424 | * |
| 296 | 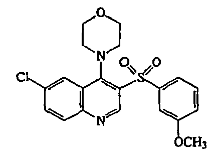 | 419 | * |
| 297 | 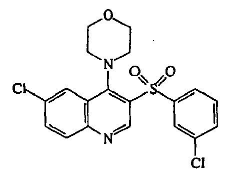 | 424 | * |
| 298 | 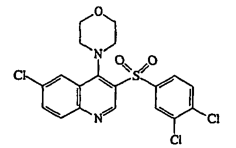 | 459 | ** |
| 299 | 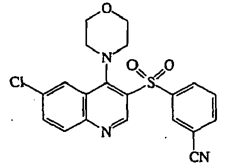 | 414 | * |
| 300 | 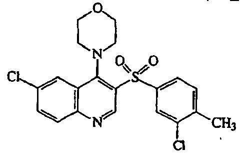 | 438 | ** |
| 301 | 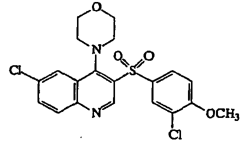 | 454 | * |
| 302 | 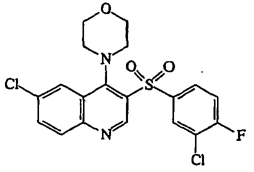 | 442 | * |
| 303 | 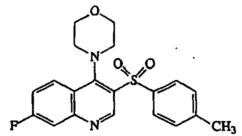 | 387 | * |
| 304 | 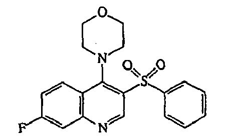 | 373 | * |
| 305 | 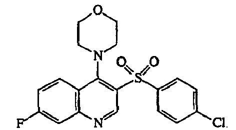 | 407 | * |
| 306 | 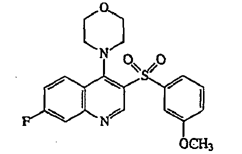 | 403 | ** |
| 307 | 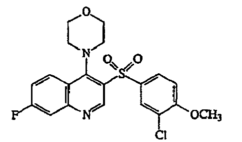 | 437 | ** |
| 308 | 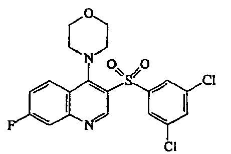 | 442 | ** |
| 309 | 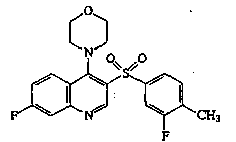 | 405 | ** |
| 310 | 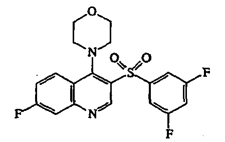 | 409 | * |
| 311 | 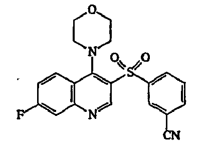 | 398 | ** |
| 312 | 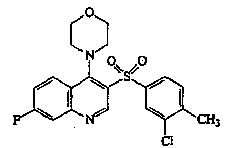 | 421 | *** |
| 313 | 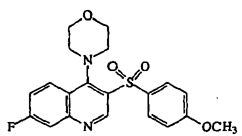 | 403 | * |
| 314 | 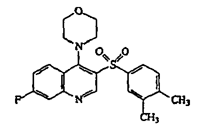 | 401 | ** |
| 315 | 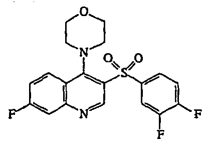 | 409 | ** |
| 316 | 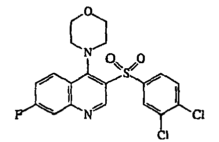 | 442 | *** |
| 317 | 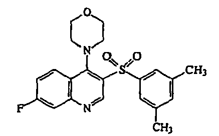 | 401 | ** |
| 318 | 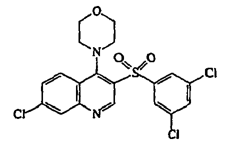 | 459 | *** |
| 319 | 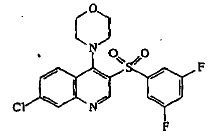 | 425 | ** |
| 320 | 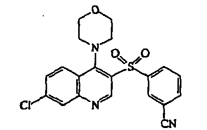 | 414 | ** |
| 321 | 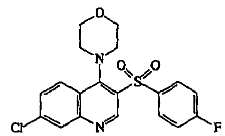 | 407 | * |
| 322 | 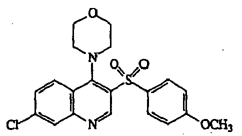 | 419 | * |

| | | | |
|---|---|---|---|
| 323 | 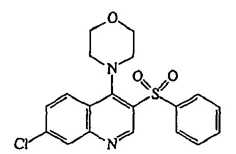 | 389 | * |
| 324 | 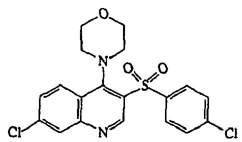 | 424 | ** |
| 325 | 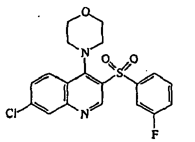 | 407 | * |
| 326 | 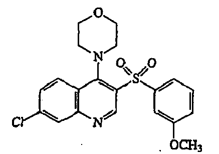 | 419 | ** |
| 327 | 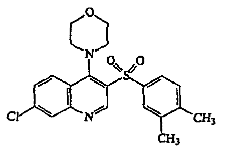 | 417 | *** |
| 328 | 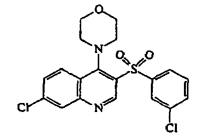 | 424 | *** |
| 329 | 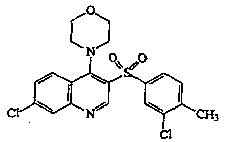 | 438 | *** |
| 330 | 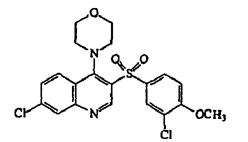 | 454 | ** |
| 331 | 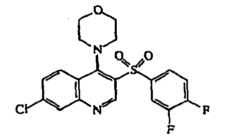 | 425 | * |
| 332 | 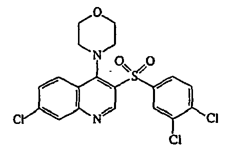 | 459 | *** |
| 333 | 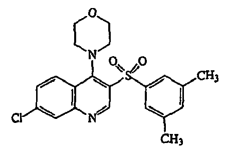 | 417 | * |
| 334 | 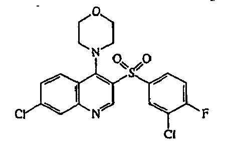 | 441 | *** |
| 335 | 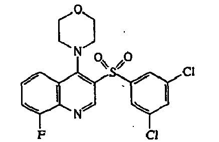 | 441 | ** |
| 336 | 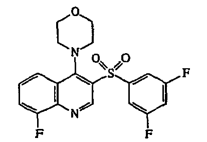 | 409 | ** |
| 337 | 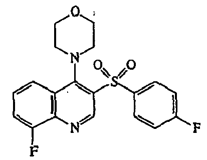 | 391 | * |
| 338 | 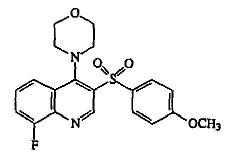 | 403 | * |
| 339 | 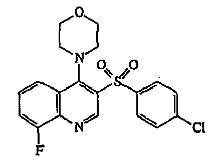 | 407 | ** |
| 340 | 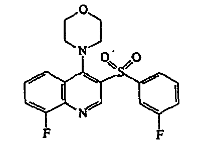 | 391 | * |
| 341 | 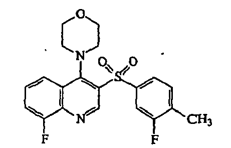 | 405 | ** |
| 342 | 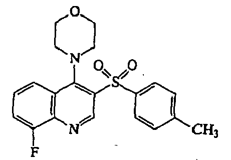 | 387 | * |
| 343 | 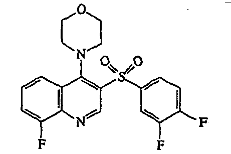 | 409 | ** |
| 344 | 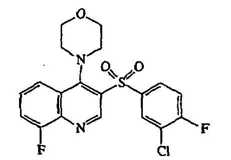 | 425 | *** |
| 345 | 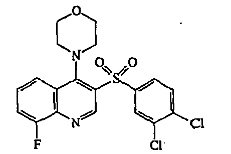 | 442 | *** |
| 346 | 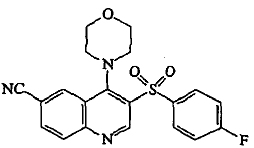 | 398 | ** |
| 347 | 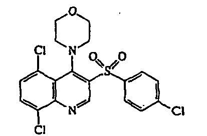 | 459 | ** |
| 348 | 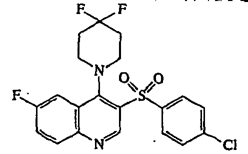 | 441 | * |
| 349 | 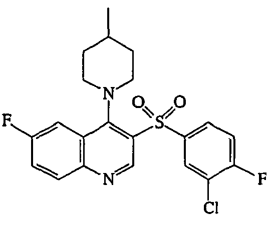 | 437 | ** |
| 350 | 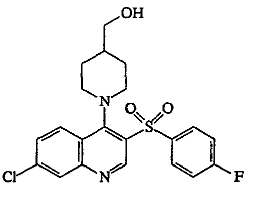 | 435 | ** |
| 351 | 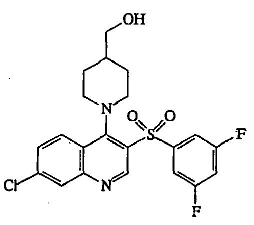 | 453 | ** |

| | | | |
|---|---|---|---|
| *** Kᵢ < 200 nM ** 200nM < Kᵢ < 500 nM * 500nM<Kᵢ | | | |

### Example 6

### Preparation of pharmaceutical compositions:

### a) Tablets:

0.01-50 % of active ingredient of formula (I), 15-50 % of lactose, 15-50 % of potato starch, 5-15 % of polyvinyl pyrrolidone, 1-5 % of talc, 0.01-3 % of magnesium stearate, 1-3 % of colloid silicon dioxide and 2-7 % of ultraamylopectin were mixed, then granulated by wet granulation and pressed to tablets.

### b) Dragées, filmcoated tablets:

The tablets made according to the method described above were coated by a layer consisting of entero- or gastrosolvent film, or of sugar and talc. The dragées were polished by a mixture of beeswax and carnuba wax.

### c) Capsules:

0.01-50 % of active ingredient of formula (I), 1-5 % of sodium lauryl sulfate, 15-50 % of starch, 15-50 % of lactose, 1-3 % of colloid silicon dioxide and 0.01-3 % of magnesium stearate were thoroughly mixed, the mixture was passed through a sieve and filled in hard gelatin capsules.

### d) Suspension.

Ingredients: 0.01-15 % of active ingredient of formula (I), 0.1-2 % of sodium hydroxide, 0.1-3 % of citric acid, 0.05-0.2 % of nipagin (sodium methyl 4-hydroxybenzoate), 0.005-0.02 % of nipasol, 0.01-0.5 % of carbopol (polyacrilic acid), 0.1-5 % of 96 % ethanol, 0.1-1 % of flavoring agent, 20-70 % of sorbitol (70 % aqueous solution) and 30-50 % of distilled water.

To solution of nipagin and citric acid in 20 ml of distilled water, carbopol was added in small portions under vigorous stirring, and the solution was left to stand for 10-12 h. Then the sodium hydroxide in 1 ml of distilled water, the aqueous solution of sorbitol and finally , the ethanolic raspberry flavor were added with stirring. To this carrier the active ingredient was added in small portions and suspended with an immersing homogenizator. Finally the suspension was filled up to the desired final volume with distilled water and the suspension syrup was passed through a colloid milling equipment.

### e) Suppositories:

For each suppository 0.01-15% of active ingredient of formula (I) and 1-20% of lactose were thoroughly mixed, then 50-95% of adeps pro suppository (for example Witepsol 4) was melted, cooled to 35 °C and the mixture of active ingredient and lactose was mixed in it with homogenizator. The obtained mixture was mould in cooled forms.

### f) Lyophilized powder ampoule compositions:

A 5 % solution of mannitol or lactose was made with bidistilled water for injection use, and the solution was filtered so as to have sterile solution. A 0.01-5 % solution of the active ingredient of formula (I) was also made with bidistilled water for injection use, and this solution was filtered so as to have sterile solution. These two solutions were mixed under aseptic conditions, filled in 1 ml portions into ampoules, the content of the ampoules was lyophilized, and the ampoules were sealed under nitrogen. The contents of the ampoules were dissolved in sterile water or 0.9 % (physiological) sterile aqueous sodium chloride solution before administration.

## Claims

1. A compound having the formula (I) for use in a method of preventi on and/or treatment of mGluR5 receptor-mediated disorders, 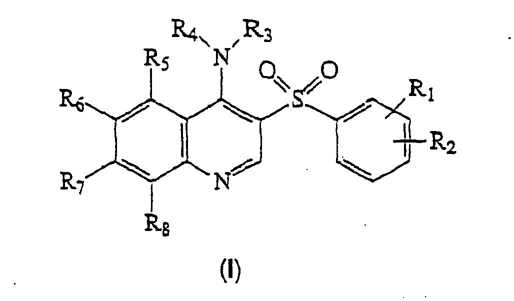 wherein
R₁ and R₂ are independently selected from the group consisting of hydrogen, halogen, alkyl, alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, alkyl, alkylhydroxy, alkyloxycarbonyl, aminocarbonyl, -OCH₂CH₂O-, benzyl and substituted phenyl group;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

2. A compound having the formula (I) for use in a method of prevention and/or treatment of mGluR5 receptor-mediated disorders, 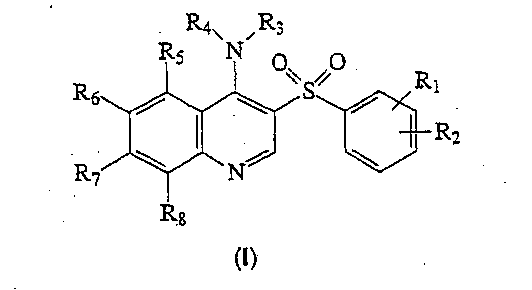 wherein
R₁ and R₂ are independently selected from the group consisting of hydrogen, halogen, C₁₋₄ alkyl, C₁₋₄ alkoxy, cyano, optionally substituted amino group or saturated heterocyclyl group, wherein the heteroatom is N;
R₃ and R₄ are independently selected from the group consisting of hydrogen, C₁₋₄ alkyl, substituted aryl group having at least one substituent selected from the group of hydrogen, halogen, C₁₋₄ alkyl, C₁₋₄ alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, C₁₋₄ alkyl, C₁₋₄ alkylhydroxy, alkyloxycarbonyl, aminocarbonyl, -OCH₂CH₂O- , benzyl and substituted phenyl group, which may be optionally substituted by 1 or 2 groups selected from hydrogen, halogen, C₁₋₄ alkyl, C₁₋₄ alkoxy group;
R₅, R₆, R₇ and R₈ are independently selected from the group consisting of hydrogen, halogen, C₁₋₄ alkyl, C₁₋₄ alkoxy, cyano group, or
R₆ and R₇ together with the atoms to which they are attached can form an unsaturated 5 to 7 membered heterocyclyl group, containing 1 or 2 O atom(s);
and/or enantiomers and/or racemates and/or diastereomers and/or pharmaceutically acceptable salts thereof formed with acids or bases.

3. A compound having the formula (I) for use in a method of prevention and/or treatment of mGluR5 receptor-mediated disorders, 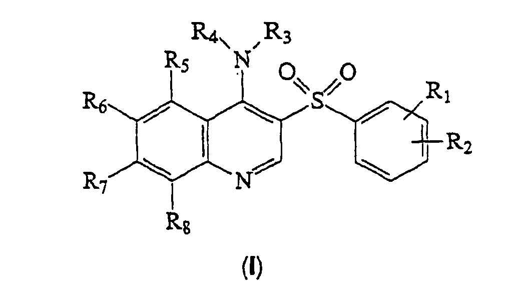 wherein
R¹ and R² are independently selected from the group consisting of hydrogen, chloro, fluoro, C₁₋₂ alkyl, C₁₋₂ alkoxy, cyano or piperidinyl group;
R₃ and R₄ are independently selected from the group consisting of hydrogen, C₁₋₂ alkyl, benzyl group substituted by 1 or 2 groups independently selected from hydrogen, halogen, C₁₋₄ alkyl, C₁₋₄ alkoxy group,
R₃ and R₄ together with the N atoms to which they are attached can form pyrrolidinyl, homopiperidinyl, morpholinyl group or piperidinyl group which may be optionally substituted by the groups selected from hydrogen, halogen, C₁₋₄ alkyl, hydroxymethyl, alkyloxycarbonyl, aminocarbonyl, and -OCH₂CH₂O- group or piperazinyl group which may be substituted at N(4) by the groups selected from C₁₋₄ alkyl, benzyl, -alkyloxycarbonyl and phenyl, which may be optionally substituted by 1 or 2 groups selected from hydrogen, halogen, C₁₋₄ alkyl, alkoxy, group;
R₅, R₆, R₇ and R₈ are independently selected from the group consisting of hydrogen, chloro, fluoro, C₁₋₂ alkyl, C₁₋₂ alkoxy, cyano group, or
R₆ and R₇ together with the atoms to which they are attached can form 2,3-dihydro-[1,4]dioxine or 2,5-dihydro-furan ring;
and/or enantiomers and/or racemates and/or diastereomers and/or pharmaceutically acceptable salts thereof formed with acids or bases.

4. A compound for use in a method of prevention and/or treatment of mGluR5 receptor-mediated disorders according to claim 1 wherein the compound is selected from :
3-(3,4-Dimethyl-benzenesulfonyl)-4-(morpholin-4-yl)quinoline
3-(4-Methyl-benzenesulfonyl)-4-(3-methyl-piperidin-1-yl)-quinoline
3-(3,4-Dimethyl-benzenesulfonyl)-4-(4-methyl-piperidin-1-yl)-quinoline
3-(4-Methyl-benzenesulfonyl)-4-(4-methyl-piperidin-1-yl)-quinoline
3-Benzenesulfonyl-4-(piperidin-1-yl)-quinoline
3-(4-Methyl-benzenesulfonyl)-4-(piperidin-1-yl)-quinoline
4-Benzylamino-3-(4-methyl-benzenesulfonyl)-quinoline
6-Ethyl-4-(4-methyl-piperidin-1-yl)-3-(4-methoxy-benzenesulfonyl)-quinoline
6-Fluoro-3-(4-methyl-benzenesulfonyl)-4-(4-methyl-piperidin-1-yl)-quinoline
6-Ethoxy-3-(4-chloro-benzenesulfonyl)-4-(4-fluoro-benzylamino)-quinoline
4-(Azepan-1-yl)-3-(4-methyl-benzenesulfonyl)-quinoline
4-(Azepan-1-yl)-3-(4-chloro-benzenesulfonyl)-quinoline
6-Methyl-3-(4-methyl-benzenesulfonyl)-4-(4-methyl-piperidin-1-yl)-quinoline
4-(4-Methylpiperidin-1-yl)-3-benzenesulfonyl-quinoline
9-(4-methyl-piperidin-1-yl)-8-benzenesulfonyl-2,3-dihydro-[1,4]dioxino[2,3-g]quinoline
6-Ethyl-4-(4-ethyloxycarbonyl-piperidin-1-yl)-3-(4-chloro-benzenesulfonyl)-quinoline
4-Diethylamino-3-(4-methyl-benzenesulfonyl)-quinoline
4-(4-Benzyl-piperazin-1-yl)-3-(4-ehloro-benzenesulfonyl)-quinoline
4-(Azepan-1-yl)-3-benzenesulfonyl-quinoline
3-(3-Cyano-benzenesulfonyl)-6-fluoro-4-(4-methyl-piperidin-1-yl)-quinoline
6-Fluoro-3-(4-methoxy-benzenesulfonyl)-4-(4-methyl-piperidin-1-yl)-quinoline
6-Fluoro-3-(3-methoxy-benzenesulfonyl)-4-(4-methyl-piperidin-1-yl)-quinoline
6-Fluoro-3-(3,4-dimethyl-benzenesulfonyl)-4-(4-methyl-piperidin-1-yl)-quinoline
3-(3-Chloro-4-methoxy-benzenesulfonyl)-6-fluoro-4-(4-methyl-piperidin-1-yl)-quinoline
3-(3-Chloro-4-fluoro-benzenesulfonyl)-6-fluoro-4-(4-methyl-piperidin-1-yl)-quinoline
3-(3,4-Dichloro-benzenesulfonyl)-6-fluoro-4-(4-methyl-piperidin-1-yl)-quinoline
3-(3-Chloro-benzenesulfonyl)-6-fluoro-4-(4-methyl-piperidin-1-yl)-quinoline
3-(4-Chloro-benzenesulfonyl)-6-methyl-4-(4-methyl-piperidin-1-yl)-quinoline
3-(3-Fluoro-benzenesulfonyl)-6-methyl-4-(4-methyl-piperidin-1-yl)-quinoline
3-(3-Methoxy-benzenesulfonyl)-6-methyl-4-(4-methyl-piperidin-1-yl)-quinoline
3-(3,4-Dimethyl-benzenesulfonyl)-6-methyl-4-(4-methyl-piperidin-1-yl)-quinoline
3-(3-Chloro-4-methoxy-benzenesulfonyl)-6-methyl-4-(4-methyl-piperidin-1-yl)-quinoline
3-(3-Chloro-benzenesulfonyl)-6-methyl-4-(4-methyl-piperidin-1-yl)-quinoline
3-(3-Fluoro-4-methyl-benzenesulfonyl)-6-methyl-4-(4-methyl-piperidin-1-yl)-quinoline
3-(3-Chloro-4-methyl-benzenesulfonyl)-6-methyl-4-(4-methyl-piperidin-1-yl)-quinoline
3-(3-Chloro-4-fluoro-benzenesulfonyl)-6-methyl-4-(4-methyl-piperidin-1-yl)-quinoline
3-(3,4-Dichloro-benzenesulfonyl)-7-fluoro-4-(4-methyl-piperidin-1-yl)-quinoline
7-Fluoro-3-(3-cyano-benzenesulfonyl)-4-(4-methyl-piperidin-1-yl)-quinoline
7-Fluoro-3-(4-cyano-benzenesulfonyl)-4-(4-methyl-piperidin-1-yl)-quinoline
3-(3-Chloro-4-methyl-benzenesulfonyl)-7-fluoro-4-(4-methyl-piperidin-1-yl)-quinoline
7-Fluoro-3-(3-methoxy-benzenesulfonyl)-4-(4-methyl-piperidin-1-yl)-quinoline
3-(3,4-Difluoro-benzenesulfonyl)-7-fluoro-4-(4-methyl-piperidin-1-yl)-quinoline
3-(3-chloro-4-fluoro-benzenesulfonyl)-7-fluoro-4-(4-methyl-piperidin-1-yl)-quinoline
7-Chloro-3-(3,5-dichloro-benzenesulfonyl)-4-(4-methyl-piperidin-1-yl)-quinoline
7-Chloro-3-(3,5-difluoro-benzenesulfonyl)-4-(4-methyl-piperidin-1-yl)-quinoline
7-Chloro-3-(3-cyano-benzenesulfonyl)-4-(4-methyl-piperidin-1-yl)-quinoline
7-Chloro-3-(4-methyl-benzenesulfonyl)-4-(4-methyl-piperidin-1-yl)-quinoline
7-Chloro-3-(4-fluoro-benzenesulfonyl)-4-(4-methyl-piperidin-1-yl)-quinoline
3-Benzenesulfonyl-7-Chloro-4-(4-methyl-piperidin-1-yl)-quinoline
7-Chloro-3-(4-chloro-benzenesulfonyl)-4-(4-methyl-piperidin-1-yl)-quinoline
7-Chloro-3-(3,4-dimethoxy-benzenesulfonyl)-4-(4-methyl-piperidin-1-yl)-quinoline
7-Chloro-3-(3-fluoro-benzenesulfonyl)-4-(4-methyl-piperidin-1-yl)-quinoline
7-Chloro-3-(3-mehoxy-benzenesulfonyl)-4-(4-methyl-piperidin-1-yl)-quinoline
7-Chloro-3-(3-chloro-4-methoxy-benzenesulfonyl)-4-(4-methyl-piperidin-1-yl)-quinoline
7-Chloro-3-(3-chloro-benzenesulfonyl)-4-(4-methyl-piperidin-1-yl)-quinoline
7-Chloro-3-(3-chloro-4-fluoro-benzenesulfonyl)-4-(4-methyl-piperidin-1-yl)-quinoline
6-Chloro-3-(3,5-difluoro-benzenesulfonyl)-4-(4-methyl-piperidin-1-yl)-quinoline
6-Chloro-3-(4-methyl-benzenesulfonyl)-4-(4-methyl-piperidin-1-yl)-quinoline
6-Chloro-3-(4-chloro-benzenesulfonyl)-4-(4-methyl-piperidin-1-yl)-quinoline
8-Fluoro-3-(3-fluoro-4-methyl-benzenesulfonyl)-4-(4-methyl-piperidin-1-yl)-quinoline
8-Fluoro-3-(4-methyl-benzenesulfonyl)-4-(4-methyl-piperidin-1-yl)-quinoline
3-(3,4-Dichloro-benzenesulfonyl)-8-fluoro-4-(4-methyl-piperidin-1-yl)-quinoline
3-(3-chloro-4-fluoro-benzenesulfonyl)-8-fluoro-4-(4-methyl-piperidin-1-yl)-quinoline
3-(3,4-Difluoro-benzenesulfonyl)-8-fluoro-4-(4-methyl-piperidin-1-yl)-quinoline
3-Benzenesulfonyl-6-methyl-4-(morpholin-1-yl)-quinoline
3-(3-Chloro-benzenesulfonyl)-6-methoxy-4-(morpholin-1-yl)-quinoline
3-Benzenesulfonyl-6-fluoro-4-(morpholin-1-yl)-quinoline
6-Chloro-3-(4-chloro-benzenesulfonyl)-4-(morpholin-1-yl)-quinoline
3-(3-Chloro-4-methyl-benzenesulfonyl)-7-fluoro-4-(morpholin-1-yl)-quinoline
3-(3,4-Dichloro-benzenesulfonyl)-7-fluoro-4-(morpholin-1-yl)-quinoline
7-Chloro-3-(3,5-dichloro-benzenesulfonyl)-4-(morpholin-1-yl)-quinoline
7-Chloro-3-(3,4-dimethyl-benzenesulfonyl)-4-(morpholin-1-yl)-quinoline
7-Chloro-3-(3-chloro-benzenesulfonyl)-4-(morpholin-1-yl)-quinoline
7-Chloro-3-(3-chloro-4-methyl-benzenesulfonyl)-4-(morpholin-1-yl)-quinoline
7-Chloro-3-(3,4-dichloro-benzenesulfonyl)-4-(morpholin-1-yl)-quinoline
7-Chloro-3-(3-chloro-4-fluoro-benzenesulfonyl)-4-(morpholin-1-yl)-quinoline.

5. The compound as claimed in any one of the claims 1-4, wherein said mGluR5 receptor-mediated disorders are psychiatric disorders.

6. The compound as claimed in any one of the claims 1-4, wherein said mGluR5 receptor-mediated disorders are neurological disorders.

7. The compound as claimed in any one of the claims 1-4, wherein said mGluR5 receptor-mediated disorders are chronic and acute pain disorders.

8. The compound as claimed in any one of the claims 1-4, wherein said mGluR5 receptor-mediated disorders are neuromuscular dysfunctions of the lower urinary tract and gastrointestinal disorders.

9. A process for the preparation of a compound of formula (I) 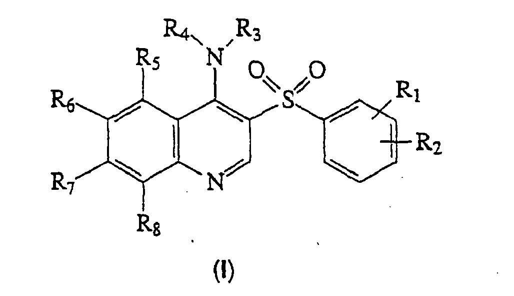 wherein
R₁ and R₂ are independently selected from the group consisting of hydrogen, halogen, alkyl, alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, alkyl, alkylhydroxy, alkyloxycarbonyl, aminocarbonyl, -OCH₂CH₂O-, benzyl and substituted phenyl group;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases
a. converting a compound of formula (VI): 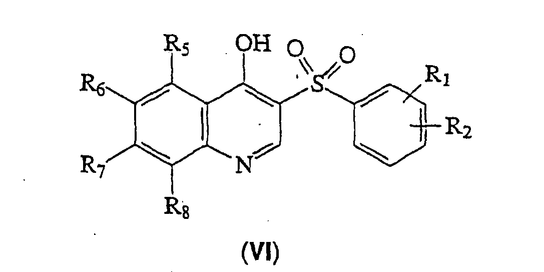 wherein R₁, R₂, R₅, R₆, R₇ and R₈ are as defined above for a compound of formula (I), to a compound of formula (VII): 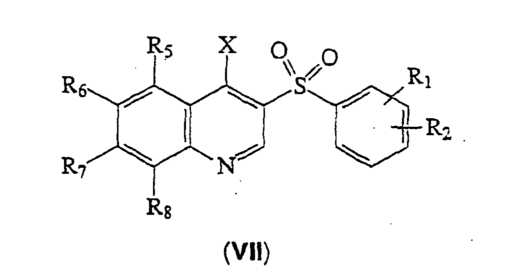 wherein X is selected from halogen, benzenesulfonyloxy or trifluoromethanesulfonyloxy groups and R₁, R₂. R₅, R₆, R₇ and R₈ are as defined above for a compound of formula (I), thereafter reacting the obtained compound of formula (VII) with a compound of formula (VIII) : 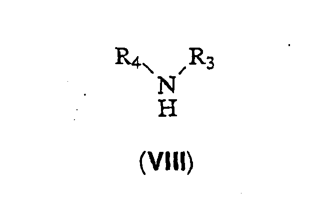 wherein R₃ and R₄ are as defined above for a compound of formula (I) to give a compound of formula (I), and optionally forming enantiomers and/or racemates and/or diastereomers and/or pharmaceutically acceptable salts thereof, or
b.) reacting a compound of formula (XV): 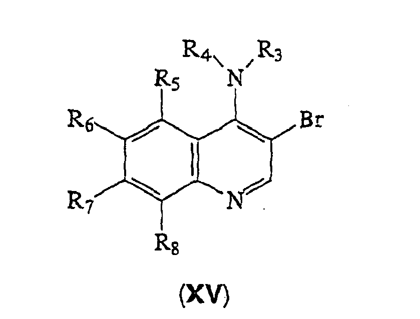 wherein R₃, R₄, R₅, R₆, R₇ and R₈ are as defined above for a compound of formula (I), with a compound of formula (III): 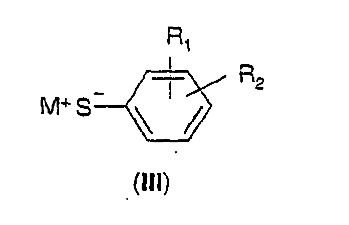 wherein M is selected from alkali or alkaline-earth metals, R₁ and R₂ are as defined above for a compound of formula (I), to give a compound of formula (XVI): 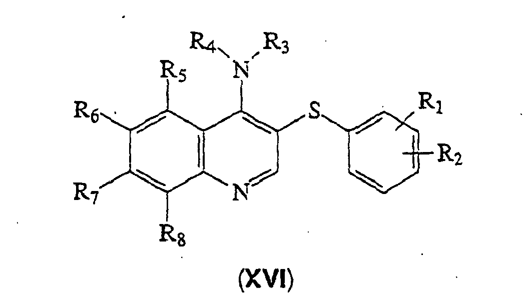 wherein R₁, R₂, R₃, R₄, R₅, R₆, R₇ and R₈ are as defined above for a compound of formula (I), thereafter oxidizing a compound of formula (XVI) to obtain a compound of formula (XVII): 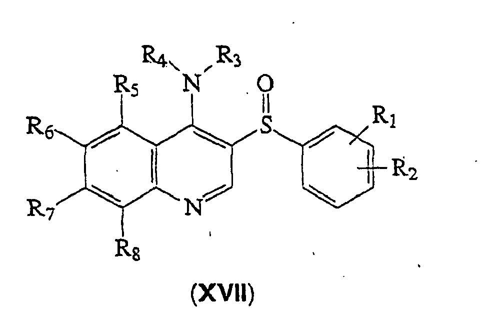 wherein R₁, R₂, R₃, R₄, R₅, R₆, R₇ and R₈ are as defined above for a compound of formula (I), thereafter oxidizing a compound of formula (XVII) to obtain a compound of formula (I), and optionally forming enantiomers and/or racemates and/or diastereomers and/or pharmaceutically acceptable salts thereof or
c.) interconverting one compound of formula (I), wherein the meaning of R₁, R₂, R₃, R₄, R₅, R₆, R₇ and R₈ is as defined above for the formula (I) to a different compound of formula (I), wherein the meaning of R₁, R₂, R₃, R₄, R₅, R₆, R₇ and R₈ is as described above for the formula (I);
where appropriate, separating the enantiomers and/or racemates and/or diastereomers of compounds of formula (I), wherein the meaning of R₁, R₂, R₃, R₄, R₅, R₆, R₇ and R₈ is as described above for the formula (I) by conventional methods;
and optionally thereafter forming salts and/or hydrates and/or solvates of compounds of formula (I).

10. A process for the preparation of a compound of formula (VI): wherein 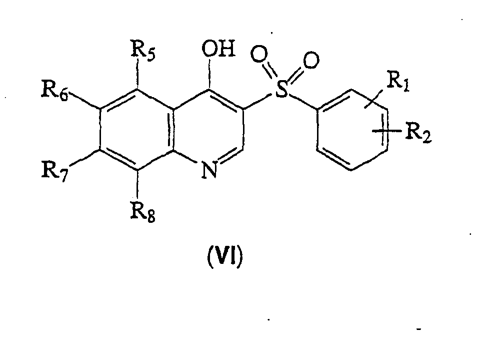 R₁ and R₂ are independently selected from the group consisting of hydrogen, halogen, alkyl, alkoxy, cyano, optionally substituted amino group or saturated heterocyclyl group, wherein the heteroatom is N;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.
a. reacting a compound of formula (II): 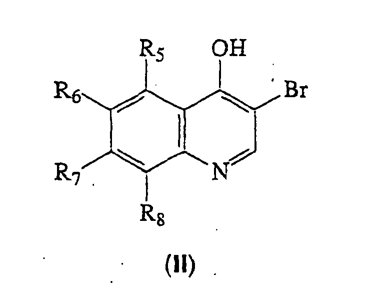 wherein R₅, R₆, R₇ and R₈ are as defined above for a compound of formula (I), with a compound of formula (III): 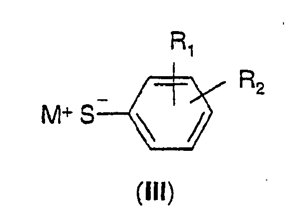 wherein M is selected from alkali metals or alkaline-earth metals, R₁ and R₂ are as defined above for a compound of formula (I), to give a compound of formula (IV): 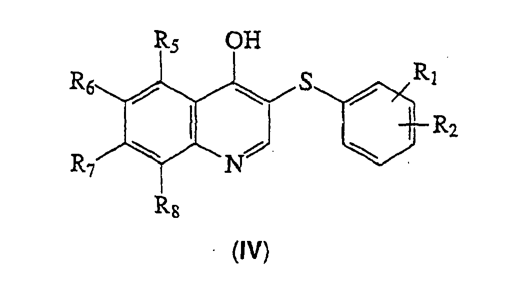 wherein R₁, R₂, R₅, R₆, R₇ and R₈ are as defined above for a compound of formula (I), thereafter oxidizing a compound of formula (IV) to obtain a compound of formula (V): 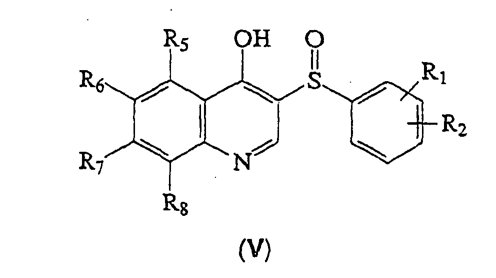 wherein R₁, R₂, R₅, R₆, R₇ and R₈ are as defined above for a compound of formula (I), thereafter oxidizing a compound of formula (V) to obtain a compound of formula (VI), and optionally forming enantiomers and/or racemates and/or diastereomers and/or pharmaceutically acceptable salts thereof or
b. reacting a compound of formula (IX): 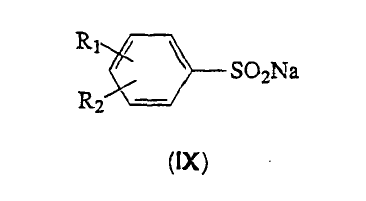 whererein R₁ and R₂ are as defined above for a compound of formula (I), with α-halogen-acetic acid esters of formula (X)
Hlg-CH₂-COOR₉ (**X**)
wherein ,Hlg is halogen and R₉ is an ethyl or methyl group, to obtain a compound of formula (XI) 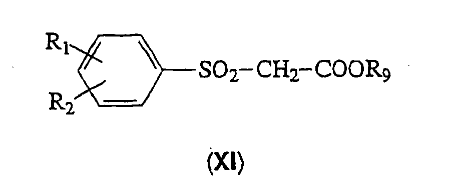
wherein R₁ and R₂ are as defined above for a compound of formula (I) and R₉ is as defined above for compounds of formula (X); reacting a compound of formula (XI) with a trialkyl orthoformate of formula (XII):
CH(OR₁₀)₃ (**XII**)
wherein R₁₀ is an ethyl or methyl group, to obtain a compound of formula (XIII): 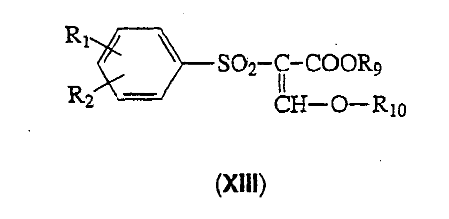 wherein R₁ and R₂ are as defined above for a compound of formula (I), R₉ is as defined above for compounds of formula (X) and R₁₀ is as defined above for compounds of formula (XII); reacting a compound of formula (XIII) with an aniline derivative of formula (XIV): 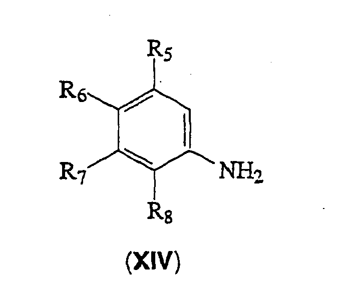 wherein R₅, R₆, R₇ and R₈ are as defined above for a compound of formula (I), to obtain a compound of formula (VI), and optionally forming enantiomers and/or racemates and/or diastereomers and/or pharmaceutically acceptable salts thereof.

11. An intermediate having the formula (IV)
wherein 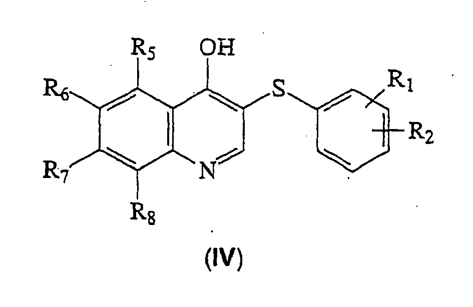 R₁ and R₂ are independently selected from the group consisting of hydrogen, halogen, alkyl, alkoxy, cyano, optionally substituted amino group or saturated heterocyclyl group, wherein the heteroatom is N;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

12. An intermediate having the formula (V)
wherein 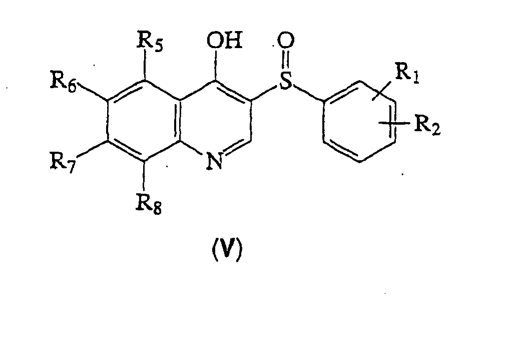 R₁ and R₂ are independently selected from the group consisting of hydrogen, halogen, alkyl, alkoxy, cyano, optionally substituted amino group or saturated heterocyclyl group, wherein the heteroatom is N;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl groups
and/or enantiomers and/or racemates and/or diastereomers and/or pharmaceutically acceptable salts thereof formed with acids or bases.

13. An intermediate having the formula (VI) 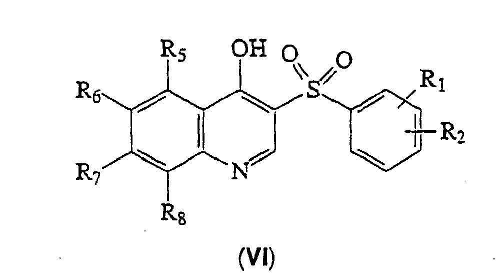 wherein
R₁ and R₂ are independently selected from the group consisting of hydrogen, halogen, alkyl, alkoxy, cyano, optionally substituted amino group or saturated heterocyclyl group, wherein the heteroatom is N;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

14. An intermediate having the formula (VII) 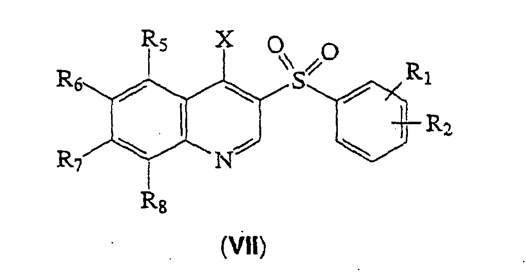 wherein X is selected from the group consisting of halogen, benzenesulphonyloxy or trifluoromethanesulphonyloxy group,
R₁ and R₂ are independently selected from the group consisting of hydrogen, halogen, alkyl, alkoxy, cyano, optionally substituted amino group or saturated heterocyclyl group, wherein the heteroatom is N;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

15. An intermediate having the formula (XVI) 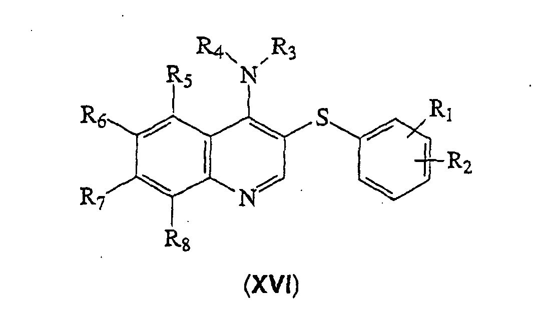 wherein
R₁ and R₂ are independently selected from the group consisting of hydrogen, halogen, alkyl, alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, allyl, alkylhydroxy, alkyloxycarbonyl, aminocarbonyl, -OCH₂CH₂O-, benzyl and substituted phenyl group;
R₅, R₆, R₇ and R₈ are independently selected from the group consisting of hydrogen, halogen, alkyl, alkoxy, cyano group, or
R₆ and R₇ together with the atoms to which they are attached can form an unsaturated heterocyclyl group;
and/or enantiomers and/or racemates and/or diastereomers and/or pharmaceutically acceptable salts thereof formed with acids or bases.

16. An intermediate having the formula (XVII) 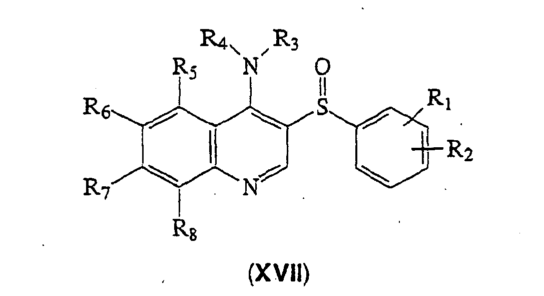 wherein
R₁ and R₂ are independently selected from the group consisting of hydrogen, halogen, alkyl, alkoxy, cyano, optionally substituted amino group or saturated heterocyclyl group, wherein the heteroatom is N;
R₃ and R₄ are independently selected from the group consisting of hydrogen, alkyl, substituted aryl group having at least one substituent selected from the group of hydrogen, halogen, alkyl, alkoxy group, or
R₃ and R₄ together with the N atom to which they are attached can form C₅₋₇ heterocyclyl group, containing 1 or 2 heteroatom(s) selected from the group of N, O, which may be optionally substituted by the groups selected from hydrogen, halogen, alkyl, alkylhydroxy, alkyloxycarbonyl, aminocarbonyl, -OCH₂CH₂O-, benzyl and substituted phenyl group;
R₅, R₆, R₇ and R₈ are independently selected from the group consisting of hydrogen, halogen, alkyl, alkoxy, cyano group, or
R₆. and R₇ together with the atoms to which they are attached can form an unsaturated heterocyclyl group;
and/or enantiomers and/or racemates and/or diastereomers and/or pharmaceutically acceptable salts thereof formed with acids or bases.

17. A pharmaceutical formulation comprising as an active ingredient a therapeutically effective amount of a compound of formula (I) 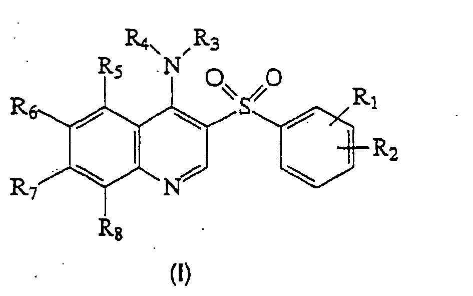 as defined in any one of claims 1 to 3 with one or more pharmaceutically acceptable diluents, excipients and/or inert carriers.

18. A pharmaceutical formulation according to claim 17, for use in the prevention and/or treatment of mGluR5 receptor-mediated disorders.

19. The use of a compound of formula (I) 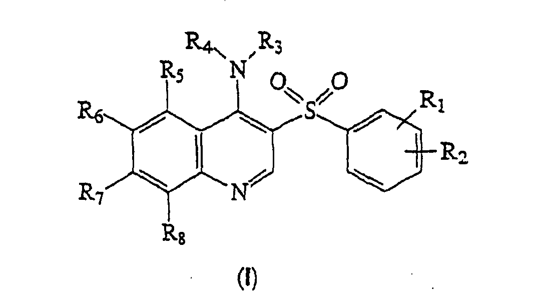 as defined in any one of claims 1 to 3 in the manufacture of a medicament for the prevention and/or treatment of mGluR5 receptor-mediated disorders.

20. Use according to claim 19, wherein said mGluR5 receptor-mediated disorders are psychiatric disorders.

21. Use according to claim 19, wherein said mGluR5 receptor-mediated disorders are neurological disorders.

22. Use according to claim 19, wherein said mGluR5 receptor-mediated disorders are chronic and acute pain.

23. Use according to claim 19, wherein said mGluR5 receptor-mediated disorders are neuromuscular dysfunctions of the lower urinary tract and gastrointestinal disorders.

## Patentansprüche

1. Verbindung der Formel (I) zur Verwendung in einem Verfahren der Verhinderung und/oder Behandlung von mGluR5-Rezeptor-vermittelten Störungen, 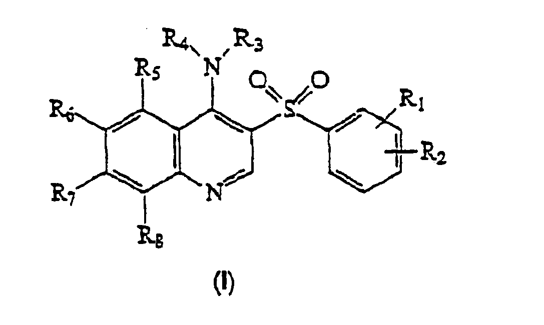 worin
R₁ und R₂ unabhängig aus der Gruppe ausgewählt sind, die aus Wasserstoff, Halogen, Alkyl, Alkoxy, Cyano, optional substituierter Aminogruppe oder gesättigter heterocyclischer Gruppe besteht, worin das Heteroatom N ist;
R₃ und R₄ unabhängig aus der Gruppe ausgewählt sind, die aus Wasserstoff, Alkyl, substituierter Arylgruppe mit mindestens einem Substituenten, der aus der Gruppe aus Wasserstoff, Halogen, Alkyl-, Alkoxygruppe ausgewählt ist, oder
R₃ und R₄ zusammen mit dem Stickstoffatom, woran sie angebracht sind, eine C₅₋₇-heterocyclische Gruppe, die 1 oder 2 Heteroatome enthält, die aus der Gruppe aus N, O, die gegebenenfalls durch die Gruppen substituiert sind, die aus Wasserstoff, Halogen, Alkyl, Alkylhydroxy, Alkyloxycarbonyl, Aminocarbonyl, -OCH₂CH₂O-, Benzyl und substituierte Phenylgruppe ausgewählt sind, bilden können;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptablen Salze, die mit Säuren oder Basen gebildet sind.

2. Verbindung der Formel (I) zur Verwendung im Verfahren der Verhinderung und/oder Behandlung von mGluR5-Rezeptor-mittelten Störungen, 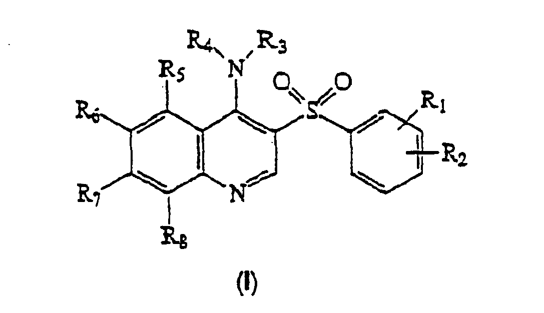 worin
R¹ und R² unabhängig aus der Gruppe ausgewählt sind, die aus Wasserstoff, Halogen, C₁₋₄-Alkyl, C₁₋₄-Alkoxy, Cyano, optional substituierte Aminogruppe oder gesättigter heterocyclischer Gruppe besteht, worin das Heteroatom N ist;
R₃ und R₄ unabhängig aus der Gruppe ausgewählt sind, die aus Wasserstoff, C₁₋₄-Alkyl, substituierter Arylgruppe mit mindestens einem Substituenten, der aus der Gruppe aus Wasserstoff, C₁₋₄-Alkyl, C₁₋₄-Alkoxygruppe ausgewählt sind, oder
R₃ und R₄ zusammen mit dem Stickstoffatom, woran sie angebracht sind, eine C₅₋₇-heterocyclische Gruppe, die 1 oder 2 Heteroatome enthält, die aus der Gruppe aus N, O, die gegebenenfalls durch die Gruppen substituiert sind, die aus Wasserstoff, Halogen, C₁₋₄-Alkyl, C₁₋₄-Alkylhydroxy, Alkyloxycarbonyl, Aminocarbonyl, -OCH₂CH₂O-, Benzyl und substituierte Phenylgruppe, die gegebenenfalls durch 1 oder 2 Gruppen substituiert sein können, die aus Wasserstoff, Halogen, C₁₋₄-Alkyl, Alkoxygruppe ausgewählt sind, bilden können;
R₅, R₆, R₇ und R₈ unabhängig aus der Gruppe ausgewählt sind, die aus Wasserstoff, Halogen, C₁₋₄-Alkyl, C₁₋₄-Alkoxygruppe besteht, oder
R₆ und R₇ zusammen mit den Atomen, woran sie angebracht sind, eine ungesättigte 5- bis 7-gliedrige heterocyclische Gruppe, enthaltend 1 oder 2 Sauerstoffatome bilden können;
und/oder Enantiomere und/oder Racemate und/oder Diastereomere und/oder deren pharmazeutisch akzeptable Salze, die mit Säuren oder Basen gebildet sind.

3. Verbindung der Formel (I) zur Verwendung in einem Verfahren zur Verhinderung und/oder Behandlung von mGluR5-Rezeptor-vermittelten Störungen, 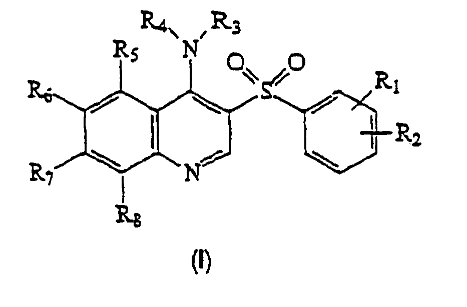 worin
R¹ und R² unabhängig aus der Gruppe ausgewählt sind, die aus Wasserstoff, Chlor, Fluor, C₁₋₂-Alkyl, C₁₋₂-Alkoxy, Cyano oder Piperidinylgruppe besteht;
R₃ und R₄ unabhängig aus der Gruppe ausgewählt sind, die aus Wasserstoff, C₁₋₂-Alkyl, Benzylgruppe, die mit 1 oder 2 Gruppen substituiert ist, die unabhängig aus Wasserstoff, Halogen, C₁₋₄-Alkyl-, C₁₋₄-Alkoxygruppe ausgewählt sind,
R₃ und R₄ zusammen mit den N-Atomen, woran sie angebracht sind, Pyrolidinyl, Homopiperidinyl, Morpholingruppe oder Piperidinylgruppe bilden können, die gegebenenfalls durch die Gruppen substituiert sind, die aus Wasserstoff, Halogen, C₁₋₄-Alkyl, Hydroxymethyl, Alkoxycarbonyl, Aminocarbonyl und -OCH₂CH₂O-Gruppe oder Piperazinylgruppe, die gegebenenfalls an N(4) durch die Gruppen substituiert sein können, die aus C₁₋₄-Alkyl, Benzyl, -Alkyloxycarbonyl und Phenyl ausgewählt sind, die gegebenenfalls durch 1 oder 2 Gruppen substituiert sein können, die aus Wasserstoff, Halogen, C₁₋₄-Alkyl, Alkoxygruppe ausgewählt sind;
R₅, R₆, R₇ und R₈ unabhängig aus der Gruppe ausgewählt sind, die aus Wasserstoff, Chlor, Fluor, C₁₋₂-Alkyl, C₁₋₂-Alkoxy, Cyanogruppe besteht, oder
R₆ und R₇ zusammen mit den Atomen, woran sie angebracht sind, 2,3-Dihydro-[1,4]dioxin oder 2,5-Dihydro-Furanring bilden können;
und/oder Enantiomere und/oder Racemate und/oder Diastereomere und/oder deren pharmazeutisch akzeptable Salze, die mit Säuren oder Basen gebildet sind.

4. Verbindung zur Verwendung in einem Verfahren der Verhinderung und/oder Behandlung von mGluR5-Rezeptorvermittelten Störungen gemäß Anspruch 1, worin die Verbindung ausgewählt ist aus:
3-(3,4-Dimethylbenzolsulfonyl)-4-(morpholin-4-yl)chionolin,
3-(4-Methylbenzolsulfonyl)4-(3-methylpiperidin-1-yl)chinolin,
3-(3,4-Dimethylbenzolsulfonyl)-4-(4-methylpiperidin-1-yl)chinolin,
3-(4-Methylbenzolsulfonyl(-4-(4-methylpiperidin-1-yl)chonolin,
3-benzolsulfonyl-4-(piperidin-1-yl)chinolin,
3-(4-Methylbenzolsulfonyl)-4-(piperidin-1-yl)chinolin,
4-Benzylamino-3-(4-methylbenzolsulfonyl)chinolin,
6-ethyl-4-(4-methylpiperidin-1-yl)-3-(4-methoxybenzolsulfonyl)chinolin,
6-Fluor-3-(4-methylbenzolsulfonyl)-4-(4-methylpiperidin-1-yl)chinolin,
6-Ethoxy-3-(4-chlorbenzolsulfonyl)-4-(4-fluorbenzylamino)chinolin,
4-(Azepan-1-yl)-3-(4-methylbenzolsulfonyl)chinolin,
4-(Azepan-1-yl)-3-(4-chlorbenzolsulfonyl)chinolin,
6-Methyl-3-(4-methylbenzolsulfonyl)-4-(4-methylpiperidin-1-yl)chinolin,
4-(4-Methylpiperidin-1-yl)-3-benzolsulfonylchinolin,
9-(4-Methylpiperidin-1-yl)-8-benzolsulfonyl-2,3-dihydro[1,4]dioxin[2,3-g]chinolin,
6-Ethyl-4-(4-ethyloxycarbonylpiperidin-1-yl)-3-(4-chlorbenzolsulfonyl)chinolin,
4-Diethylamino-3-(4-methylbenzolsulfonyl)chinolin, 4-(4-Benzylpiperazin-1-yl)-3-(4-chlorbenzolsulfonyl)chinolin,
4-(Azepan-1-yl)-3-benzolsulfonylchinolin,
3-(3-Cyanobezolsulfonyl)-6-fluor-4-(4-methylpiperidin-1-yl)chinolin,
3-(3-Cyanobenzolsulfonyl)-6-fluor-4-(4-methylpiperidin-1-yl)chinolin,
6-Fluor-3-(4-methoxybenzolsulfonyl)-4-(4-methylpiperidin-1-yl)chinolin,
6-Fluor-3-(4-methoxybenzolsulfonyl)-4-(4-methylpiperidin-1-yl)chinolin,
6-Fluor-3-(3,4-dimethylbenzolsulfonyl)-4-(4-methylpiperidin-1-yl)chinolin,
3-(3-Chlor-4-methoxybenzolsulfonyl)-6-fluor-4-(4-methylpiperidin-1-yl)chinolin,
3-(3-Chlor-4-fluorbenzolsulfonyl)-6-fluor-4-(4-methylpiperidin-1-yl)chinolin,
3-(3,4-Dichlorbenzolsulfonyl)-6-fluor-4-(4-methylpiperidin-1-yl)chinolin,
3-(3-Chlorbenzolsulfonyl)-6-fluor-4-(4-methylpiperidin-1-yl)chinolin,
3-(4-Chlorbenzolsulfonyl)-6-methyl-4-(4-methylpiperidin-1-yl)chinolin,
3-(3-Fluorbenzolsulfonyl)-6-methyl-4-(4-methylpiperidin-1-yl)chinolin,
3-(3-Methoxybenzolsulfonyl)-6-methyl-4-(4-methylpiperidin-1-yl)chinolin,
3-(3,4Dimethylbenzolsulfonyl)-6-methyl-4-(4-methylpiperidin-1-yl)chinolin,
3-(3-Chlor-4-methoxybenzolsulfonyl)-6-methyl-4-(4-methylpiperidin-1-yl)chinolin,
3-(3-Chlorbenzolsulfonyl)-6-methyl-4-(4-methylpiperidin-1-yl)chinolin,
3-(3-Fluor-4-methylbenzolsulfonyl)-6-methyl-4-(4-methylpiperidin-1-yl)chinolin,
3-(3-Chlor-4-methylbenzolsulfonyl)-6-methyl-4-(4-methylpiperidin-1-yl)chinolin,
3-(3-Chlor-4-fluorbenzolsulfonyl)-6-methyl-4-(4-methylpiperidin-1-yl)chinolin,
3-(3,4-Dichlorbenzolsulfonyl)-7-fluor-4-(4-methylpiperidin-1-yl)chinolin,
7-Fluor-3-(3-cyanobenzolsulfonyl)-4-(4-methylpiperidin-1-yl)chinolin,
7-Fluor-3-(4-cyanobenzolsulfonyl)-4-(4-methylpiperidin-1-yl)chinolin,
3-(3-Chlor-4-methylbenzolsulfonyl)-7-fluor-4-(4-methylpiperidin-1-yl)chinolin,
7-Fluor-3-(3-methoxybenzolsulfonyl)-4-(4-methylpiperidin-1-yl)chinolin,
3-(3,4-Difluorbenzolsulfonyl)-7-fluor-4-(4-methylpiperidin-1-yl)chinolin,
3-(3-Chlor-4-fluorbenzolsulfonyl)-7-fluor-4-(4-methylpiperidin-1-yl)chinolin,
7-Chlor-3-(3,5-dichlorbenzolsulfonyl)-4-(4-methylpiperidin-1-yl)chinolin,
7-Chlor-3-(3,5-difluorbenzolsulfonyl)-4-(4-methylpiperidin-1-yl)chinolin,
7-Chlor-3-(3-cyanobenzolsulfonyl)-4-(4-methylpiperidin-1-yl)chinolin,
7-Chlor-3-(4-methylbenzolsulfonyl)-4-(4-methylpiperidin-1-yl)chinolin,
7-Chlor-3-(4-fluorbenzolsulfonyl)-4-(4-methylpiperidin-1-yl)chinolin,
3-Benzolsulfonyl-7-chlor-4-(4-methylpiperidin-1-yl)chinolin,
7-Chlor-3-(4-chlorbenzylsulfonyl)-4-(4-methylpiperidin-1-yl)chinolin,
7-Chlor-3-(3,4-dimethoxybenzylsulfonyl)-4-(4-methylpiperidin-1-yl)chinolin,
7-Chlor-3-(3-fluorbenzylsulfonyl)-4-(4-methylpiperidin-1-yl)chinolin,
7-Chlor-3-(3-methoxybenzylsulfonyl)-4-(4-methylpiperidin-1-yl)chinolin,
7-Chlor-3-(3-chlor-4-methoxybenzylsulfonyl)-4-(4-methylpiperidin-1-yl)chinolin,
7-Chlor-3-(3-chlorbenzylsulfonyl)-4-(4-methylpiperidin-1-yl)chinolin,
7-Chlor-3-(3-chlor-4-fluorbenzylsulfonyl)-4-(4-methylpiperidin-1-yl)chinolin,
6-Chlor-3-(3,5-difluorbenzylsulfonyl)-4-(4-methylpiperidin-1-yl)chinolin,
6-Chlor-3-(4-methylbenzylsulfonyl)-4-(4-methylpiperidin-1-yl)chinolin,
6-Chlor-3-(4-chlorbenzylsulfonyl)-4-(4-methylpiperidin-1-yl)chinolin,
8-Fluor-3-(3-fluorbenzylsulfonyl)-4-(4-methylpiperidin-1-yl)chinolin,
8-Fluor-3-(4-methylbenzylsulfonyl)-4-(4-methylpiperidin-1-yl)chinolin,
3-(3,4-Dichlorbenzylsulfonyl)-8-fluor-4-(4-methylpiperidin-1-yl)chinolin,
3-(3-Chlorbenzylsulfonyl)-8-fluor-4-(4-methylpiperidin-1-yl)chinolin,
3-(3,4-Difluorbenzylsulfonyl)-8-fluor-4-(4-methylpiperidin-1-yl)chinolin,
3-Benzylsulfonyl-6-methyl-4-(morpholin-1-yl)chinolin, 3-(3-Chlorbenzylsulfonyl)-6-methoxy-4-(morpholin-1-yl)chinolin,
3-Benzylsulfonyl-6-fluor-4-(morpholin-1-yl)chinolin, 6-Chlor-3-(4-chlorbenzylsulfonyl)-4-(morpholin-1-yl)chinolin,
3-(3-Chlor-4-methylbenzylsulfonyl)-7-fluor-4-(morpholin-1-yl)chinolin,
3-(3,4-Dichlorbenzylsulfonyl)-7-fluor-4-(morpholin-1-yl)chinolin,
7-Chlor-3-(3,5-Dichlorbenzylsulfonyl)-4-(morpholin-1-yl)chinolin,
7-Chlor-3-(3,4-dimethylbenzylsulfonyl)-4-(morpholin-1-yl)chinolin,
7-Chlor-3-(3-chlorbenzylsulfonyl)-4-(morpholin-1-yl)chinolin,
7-Chlor-3-(3-chlor-4-methylbenzylsulfonyl)-4-(morpholin-1-yl)chinolin,
7-Chlor-3-(3,4-dichlorbenzylsulfonyl)-4-(morpholin-1-yl)chinolin,
7-Chlor-3-(3-chlor-4-fluorbenzylsulfonyl)-4-(morpholin-1-yl)chinolin.

5. Verbindung, wie in einem der Ansprüche 1 bis 4 beansprucht, worin die mGluR5-Rezeptor-vermittelten Störungen psychiatrische Störungen sind.

6. Verbindung, wie in einem der Ansprüche 1 bis 4 beansprucht, worin die mGluR5-Rezeptor-vermittelten Störungen neurologische Störungen sind.

7. Verbindung, wie in einem der Ansprüche 1 bis 4 beansprucht, worin die mGluR5-Rezeptor-vermittelten Störungen chronische und akute Schmerzstörungen sind.

8. Verbindung, wie in einem der Ansprüche 1 bis 4 beansprucht, worin die mGluR5-Rezeptor-vermittelten Störungen neuromuskulare Funktionsstörungen des unteren Harntrakts und Magen-Darm-Störungen sind.

9. Verfahren zur Herstellung einer Verbindung der Formel (I) 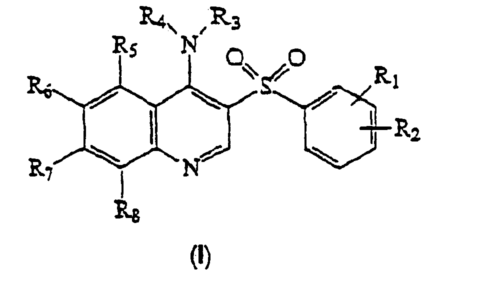 worin
R₁ und R₂ unabhängig aus der Gruppe ausgewählt sind, die aus Wasserstoff, Halogen, Alkyl, Alkoxy, Cyano, optional substituierte Aminogruppe oder gesättigter heterocyclischen Gruppe besteht, worin das Heteroatom N ist;
R₃ und R₄ unabhängig aus der Gruppe ausgewählt sind, die aus Wasserstoff, Alkyl, substituierter Arylgruppe mit mindestens einem Substituenten, der aus der Gruppe aus Wasserstoff, Halogen, Alkyl, Alkoxygruppe ausgewählt sind, oder
R₃ und R₄ zusammen mit dem N-Atom, woran sie angebracht sind, eine C₅₋₇-heterocyclische Gruppe bilden können, die 1 oder 2 Heteroatome enthält, die aus der Gruppe aus N, O ausgewählt sind, die gegebenenfalls mit den Gruppen substituiert sein können, die aus Wasserstoff, Halogen, Alkyl, Alkylhydroxy, Alkyloxycarbonyl, Aminocarbonyl, -OCH₂CH₂O-, Benzyl und substituierte Phenylgruppe besteht;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptablen Salze, die mit Säuren oder Basen gebildet sind.
a. Umwandeln einer Verbindung der Formel (VI): 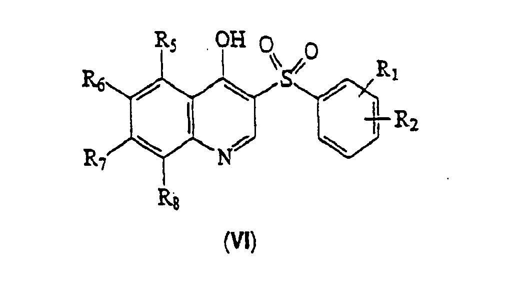 worin R₁, R₂, R₅, R₆, R₇ und R₈ wie vorstehend für eine Verbindung der Formel (I) definiert sind, in eine Verbindung der Formel (VII): 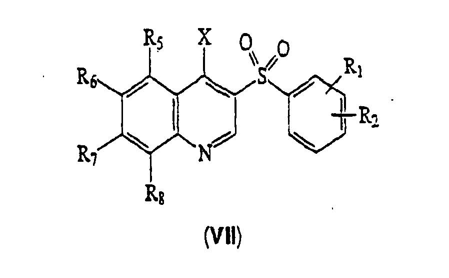 worin X aus Halogen, Benzolsulfonyloxy- oder Trifluormethansulfonyloxygruppen ausgewählt ist und R₁, R₂, R₅, R₆, R₇ und R₈ wie vorstehend für eine Verbindung der Formel (I) definiert sind, danach Umsetzen der erhaltenen Verbindung der Formel (VII) mit einer Verbindung der Formel (VIII): 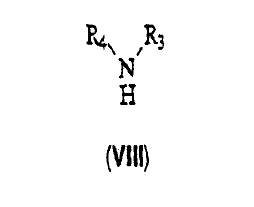 worin R₃ und R₄ wie vorstehend für eine Verbindung der Formel (I) definiert sind, um eine Verbindung der Formel (I) zu erhalten, und gegebenenfalls erzeugen von Enantiomeren und/oder Racematen und/oder Diastereomeren und/oder ihren pharmazeutisch akzeptablen Salzen, oder
b. Umsetzen einer Verbindung der Formel (XV): 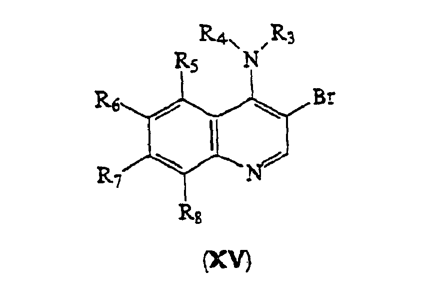 worin R₃, R₄, R₅, R₆, R₇ und R₈, wie vorstehend für eine Verbindung der Formel (I) definiert sind, mit einer Verbindung der Formel (III): 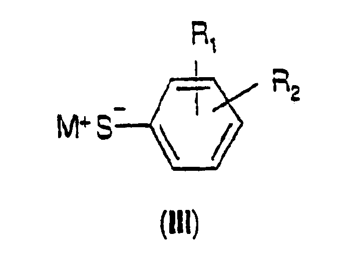 worin M aus Alkali oder Erdalkalimetallen ausgewählt ist, R₁ und R₂ wie vorstehend für eine Verbindung der Formel (I) definiert sind, um eine Verbindung der Formel (XVI) zu ergeben: 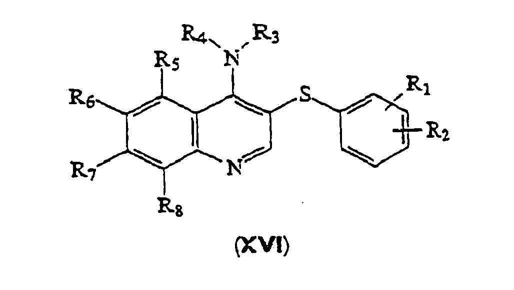 worin R₁, R₂, R₃, R₄, R₅, R₆, R₇ und R₈ wie vorstehend für eine Verbindung der Formel (I) definiert sind, danach Oxidieren einer Verbindung der Formel (XVI), um eine Verbindung der Formel (XVII) zu erhalten: 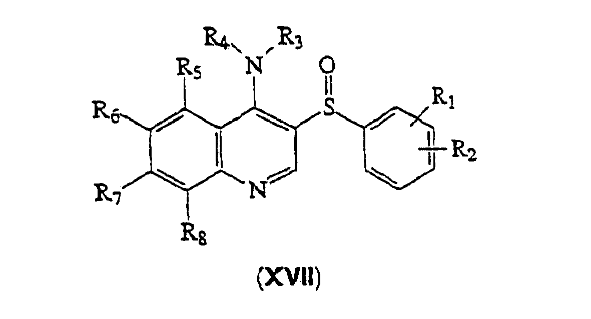 worin R₁, R₂, R₃, R₄, R₅, R₆, R₇ und R₈ wie vorstehend für eine Verbindung der Formel (I) definiert sind, danach Oxidieren einer Verbindung der Formel (XVII), um eine Verbindung der Formel (I) zu erhalten, und gegebenenfalls Erzeugen von Enantiomeren und/oder Racematen und/oder Diastereomeren und/oder deren pharmazeutisch akzeptablen Salze oder
c. Umwandeln einer Verbindung der Formel (I), worin die Bedeutung von worin R₁, R₂, R₃, R₄, R₅, R₆, R₇ und R₈ wie vorstehend für die Formel (I) für eine unterschiedliche Verbindung der Formel (I) in eine andere Verbindung der Formel (I), worin die Bedeutung von worin R₁, R₂, R₃, R₄, R₅, R₆, R₇ und R₈ wie vorstehend für eine Verbindung der Formel (I) beschrieben ist;
soweit zweckmäßig, Separieren der Enantiomere und/oder Racemate und/oder Diasteromere der Verbindungen der Formel (I), worin die Bedeutung R₁, R₂, R₃, R₄, R₅, R₆, R₇ und R₈ wie vorstehend für die Formel (I) beschrieben ist, durch herkömmliche Verfahren;
und gegebenenfalls danach Erzeugen von Salzen und/oder Hydraten und/oder Solvaten der Verbindungen der Formel (I).

10. Verfahren zur Herstellung einer Verbindung der Formel (VI) : 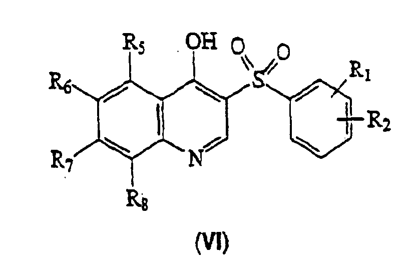 worin R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigter heterocyclischer Gruppe besteht, worin das Heteroatom N ist;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptablen Salze, die mit Säuren oder Basen gebildet sind.
a. Umsetzen einer Verbindung der Formel (II): 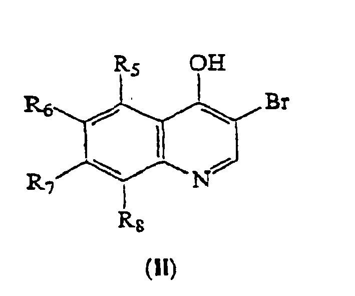 worin R₅, R₆, R₇ und R₈ wie vorstehend für eine Verbindung der Formel (I) definiert sind, mit einer Verbindung der Formel (III): 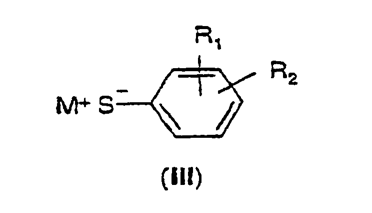 worin M aus Alkalimetallen oder Erdalkalimetallen ausgewählt ist, R₁ und R₂ wie vorstehend für eine Verbindung der Formel (I) definiert sind, um eine Verbindung der Formel (IV) zu erhalten: 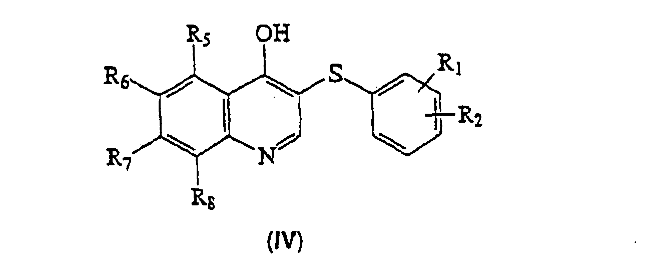 worin R₁, R₂, R₅, R₆, R₇ und R₈ wie vorstehend für eine Verbindung der Formel (I) definiert sind, danach Oxidieren einer Verbindung der Formel (IV), um eine Verbindung der Formel (V) zu erhalten: 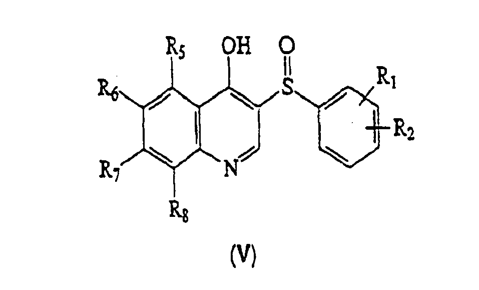 worin R₁, R₂, R₅, R₆, R₇ und R₈ wie vorstehend für eine Verbindung der Formel (I) definiert sind, danach Oxidieren einer mit einer Verbindung der Formel (V), um eine Verbindung der Formel (VI) zu erhalten, und gegebenenfalls erzeugen on Enantiomeren und/oder Racematen und/oder Diastereomeren und/oder pharmazeutisch akzeptablen Salzen oder
b. Umsetzen einer Verbindung der Formel (IX): 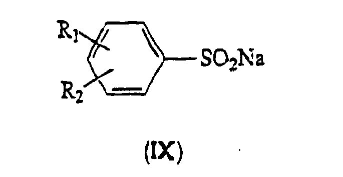
worin R₁ und R₂ wie vorstehend für eine Verbindung der Formel (I) definiert sind, mit α-Halogen-Essigsäureestern der Formel (X):
Hlg-CH₂-COOR₉ (X)
worin Hlg Halogen ist und R₉ eine Ethyl- oder Methylgruppe ist, um eine Verbindung der Formel (XI) zu erhalten 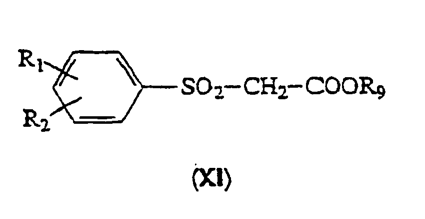 worin R₁ und R₂ wie vorstehend für eine Verbindung der Formel (I) definiert sind und R₉ wie vorstehend für Verbindungen der Formel (X) definiert ist; Umsetzen einer Verbindung der Formel (XI) mit Trialkylorthoformat der Formel (XII):
CH(OR₁₀)₃ (XII)
worin R₁₀ eine Ethyl- oder Methylgruppe ist, um eine Verbindung der Formel (XIII) zu erhalten: 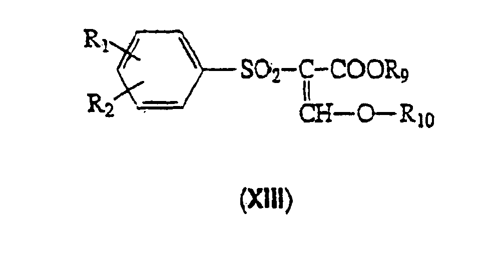 worin R₁ und R₂ wie vorstehend für eine Verbindung der Formel (I) definiert sind, R₉ wie vorstehend für Verbindungen der Formel (X) definiert ist und R₁₀ wie vorstehend für Verbindungen der Formel (XII) definiert ist; Umsetzen einer Verbindung der Formel (XIII) mit einem Anilinderivat der Formel (XIV) : 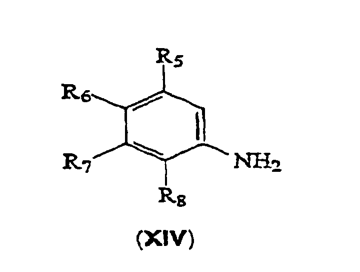 worin R₅, R₆, R₇ und R₈ wie vorstehend für eine Verbindung der Formel (I) definiert sind, um eine Verbindung der Formel (VI) zu erhalten, und optional Erzeugen von Enantiomeren und/oder Racematen und/oder Diastereomeren und/oder deren pharmazeutisch akzeptable Salze.

11. Intermediat mit der Formel (IV) 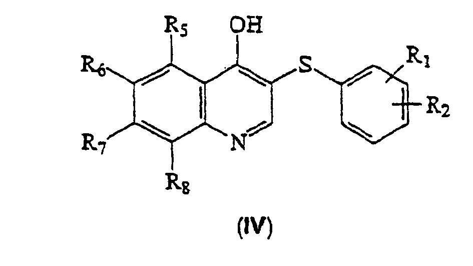 worin
R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigte heterocyclischer Gruppe besteht, worin das Heteroatom N ist;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und /oder Enantiomere und/oder Racemate und/oder Diastereomere und/oder deren pharmazeutisch akzeptable Salze, die mit Säuren oder Basen gebildet werden.

12. Intermediat mit der Formel (V) 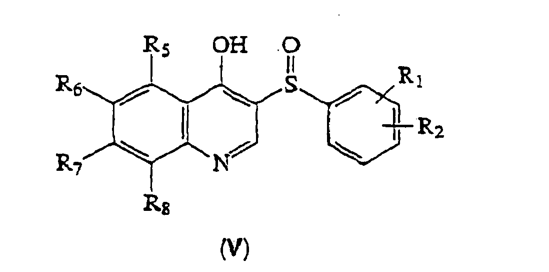 worin
R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigter heterocyclischer Gruppe besteht, worin das Heteroatom N ist;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptable Salze, die mit Säuren oder Basen gebildet werden.

13. Intermediat mit der Formel (VI) 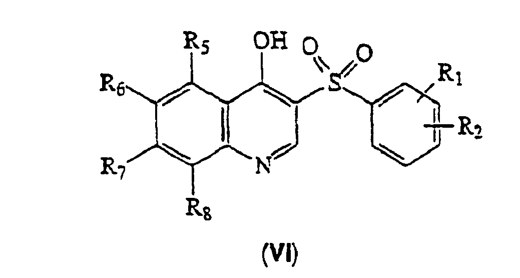 worin
R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigter heterocyclischer Gruppe besteht, worin das Heteroatom N ist;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptable Salze, die mit Säuren oder Basen gebildet werden.

14. Intermediat mit der Formel (VII) 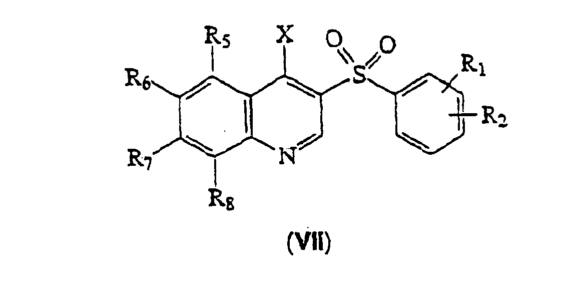 worin X aus der Gruppe ausgewählt ist, die aus Halogen, Benzolsulfonyloxy- oder Trifluormethansulfonyloxygruppe besteht,
R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigter heterocyclischer Gruppe besteht, worin das Heteroatom N ist;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptable Salze, die mit Säuren oder Basen gebildet werden.

15. Intermediat mit der Formel (XVI) 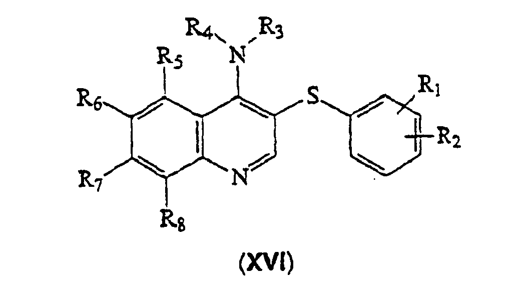 worin
R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigter heterocyclischer Gruppe besteht, worin das Heteroatom N ist;
R₃ und R₄ unabhängig aus der Gruppe ausgewählt sind, die aus Wasserstoff, Alkyl, substituierte Arylgruppe mit mindestens einem Substituenten, der aus der Gruppe aus Wasserstoff, Halogen, Alkyl, Alkoxygruppe ausgewählt ist, oder
R₃ und R₄ zusammen mit dem N-Atom, woran sie angebracht sind, eine C₅₋₇-heterocyclische Gruppe bilden können, die 1 oder 2 Heteroatome enthält, die aus der Gruppe aus N, O ausgewählt sind, die gegebenenfalls durch die Gruppen substituiert sein können, die aus Wasserstoff, Halogen, Alkyl, Alkylhydroxy, Alkyloxycarbonyl, Aminocarbonyl, -OCH₂CH₂O-, Benzyl und substituierte Phenylgruppe ausgewählt sind;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptable Salze, die mit Säuren oder Basen gebildet werden können.

16. Intermediat mit der Formel (XVII) 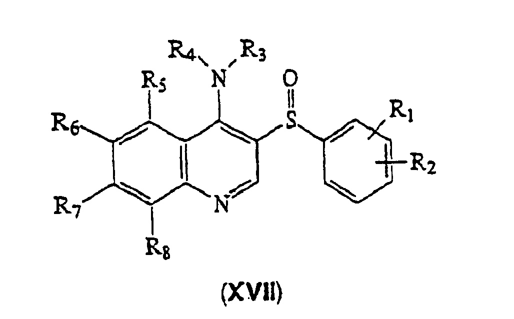 worin
R₁ und R₂ unabhängig aus der Gruppe ausgewählt sind, die aus Wasserstoff, Halogen, Alkyl, Alkoxy, Cyano, gegebenenfalls substituierte Aminogruppe oder gesättigter heterocyclischer Gruppe besteht, worin das Heteroatom N ist;
R₃ und R₄ unabhängig aus der Gruppe ausgewählt sind, die aus Wasserstoff, Alkyl, substituierte Arylgruppe mit mindestens einem Substituenten, der aus der Gruppe aus Wasserstoff, Halogen, Alkyl, Alkoxygruppe ausgewählt ist, oder
R₃ und R₄ zusammen mit dem N-Atom, woran sie angebracht sind, eine C₅₋₇-heterocyclische Gruppe bilden können, die 1 oder 2 Heteroatome enthält, die aus der Gruppe aus N, O ausgewählt sind, die gegebenenfalls durch die Gruppen substituiert sein können, die aus Wasserstoff, Halogen, Alkyl, Alkylhydroxy, Alkyloxycarbonyl, Aminocarbonyl, -OCH₂CH₂O-, Benzyl und substituierte Phenylgruppe ausgewählt sind;
R₅, R₆, R₇ und R₈ unabhängig aus der Gruppe ausgewählt sind, die aus Wasserstoff, Halogen, Alkyl, Alkoxy, Cyanogruppe besteht, oder
R₆ und R₇ zusammen mit den Atomen, woran sie angebracht sind, eine ungesättigte heterocyclische Gruppe bilden können;
und/oder Enantiomere und/oder Racemate und/oder Diastereomere und/oder deren pharmazeutisch akzeptable Salze, die mit Säuren oder Basen gebildet können.

17. Pharmazeutische Formulierung, die als einen aktiven Wirkstoff eine therapeutisch effektive Menge einer Verbindung der Formel (I) umfasst 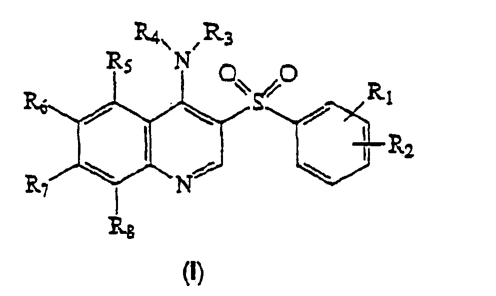 wie in irgendeinem der Ansprüche 1 bis 3 formuliert, mit einen oder mehreren pharmazeutisch akzeptablen Verdünnungsmitteln, Auszügen und/oder inerten Trägern.

18. Pharmazeutische Formulierung gemäß Anspruch 17, zur Verwendung bei der Verhinderung und/oder Behandlung von mGluR5-Rezeptor-vermittelten Störungen.

19. Verwendung einer Verbindung der Formel (I) 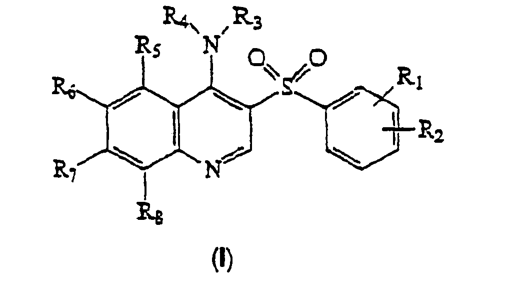 wie in irgendeinem der Ansprüche 1 bis 3 definiert, bei der Herstellung eines Medikaments zur Verhinderung und/oder Behandlung von mGluR5-Rezeptor-vermittelten Störungen.

20. Verwendung gemäß Anspruch 19, worin die mGluR5-Rezeptorvermittelten Störungen psychiatrische Störungen sind.

21. Verwendung gemäß Anspruch 19, worin die mGluR5-Rezeptorvermittelten Störungen neurologische Störungen sind.

22. Verwendung gemäß Anspruch 19, worin die mGluR5-Rezeptorvermittelten Störungen chronischer und akuter Schmerz sind.

23. Verwendung gemäß Anspruch 19, worin die mGluR5-Rezeptorvermittelten Störungen neuromuskuläre Dysfunktionen des unteren Harntrakts und gastrointestinale Störungen sind.

## Revendications

1. Composé répondant à la formule (I) à utiliser dans un procédé de prévention et/ou de traitement de troubles véhiculés par le récepteur mGluR5, 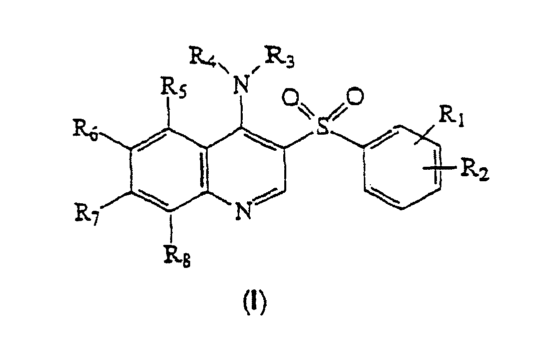 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₃ et R₄ sont indépendamment choisis dans le groupe consistant en un atome d'hydrogène, un groupe alkyle, un groupe aryle substitué comportant au moins un substituant choisi dans le groupe consistant en un atome d'hydrogène, d'halogène, un groupe alkyle ou alcoxy, ou
R₃ et R₄ conjointement avec l'atome N auquel ils sont attachés peuvent former un groupe hétérocyclyle en C_{5 à 7}, contenant 1 ou 2 hétéroatomes choisis dans le groupe de N, O, qui peut être facultativement substitué par les groupes choisis parmi un atome d'hydrogène, d'halogène, un groupe alkyle, alkylhydroxy, alkyloxycarbonyle, aminocarbonyle, -OCH₂CH₂O-, benzyle et phényle substitué ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

2. Composé répondant à la formule (I) à utiliser dans un procédé de prévention et/ou de traitement de troubles véhiculés par le récepteur mGluR5, 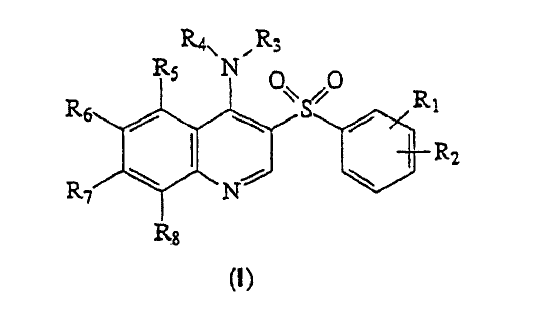 dans laquelle
R₁ et R₂ sont indépendamment choisis dans le groupe consistant en un atome d'hydrogène, d'halogène, un groupe alkyle en C_{1 à 4}, alcoxy en C_{1 à 4}, cyano, amino facultativement substitué ou hétérocyclyle saturé, où l'hétéroatome est N ;
R₃ et R₄ sont indépendamment choisis dans le groupe consistant en un atome d'hydrogène, un groupe alkyle en C_{1 à 4}, un groupe aryle substitué comportant au moins un substituant choisi dans le groupe constitué d'un atome d'hydrogène, d'un atome d'halogène, d'un groupe alkyle en C_{1 à 4} ou alcoxy en C_{1 à 4}, ou
R₃ et R₄ conjointement avec l'atome N auquel ils sont attachés peuvent former un groupe hétérocyclyle en C_{5 à 7}, contenant 1 ou 2 hétéroatomes choisis dans le groupe consistant en N, O, qui peut être facultativement substitué par les groupes choisis parmi un atome d'hydrogène, d'halogène, un groupe alkyle en C_{1 à 4}, alkylhydroxy en C_{1 à 4}, alkyloxycarbonyle, aminocarbonyle, -OCH₂CH₂O-, benzyle et phényle substitué, qui peuvent être facultativement substitués par 1 ou 2 groupes choisis parmi un atome d'hydrogène, d'halogène, un groupe alkyle en C_{1 à 4} ou alcoxy en C_{1 à 4} ;
R₅, R₆, R₇ et R₈ sont indépendamment choisis dans le groupe consistant en un atome d'hydrogène, d'halogène, un groupe alkyle en C_{1 à 4}, alcoxy en C_{1 à 4} ou cyano, ou
R₆ et R₇ conjointement avec les atomes auxquels ils sont attachés peuvent former un groupe hétérocyclyle insaturé à 5 à 7 chaînons, contenant 1 ou 2 atomes O ;
et/ou leurs énantiomères et/ou racémates et/ou diastéréoisomères et/ou sels pharmaceutiquement acceptables formés avec des acides ou des bases.

3. Composé répondant à la formule (I) à utiliser dans un procédé de prévention et/ou de traitement de troubles véhiculés par le récepteur mGluR5, 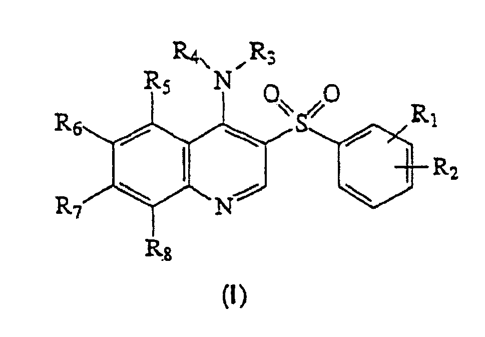 dans laquelle
R₁ et R₂ sont indépendamment choisis dans le groupe consistant en un atome d'hydrogène, un groupe chloro, fluoro, alkyle en C_{1 à 2}, alcoxy en C_{1 à 2}, cyano ou pipéridinyle ;
R₃ et R₄ sont indépendamment choisis dans le groupe consistant en un atome d'hydrogène, un groupe alkyle en C_{1 à 2}, un groupe benzyle substitué par 1 ou 2 groupes indépendamment choisis parmi un atome d'hydrogène, d'halogène, un groupe alkyle en C_{1 à 4} ou alcoxy en C_{1 à 4},
R₃ et R₄ conjointement avec les atomes N auxquels ils sont attachés peuvent former un groupe pyrrolidinyle, homopipéridinyle, morpholinyle ou pipéridinyle qui peuvent être facultativement substitués par les groupes choisis parmi un atome d'hydrogène, un atome d'halogène, un groupe alkyle en C_{1 à 4}, hydroxyméthyle, alkyloxycarbonyle, aminocarbonyle et - OCH₂CH₂O- ou un groupe pipérazinyle qui peuvent être substitués en N(4) par les groupes choisis parmi alkyle en C_{1 à 4}, benzyle, alkyloxycarbonyle et phényle, qui peuvent être facultativement substitués par 1 ou 2 groupes choisis parmi un atome d'hydrogène, un atome d'halogène, un groupe alkyle en C_{1 à 4} ou alcoxy ;
R₅, R₆, R₇ et R₈ sont indépendamment choisis dans le groupe consistant en un atome d'hydrogène, un groupe chloro, fluoro, alkyle en C₁ as alcoxy en C_{1 à 2} ou cyano, ou
R₆ et R₇ conjointement avec les atomes auxquels ils sont attachés peuvent former un cycle 2,3-dihydro-[1,4]dioxine ou 2,5-dihydro-furane ;
et/ou leurs énantiomères et/ou racémates et/ou diastéréoisomères et/ou sels pharmaceutiquement acceptables formés avec des acides ou des bases.

4. Composé à utiliser dans un procédé de prévention et/ou de traitement de troubles véhiculés par le récepteur mGluR5 selon la revendication 1, dans lequel le composé est choisi parmi :
la 3-(3,4-diméthyl)-benzènesulfonyl)-4-(morpholin-4-yl)quinoléine,
la 3-(4-méthyl-benzènesulfonyl)-4-(3-méthyl-pipéridin-1-yl)-quinoléine
la 3-(3,4-diméthyl-benzènesulfonyl)-4-(9-méthyl-pipéridin-1-yl)-quinoléine
la 3-(4-méthyl-benzènesulfonyl)-4-(4-méthyl-pipéridin-1-yl)-quinoléine,
la 3-benzènesulfonyl-4-(pipéridin-1-yl)-quinoléine,
la 3-(4-méthyl-benzènesulfonyl)-4-(pipéridin-1-yl)-quinoléine,
la 4-benzylamino-3-(4-méthyl-benzènesulfonyl)-quinoléine
la 6-éthyl-4-(4-méthyl-pipéridin-1-yl)-3-(4-méthoxy-benzènesulfonyl)-quinoléine,
la 6-fluoro-3-(4-méthyl-benzènesulfonyl)-4-(4-méthyl-pipéridin-1-yl)-quinoléine,
la 6-éthoxy-3-(4-chloro-benzènesulfonyl)-4-(4-fluoro-benzylamino)-quinoléine,
la 4-(azépan-1-yl)-3-(4-méthyl-benzènesulfonyl)-quinoléine
la 4-(azépan-1-yl)-3-(4-chloro-benzènesulfonyl)-quinoléine
la 6-méthyl-3-(4-méthyl-benzènesulfonyl)-4-(4-méthyl-pipéridin-1-yl)-quinoléine,
la 4-(4-méthylpipéridin-1-yl)-3-benzènesulfonyl-quinoléine
la 9-(4-méthyl-pipéridin-1-yl)-8-benzènesulfonyl-2,3-dihydro-[1,4]dioxino[2,3-g]quinoléine,
la 6-éthyl-4-(4-éthyloxycarbonyl-pipéridin-1-yl)-3-(4-chloro-benzènesulfonyl)-quinoléine,
la 4-diéthylamino-3-(4-méthyl-benzènesulfonyl)-quinoléine
la 4-(4-benzyl-pipérazin-1-yl)-3-(4-chloro-benzènesulfonyl)-quinoléine,
la 4-(azépan-1-yl)-3-benzènesulfonyl-quinoléine,
la 3-(3-cyano-benzènesulfonyl)-6-fluoro-4-(4-méthyl-pipéridin-1-yl)-quinoléine,
la 6-fluoro-3-(4-méthoxy-benzènesulfonyl)-4-(4-méthyl-pipéridin-1-yl)-quinoléine,
la 6-fluoro-3-(3-méthoxy-benzènesulfonyl)-4-(4-méthyl-pipéridin-1-yl)-quinoléine,
la 6-fluoro-3-(3,4-diméthyl-benzènesulfonyl)-4-(4-méthyl-pipéridin-1-yl)-quinoléine,
la 3-(3-chloro-4-méthoxy-benzènesulfonyl)-6-fluoro-4-(4-méthyl-pipéridin-1-yl)-quinoléine,
la 3-(3-chloro-4-fluoro-benzènesulfonyl)-6-fluoro-4-(4-méthyl-pipéridin-1-yl)-quinoléine,
la 3-(3,4-dichloro-benzènesulfonyl)-6-fluoro-4-(4-méthyl-pipéridin-1-yl)-quinoléine,
la 3-(3-chloro-benzènesulfonyl)-6-fluoro-4-(4-méthyl-pipéridin-1-yl)-quinoléine,
la 3-(4-chloro-benzènesulfonyl)-6-méthyl-4-(4-méthyl-pipéridin-1-yl)-quinoléine,
la 3-(3-fluoro-benzènesulfonyl)-6-méthyl-4-(4-méthyl-pipéridin-1-yl)-quinoléine,
la 3-(3-méthoxy-benzènesulfonyl)-6-méthyl-4-(4-méthyl-pipéridin-1-yl)-quinoléine,
la 3-(3,4-diméthyl-benzènesulfonyl)-6-méthyl-4-(4-méthyl-pipéridin-1-yl)-quinoléine,
la 3-(3-chloro-4-méthoxy-benzènesulfonyl)-6-méthyl-4-(4-méthyl-pipéridin-1-yl)-quinoléine,
la 3-(3-chloro-benzènesulfonyl)-6-méthyl-4-(4-méthyl-pipéridin-1-yl)-quinoléine,
la 3-(3-fluoro-4-méthyl-benzènesulfonyl)-6-méthyl-4-(4-méthyl-pipéridin-1-yl)-quinoléine,
la 3-(3-chloro-4-méthyl-benzènesulfonyl)-6-méthyl-4-(4-méthyl-pipéridin-1-yl)-quinoléine,
la 3-(3-chloro-4-fluoro-benzènesulfonyl)-6-méthyl-4-(4-méthyl-pipéridin-1-yl)-quinoléine,
la 3-(3,4-dichloro-benzènesulfonyl)-7-fluoro-4-(4-méthyl-pipéridin-1-yl)-quinoléine,
la 7-fluoro-3-(3-cyano-benzènesulfonyl)-4-(4-méthyl-pipéridin-1-yl)-quinoléine,
la 7-fluoro-3-(4-cyano-benzènesulfonyl)-4-(4-méthyl-pipéridin-1-yl)-quinoléine,
la 3-(3-chloro-4-méthyl-benzènesulfonyl)-7-fluoro-4-(4-méthyl-pipéridin-1-yl)-quinoléine,
la 7-fluoro-3-(3-méthoxy-benzènèsulfonyl)-4-(4-méthyl-pipéridin-1-yl)-quinoléine,
la 3-(3,4-difluoro-benzènesulfonyl)-7-fluoro-4-(4-méthyl-pipéridin-1-yl)-quinoléine,
la 3-(3-chloro-4-fluoro-benzènesulfonyl)-7-fluoro-4-(4-méthyl-pipéridin-1-yl)-quinoléine,
la 7-chloro-3-(3,5-dichloro-benzènesulfonyl)-4-(4-méthyl-pipéridin-1-yl)-quinoléine,
la 7-chloro-3-(3,5-difluoro-benzènesulfonyl)-4-(4-méthyl-pipéridin-1-yl)-quinoléine,
la 7-chloro-3-(3-cyano-benzènesulfonyl)-4-(4-méthyl-pipéridin-1-yl)-quinoléine,
la 7-chloro-3-(4-méthyl-benzènesulfonyl)-4-(4-méthyl-pipéridin-1-yl)-quinoléine,
la 7-chloro-3-(4-fluoro-benzènesulfonyl)-4-(4-méthyl-pipéridin-1-yl)-quinoléine,
la 3-benzènesulfonyl-7-chloro-4-(4-méthyl-pipéridin-1-yl)-quinoléine,
la 7-chloro-3-(4-chloro-benzènesulfonyl)-4-(4-méthyl-pipéridin-1-yl)-quinoléine,
la 7-chloro-3-(3,4-diméthoxy-benzènesulfonyl)-4-(4-méthyl-pipéridin-1-yl)-quinoléine,
la 7-chloro-3-(3-fluoro-benzènesulfonyl)-4-(4-méthyl-pipéridin-1-yl)-quinoléine,
la 7-chloro-3-(3-méthoxy-benzènesulfonyl)-4-(4-méthyl-pipéridin-1-yl)-quinoléine,
la 7-chloro-3-(3-chloro-4-méthoxy-benzènesulfonyl)-4-(4-méthyl-pipéridin-1-yl)-quinoléine,
la 7-chloro-3-(3-chloro-benzènesulfonyl)-4-(4-méthyl-pipéridin-1-yl)-quinoléine,
la 7-chloro-3-(3-chloro-4-fluoro-benzènesulfonyl)-4-(4-méthyl-pipéridin-1-yl)-quinoléine,
la 6-chloro-3-(3,5-difluoro-benzènesulfonyl)-4-(4-méthyl-pipéridin-1-yl)-quinoléine,
la 6-chloro-3-(4-méthyl-benzènesulfonyl)-4-(4-méthyl-pipéridin-1-yl)-quinoléine,
la 6-chloro-3-(4-chloro-benzènesulfonyl)-4-(4-méthyl-pipéridin-1-yl)-quinoléine,
la 8-fluoro-3-(3-fluoro-4-méthyl-benzènesulfonyl)-4-(4-méthyl-pipéridin-1-yl)-quinoléine,
la 8-fluoro-3-(4-méthyl-benzènesulfonyl)-4-(4-méthyl-pipéridin-1-yl)-quinoléine,
la 3-(3,4-dichloro-benzènesulfonyl)-8-fluoro-4-(4-méthyl-pipéridin-1-yl)-quinoléine,
la 3-(3-chloro-4-fluoro-benzènesulfonyl)-8-fluoro-4-(4-méthyl-pipéridin-1-yl)-quinoléine,
la 3-(3,4-difluoro-benzènesulfonyl)-8-fluoro-4-(4-méthyl-pipéridin-1-yl)-quinoléine,
la 3-benzènesulfonyl-6-méthyl-4-(morpholin-1-yl)-quinoléine,
la 3-(3-chloro-benzènesulfonyl)-6-méthoxy-4-(morpholin-1-yl)-quinoléine,
la 3-benzènesulfonyl-6-fluoro-4-(morpholin-1-yl)-quinoléine,
la 6-chloro-3-(4-chloro-benzènesulfonyl)-4-(morpholin-1-yl)-quinoléine,
la 3-(3-chloro-4-méthyl-benzènesulfonyl)-7-fluoro-4-(morpholin-1-yl)-quinoléine,
la 3-(3,4-dichloro-benzènesulfonyl)-7-fluoro-4-(morpholin-1-yl)-quinoléine,
la 7-chloro-3-(3,5-dichloro-benzènesulfonyl)-4-(morpholin-1-yl)-quinoléine,
la 7-chloro-3-(3,4-diméthyl-benzènesulfonyl)-4-(morpholin-1-yl)-quinoléine,
la 7-chloro-3-(3-chloro-benzènesulfonyl)-4-(morpholin-1-yl)-quinoléine,
la 7-chloro-3-(3-chloro-4-méthyl-benzènesulfonyl)-4-(morpholin-1-yl)-quinoléine,
la 7-chloro-3-(3,4-dichloro-benzènesulfonyl)-4-(morpholin-1-yl)-quinoléine,
la 7-chloro-3-(3-chloro-4-fluoro-benzènesulfonyl)-4-(morpholin-1-yl)-quinoléine,

5. Composé selon l'une quelconque des revendications 1 à 4, dans lequel lesdits troubles véhiculés par le récepteur mGluR5 sont des troubles psychiatriques.

6. Composé selon l'une quelconque des revendications 1 à 4, dans lequel lesdits troubles véhiculés par le récepteur mGluR5 sont des troubles neurologiques.

7. Composé selon l'une quelconque des revendications 1 à 4, dans lequel lesdits troubles véhiculés par le récepteur mGluR5 sont des troubles de douleur chronique et aiguë.

8. Composé selon l'une quelconque des revendications 1 à 4, dans lequel lesdits troubles véhiculés par le récepteur mGluR5 sont des dysfonctionnements neuromusculaires du tractus urinaire inférieur et des troubles gastro-intestinaux.

9. Procédé de préparation d'un composé de formule (I) 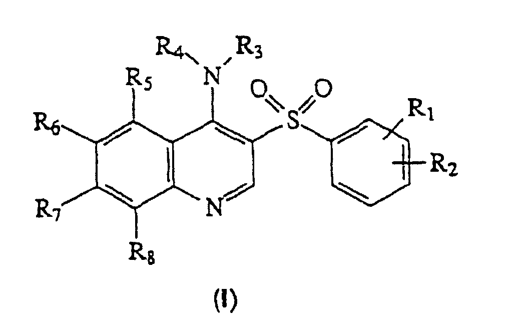 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₃ et R₄ sont indépendamment choisis dans le groupe consistant en un atome d'hydrogène, un groupe alkyle, un groupe aryle substitué comportant au moins un substituant choisi dans le groupe consistant en un atome d'hydrogène, d'halogène, un groupe alkyle ou alcoxy, ou
R₃ et R₄ conjointement avec l'atome N auquel ils sont attachés peuvent former un groupe hétérocyclyle en C_{5 à 7}, contenant 1 ou 2 hétéroatomes choisis dans le groupe de N, O, qui peut être facultativement substitué par les groupes choisis parmi un atome d'hydrogène, d'halogène, un groupe alkyle, alkylhydroxy, alkyloxycarbonyle, aminocarbonyle, -OCH₂CH₂O-, benzyle et phényle substitué ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases ;
a.) conversion d'un composé de formule (VI) : 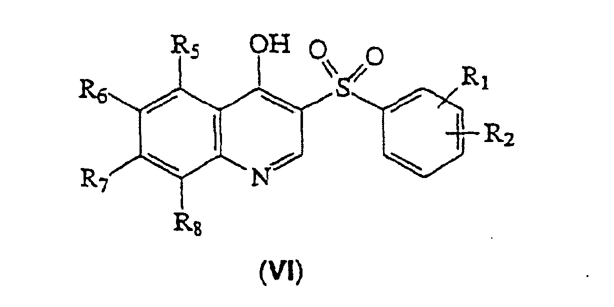 où R₁, R₂, R₅, R₆, R₇ et R₈ sont tels que définis ci-dessus pour un composé de formule (I), en un composé de formule (VII) : 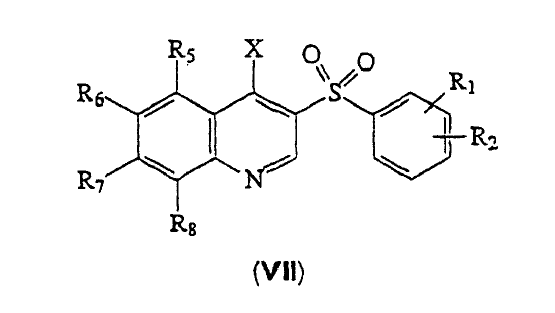 où X est choisi parmi un atome d'halogène, les groupes benzènesulfonyloxy ou trifluorométhanesulfonyloxy et R₁, R₂, R₅, R₆, R₇ et R₈ sont tels que définis ci-dessus pour un composé de formule (I), puis réaction du composé obtenu de formule (VII) avec un composé de formule (VIII) : 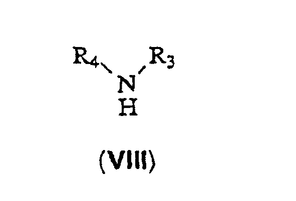 où R₃ et R₄ sont tels que définis ci-dessus pour un composé de formule (I) pour donner un composé de formule (I), et formation facultative d'énantiomères et/ou racémates et/ou diastéréoisomères et/ou sels pharmaceutiquement acceptables de celui-ci, ou
b.) réaction d'un composé de formule (XV) : 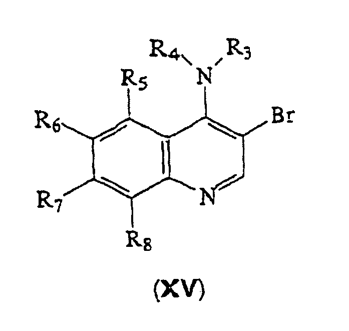 où R₃, R₄, R₅, R₆, R₇ et R₈ sont tels que définis ci-dessus pour un composé de formule (I), avec un composé de formule (III) : 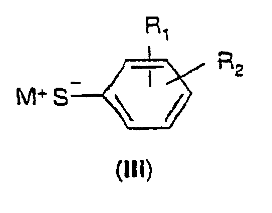 où M est choisi parmi les métaux alcalins ou alcalino-terreux, R₁ et R₂ sont tels que définis ci-dessus pour un composé de formule (I), pour donner un composé de formule (XVI) : 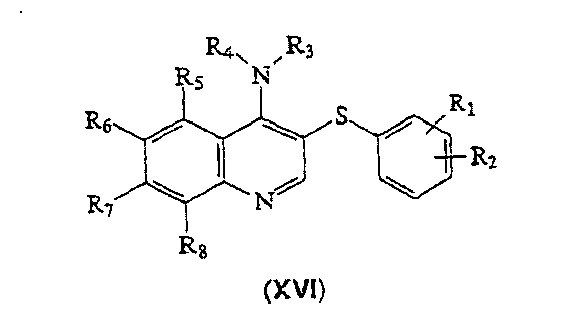 où R₁, R₂, R₃, R₄, R₅, R₆, R₇ et R₈ sont tels que définis ci-dessus pour un composé de formule (I), puis oxydation d'un composé de formule (XVI) pour obtenir un composé de formule (XVII) : 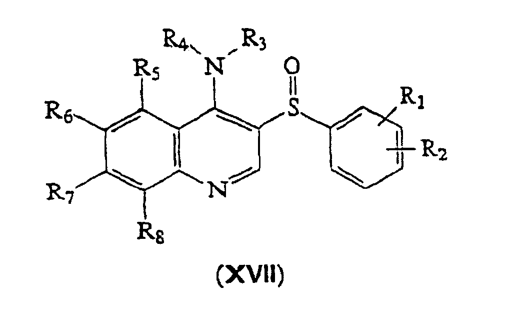 où R₁, R₂, R₃, R₄, R₅, R₆, R₇ et R₈ sont tels que définis ci-dessus pour un composé de formule (I), puis oxydation d'un composé de formule (XVII) pour obtenir un composé de formule (I), et formation facultative d'énantiomères et/ou racémates et/ou diastéréoisomères et/ou sels pharmaceutiquement acceptables de celui-ci, ou
c.) interconversion d'un composé de formule (I), où les significations de R₁, R₂, R₃, R₄, R₅, R₆, R₇ et R₈ sont telles que définies ci-dessus pour la formule (I) en un composé différent de formule (I), où les significations de R₁, R₂, R₃, R₄, R₅, R₆, R₇ et R₈ sont telles que décrites ci-dessus pour la formule (I) ;
lorsque cela est approprié, séparation des énantiomères et/ou racémates et/ou diastéréoisomères des composés de formule (I) , où les significations de R₁, R₂, R₃, R₄, R₅, R₆, R₇ et R₈ sont telles que décrites ci-dessus pour la formule (I) par des procédés classiques ;
puis formation facultative de sels et/ou hydrates et/ou solvates de composés de formule (I).

10. Procédé de préparation d'un composé de formule (VI) : 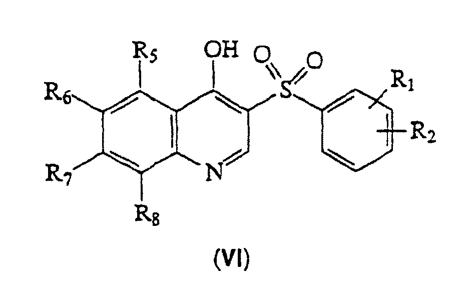 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases ;
a. réaction d'un composé de formule (II) : 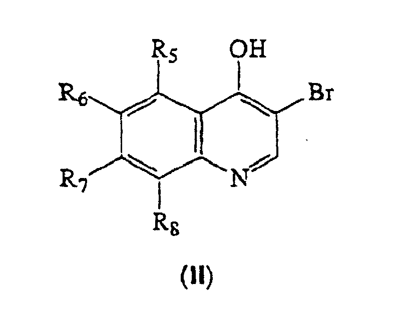 où R₅, R₆, R₇ et R₈ sont tels que définis ci-dessus pour un composé de formule (I), avec un composé de formule (III) : 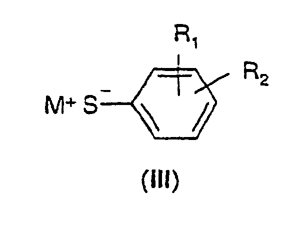 où M est choisi parmi les métaux alcalins ou alcalino-terreux, R₁ et R₂ sont tels que définis ci-dessus pour un composé de formule (I), pour donner un composé de formule (IV) : 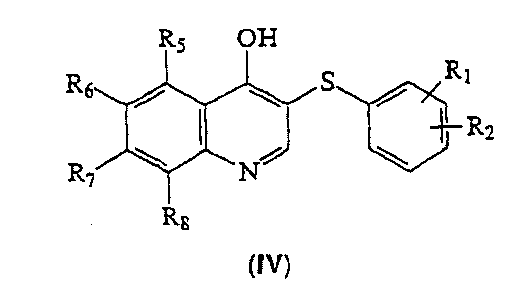 où R₁, R₂, R₅, R₆, R₇ et R₈ sont tels que définis ci-dessus pour un composé de formule (I), puis oxydation d'un composé de formule (IV) pour obtenir un composé de formule (V) : 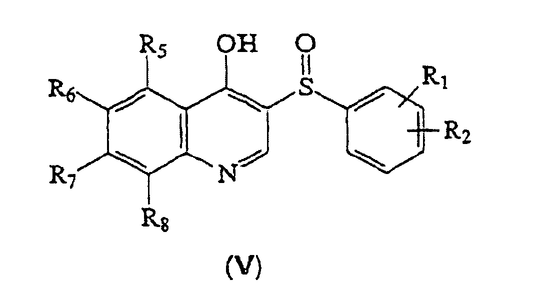 où R₁, R₂, R₅, R₆, R₇ et R₈ sont tels que définis ci-dessus pour un composé de formule (I), puis oxydation d'un composé de formule (V) pour obtenir un composé de formule (VI), et formation facultative d'énantiomères et/ou racémates et/ou diastéréoisomères et/ou sels pharmaceutiquement acceptables de celui-ci ou
b. réaction d'un composé de formule (IX) : 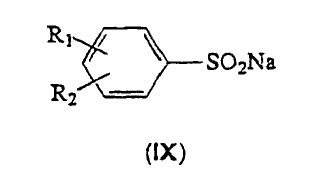
où R₁ et R₂ sont tels que définis ci-dessus pour un composé de formule (I), avec des esters d'acide α-halogén-acétique de formule (X) :
Hlg-CH₂-COOR₉ (**X**)
où Hlg est un atome d'halogène et R₉ est un groupe éthyle ou méthyle, pour obtenir un composé de formule (XI) : 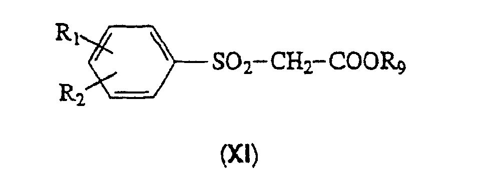 où R₁ et R₂ sont tels que définis ci-dessus pour un composé de formule (I) et R₉ est tel que défini ci-dessus pour des composés de formule (X) ; réaction d'un composé de formule (XI) avec un orthoformiate de trialkyle de formule (XII) :
CH (OR₁₀) ₃ (**XII**)
où R₁₀ est un groupe éthyle ou méthyle, pour obtenir un composé de formule (XIII) : 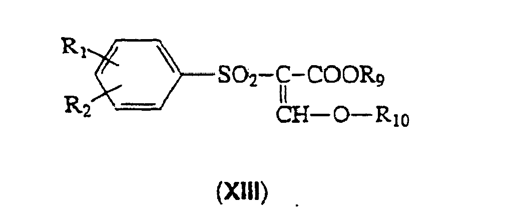 où R₁ et R₂ sont tels que définis ci-dessus pour un composé de formule (I), R₉ est tel que défini ci-dessus pour des composés de formule (X) et R₁₀ est tel que défini ci-dessus pour des composés de formule (XII) ; réaction d'un composé de formule (XIII) avec un dérivé d'aniline de formule (XIV) : 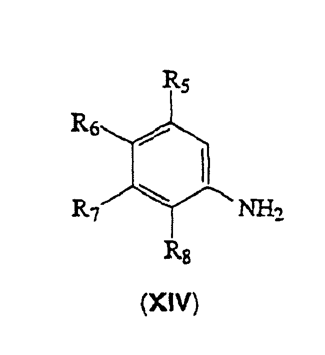 où R₅, R₆, R₇ et R₈ sont tels que définis ci-dessus pour un composé de formule (I), pour obtenir un composé de formule (VI), et formation facultative d'énantiomères et/ou racémates et/ou diastéréoisomères et/ou sels pharmaceutiquement acceptables de celui-ci.

11. Intermédiaire répondant à la formule (IV) 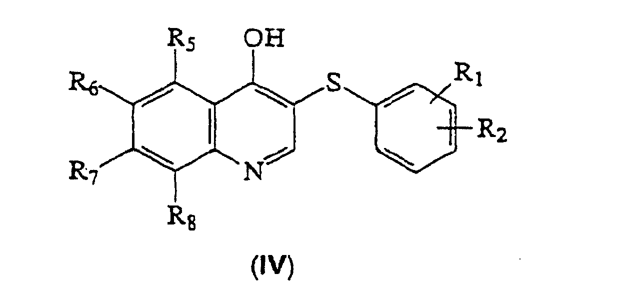 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

12. Intermédiaire répondant à la formule (V) 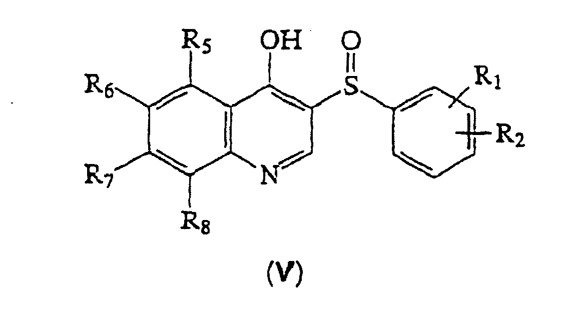 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

13. Intermédiaire répondant à la formule (VI) 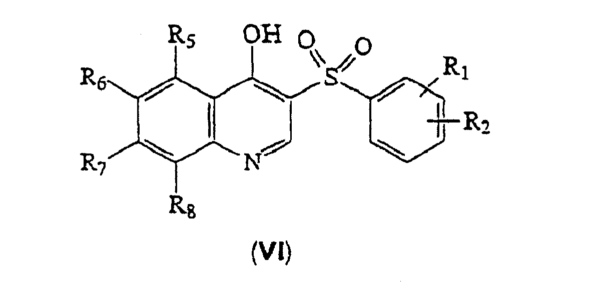 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

14. Intermédiaire répondant à la formule (VII) 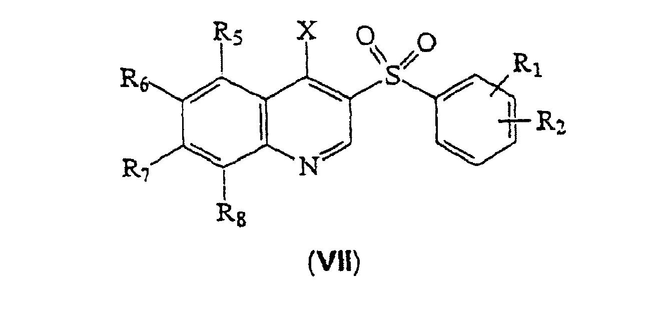 où X est choisi dans le groupe consistant en un atome d'halogène, un groupe benzènesulfonyloxy ou trifluorométhanesulfonyloxy,
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

15. Intermédiaire répondant à la formule (XVI) 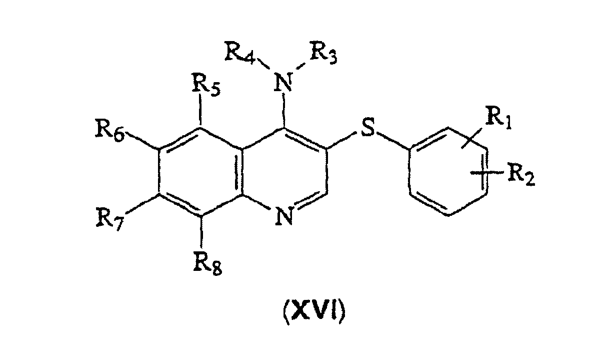 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₃ et R₄ sont indépendamment choisis dans le groupe consistant en un atome d'hydrogène, un groupe alkyle, un groupe aryle substitué comportant au moins un substituant choisi dans le groupe consistant en un atome d'hydrogène, d'halogène, un groupe alkyle ou alcoxy, ou
R₃ et R₄ conjointement avec l'atome N auquel ils sont attachés peuvent former un groupe hétérocyclyle en C_{5 à 7}, contenant 1 ou 2 hétéroatomes choisis dans le groupe de N, O, qui peut être facultativement substitué par les groupes choisis parmi un atome d'hydrogène, d'halogène, un groupe alkyle, alkylhydroxy, alkyloxycarbonyle, aminocarbonyle, -OCH₂CH₂O-, benzyle et phényle substitué ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

16. Intermédiaire répondant à la formule (XVII) 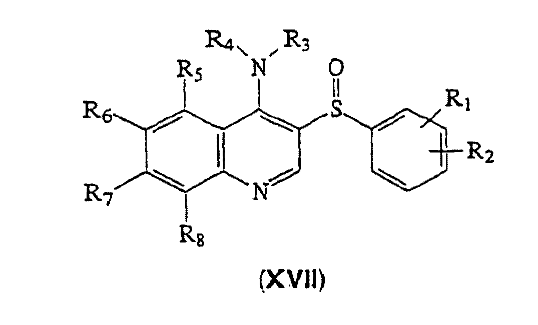 dans laquelle
R₁ et R₂ sont indépendamment choisis dans le groupe consistant en un atome d'hydrogène, d'halogène, un groupe alkyle, alcoxy, cyano, amino facultativement substitué ou un groupe hétérocyclyle saturé, où l'hétéroatome est N ;
R₃ et R₄ sont indépendamment choisis dans le groupe consistant en un atome d'hydrogène, un groupe alkyle, un groupe aryle substitué comportant au moins un substituant choisi dans le groupe consistant en un atome d'hydrogène, d'halogène, un groupe alkyle ou alcoxy, ou
R₃ et R₄ conjointement avec l'atome N auquel ils sont attachés peuvent former un groupe hétérocyclyle en C_{5 à 7}, contenant 1 ou 2 hétéroatomes choisis dans le groupe de N, O, qui peut être facultativement substitué par les groupes choisis parmi un atome d'hydrogène, d'halogène, un groupe alkyle, alkylhydroxy, alkyloxycarbonyle, aminocarbonyle, -OCH₂CH₂O-, benzyle et phényle substitué ;
R₅, R₆, R₇ et R₈ sont indépendamment choisis dans le groupe consistant en un atome d'hydrogène, d'halogène, un groupe alkyle, alcoxy ou cyano, ou
R₆ et R₇ conjointement avec les atomes auxquels ils sont attachés peuvent former un groupe hétérocyclyle insaturé ;
et/ou leurs énantiomères et/ou racémates et/ou diastéréoisomères et/ou sels pharmaceutiquement acceptables formés avec des acides ou des bases.

17. Formulation pharmaceutique comprenant comme ingrédient actif une quantité thérapeutiquement efficace d'un composé de formule (I) 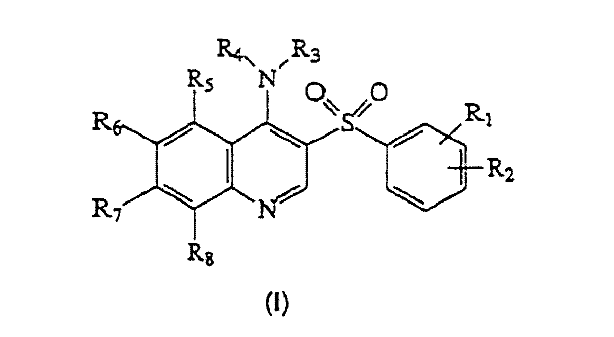 tel que défini dans l'une quelconque des revendications 1 à 3, avec un ou plusieurs diluants, excipients et/ou supports inertes pharmaceutiquement acceptables.

18. Formulation pharmaceutique selon la revendication 17, à utiliser dans la prévention et/ou le traitement de troubles véhiculés par le récepteur mGluR5.

19. Utilisation d'un composé de formule (I) 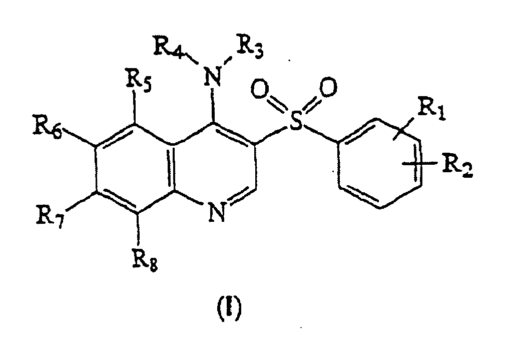 tel que défini dans l'une quelconque des revendications 1 à 3, dans la fabrication d'un médicament destiné à la prévention et/ou au traitement de troubles véhiculés par le récepteur mGluR5.

20. Utilisation selon la revendication 19, dans laquelle lesdits troubles véhiculés par le récepteur mGluR5 sont des troubles psychiatriques.

21. Utilisation selon la revendication 19, dans laquelle lesdits troubles véhiculés par le récepteur mGluR5 sont des troubles neurologiques.

22. Utilisation selon la revendication 19, dans laquelle lesdits troubles véhiculés par le récepteur mGluR5 sont des troubles de douleur chronique et aiguë.

23. Utilisation selon la revendication 19, dans laquelle lesdits troubles véhiculés par le récepteur mGluR5 sont des dysfonctionnements neuromusculaires du tractus urinaire inférieur et des troubles gastro-intestinaux.
